# EUROPEAN PATENT APPLICATION

(11) **EP 4 585 616 A1**
(43) Date of publication of application: **16.07.2025**
(21) Application number: 23862160.1
(22) Date of filing: 17.08.2023
(51) Int. Cl.: C07K 16/28, C12N 15/13, A61K 39/395, A61K 47/68, A61P 35/00, A61P 29/00, G01N 33/68

(54) **ANTIBODY CAPABLE OF SPECIFICALLY BINDING TO ROR1, CONJUGATED DRUG THEREOF, AND PREPARATION METHOD THEREFOR AND USE THEREOF**

(30) Priority: 07.09.2022 CN 202211098907; 07.07.2023 CN 202310832175
(71) Applicant: Hefei Institutes of Physical Science, Chinese Academy of Sciences, Hefei, Anhui 230031 (CN)
(72) Inventor: LIU, Qingsong, Hefei, Anhui 230031 (CN); LIU, Jing, Hefei, Anhui 230031 (CN); JIN, Rui, Hefei, Anhui 230031 (CN); WANG, Wenchao, Hefei, Anhui 230031 (CN); HU, Chen, Hefei, Anhui 230031 (CN); QI, Ziping, Hefei, Anhui 230031 (CN); WANG, Aoli, Hefei, Anhui 230031 (CN); WANG, Li, Hefei, Anhui 230031 (CN)
(74) Representative: J A Kemp LLP
(86) International application number: PCT/CN2023/113487
(87) International publication number: WO 2024/051463

(57) **Abstract**

The present application relates to an antibody specifically binding to ROR1, a conjugated drug thereof, and a preparationmethod therefor and use thereof. The inventor of the present application develops an antibody against ROR1. The antibody and an antibody-drug conjugate can bind to a purified ROR1 protein and ROR1 on the surfaces of various tumor cells with high specificity, have a unique antigen binding epitope, have significant anti-tumor activity, and have high affinity and very low immunogenicity

## Description

### RELATED APPLICATION

The present application claims the priorities of Chinese patent application No. 2022110989077, filed with CNIPA on September 7, 2022, named "ANTIBODY CAPABLE OF SPECIFICALLY BINDING TO ROR1, CONJUGATED DRUG THEREOF, AND PREPARATION METHOD THEREFOR AND USE THEREOF", and Chinese patent application No. 2023108321758, filed with CNIPA on July 7, 2023, named "ANTIBODY CAPABLE OF SPECIFICALLY BINDING TO ROR1, CONJUGATED DRUG THEREOF, AND PREPARATION METHOD THEREFOR AND USE THEREOF", which are incorporated herein by reference in their entireties.

### TECHNICAL FIELD

The present application relates to the field of medicine, and more specifically, to an antibody capable of specifically binding to ROR1, a conjugated drug thereof, and a preparation method and use thereof.

### BACKGROUND

Receptor tyrosine kinase-like orphan receptor 1 (ROR1) is a type I transmembrane protein expressed during embryonic development that contributes to polarized migration and organ formation. Structurally, ROR1 contains a unique extracellular region consisting of an immunoglobulin-like (Ig-like) domain, a curled (Fz) domain, and a kringle (Kr) domain, a transmembrane domain, and a cytoplasmic tyrosine kinase-like domain (J Biol Chem 1992, 267:26181-90). ROR1 participates in the regulation of a variety of physiological functions, including cell division, proliferation, and migration, by mediating the conduction of the non-classical Wnt signaling pathway.

Studies have shown that ROR1 is highly expressed during early embryonic development. As the fetus develops, ROR1 expression gradually decreases. In normal children and adult tissues, ROR1 is lowly expressed or even is not expressed except on rare B lymphocyte precursor cells. When tissues undergo tumor malignant change, ROR1 will initiate a transcription process similar to embryonic development and express high levels of ROR1. At present, ROR1 has been shown to be highly activated in a variety of malignant tumors, such as hematological cancers, including B-cell chronic lymphocytic leukemia (CLL), acute lymphocytic leukemia (ALL), non-Hodgkin lymphoma (NHL) and myeloid blood cancers, as well as solid tumors, including triple-negative breast cancer (TNBC), colon cancer, lung cancer, pancreatic cancer, ovarian cancer and many other cancers, and closely related to the poor prognosis of patients with a tumor (Blood 2016, 128(25):2931-40; Sci Rep 2014, 4:5811). In addition, ROR1 overexpression is involved in the occurrence of resistance to tumor chemotherapies and targeted therapies (Cells 2021, 10:142), including resistance to paclitaxel in the treatment of breast cancer (Proc Natl Acad Sci USA 2019, 116(4):1370-7), resistance to the anti-BCL-2 inhibitor venetoclax in the treatment of chronic lymphocytic leukemia (CLL) (Leukemia 2022, 36(6):1609-18), and resistance to T-DM1 in the treatment of HER2+ breast cancer ( EBioMedicine 2019, 43:211-24).

Given that ROR1 is expressed highly in a variety of solid and hematological malignancies, but at very low levels in healthy tissues, ROR1 is expected to become a new drug target with broad-spectrum anti-cancer potential like PD-1. However, there is currently a lack of highly specific ROR1 antibodies targeting different binding epitopes as well as antibody-drug conjugates. Therefore, there is still a need in the field to develop novel antibodies targeting ROR1 and antibody-drug conjugates.

In view of this, the present application is hereby proposed.

### SUMMARY

Various embodiments of the present application provide an antibody capable of specifically binding to ROR1, a conjugated drug thereof, and a preparation method and use thereof. The technical solutions are as follows.

In a first aspect of the present application, an antibody capable of specifically binding to ROR1 is provided, the antibody including a heavy chain variable region and a light chain variable region.

The heavy chain variable region including heavy chain complementarity determining regions selected from one or more sets of the following heavy chain complementarity determining regions or derivative fragments thereof:
i) VH-CDR1 as set forth in SEQ ID NO: 1, VH-CDR2 as set forth in SEQ ID NO: 2, and VH-CDR3 as set forth in SEQ ID NO: 3;
ii) VH-CDR1 as set forth in SEQ ID NO: 11, VH-CDR2 as set forth in SEQ ID NO: 12, and VH-CDR3 as set forth in SEQ ID NO: 13; and
iii) VH-CDR1 of SEQ ID NO.19, VH-CDR2 of SEQ ID NO.20, and VH-CDR3 of SEQ ID NO.21; and/or,
the light chain variable region including light chain complementarity determining regions selected from one or more sets of the following light chain complementarity determining regions or derivative fragments thereof:
i') VL-CDR1 as set forth in SEQ ID NO: 4, VL-CDR2 as set forth in SEQ ID NO: 5, and VL-CDR3 as set forth in SEQ ID NO: 6;
ii') VL-CDR1 as set forth in SEQ ID NO: 14, VL-CDR2 as set forth in SEQ ID NO: 15, and VL-CDR3 as set forth in SEQ ID NO: 16; and
iii') VL-CDR1 as set forth in SEQ ID NO: 22, VL-CDR2 as set forth in SEQ ID NO: 23, and VL-CDR3 as set forth in SEQ ID NO: 24.

The derivative fragments have no more than 6 amino acid substitutions with respect to the corresponding complementary determining regions and are capable of retaining EC₅₀ of 0.009 nM to 0.05 nM for ROR1-ECD and EC₅₀ of 0.15 nM to 1.1 nM for tumor cell ROR1.

In some embodiments of the present application, the heavy chain complementarity determining regions of the heavy chain variable region are selected from one or more sets of the following heavy chain complementarity determining regions:
i) VH-CDR1 having a sequence represented by general formula D-Y-N-Xa1-H, VH-CDR2 having a sequence represented by general formula Y-I-N-P-N-Xa2-Xa3-Xa4-T-Xa5-Y-N-Q-K-F-Xa6-G, and VH-CDR3 having a sequence represented by general formula R-Xa7- Xa8- Xa9- Xa10- Xa11-Xa12-Xa13-D-Xa14; wherein Xa1 is I, M or L, Xa2 is N or H, Xa3 is D or G, Xa4 is A, N or G, Xa5 is S, N or T, Xa6 is Q, K or E, Xa7 is V or G, Xa8 is R or Y, Xa9 is T or G, Xa10 is S or G, Xa11 is S, T or G, Xa12 is G or Y, Xa13 is L, F or AM, and Xa14 is D, F or Y;
ii) VH-CDR1 as set forth in SEQ ID NO: 11, VH-CDR2 as set forth in SEQ ID NO: 12, and VH-CDR3 as set forth in SEQ ID NO: 13; and
iii) VH-CDR1 as set forth in SEQ ID NO: 19, VH-CDR2 having a sequence represented by general formula T-I-S-D-Xb1-G-S-Y-T-Y-Y-P-D-Xb2-Xb3-K-G, and VH-CDR3 as set forth in SEQ ID NO: 21; wherein Xb1 is A or G, Xb2 is S or N, and Xb3 is V or E; and/or
the light chain variable region including light chain complementarity determining regions selected from one or more sets of the following light chain complementarity determining regions or derivative fragments thereof:
i') VL-CDR1 having a sequence represented by general formula Xa1'-S-S-Xa2'-Xa3'-I-Xa4'-H-T-N-Xa5'-N-T-Y-L-E, VL-CDR2 having a sequence represented by general formula K-V-Xa6'-N-R-F-S, and VL-CDR3 having a sequence represented by general formula F-Q-G-S-Xa7'- Xa8'-P-Y-T; wherein Xa1' is R, K or T, Xa2' is H or Q, Xa3' is I, N or S, Xa4' is V or L, Xa5' is A or G, Xa6' is F or S, Xa7' is R or L, and Xa8' is F or V;
ii') VL-CDR1 as set forth in SEQ ID NO: 14, VL-CDR2 as set forth in SEQ ID NO: 15, and VL-CDR3 as set forth in SEQ ID NO: 16; and
iii') VL-CDR1 having a sequence represented by general formula K-A-S-Q-S-V-S-F-Xb1'-G-T-S-L-M-H, VL-CDR2 as set forth in SEQ ID NO: 23, and VL-CDR3 as set forth in SEQ ID NO: 24; wherein Xb1' is A or P.

In some embodiments of the present application, in the heavy chain complementary determining regions as shown in i): Xa1 is L, Xa2 is H, Xa3 is D, Xa4 is A or G, Xa5 is S or T, Xa6 is Q, Xa7 is V, Xa8 is R, Xa9 is T, Xa10 is G, Xa11 is T, Xa12 is G, Xa13 is L or F, and Xa14 is D or Y; or
Xa1 is I or M, Xa2 is N, Xa3 is G, Xa4 is N or G, Xa5 is S, N or T, Xa6 is K or E, Xa7 is G, Xa8 is Y, Xa9 is G, Xa10 is S, Xa11 is S or G, Xa12 is Y, Xa13 is AM, and Xa14 is F or Y

In some embodiments of the present application, in the heavy chain complementary determining regions as shown in i'): Xa1' is R or K, Xa2' is H, Xa3' is N, Xa4' is V or L, Xa5' is A or G, Xa6' is S, Xa7' is R, and Xa8' is F; or
Xa1' is R or T, Xa2' is H or Q, Xa3' is I or S, Xa4' is V, Xa5' is G, Xa6' is F or S, Xa7' is R or L, and Xa8' is F or V.

In some embodiments of the present application, the antibody has one or more of the following combinations of heavy chain complementary determining regions and light chain complementary determining regions:
Combination 1: the heavy chain complementary determining regions as shown in i) or derivative fragments thereof, and the light chain complementary determining region as shown in i') or derivative fragments thereof;
Combination 2: the heavy chain complementary determining regions as shown in ii) or derivative fragments thereof, and the light chain complementary determining region as shown in ii') or derivative fragments thereof;
Combination 3: the heavy chain complementary determining regions as shown in iii) or derivative fragments thereof, and the light chain complementary determining region as shown in iii') or derivative fragments thereof.

In some embodiments of the present application, the combination 1 is selected from one or more of the following combinations:
Combination 1-1: VH-CDR1 as set forth in SEQ ID NO: 1, VH-CDR2 as set forth in SEQ ID NO: 2, VH-CDR3 as set forth in SEQ ID NO: 3, VL-CDR1 as set forth in SEQ ID NO: 4, VL-CDR2 as set forth in SEQ ID NO: 5, and VL-CDR3 as set forth in SEQ ID NO: 6;
Combination 1-2: VH-CDR1 as set forth in SEQ ID NO: 35, VH-CDR2 as set forth in SEQ ID NO: 36, VH-CDR3 as set forth in SEQ ID NO: 37, VL-CDR1 as set forth in SEQ ID NO: 38, VL-CDR2 as set forth in SEQ ID NO: 39, and VL-CDR3 as set forth in SEQ ID NO: 40;
Combination 1-3: VH-CDR1 as set forth in SEQ ID NO: 51, VH-CDR2 as set forth in SEQ ID NO: 52, VH-CDR3 as set forth in SEQ ID NO: 53, VL-CDR1 as set forth in SEQ ID NO: 54, VL-CDR2 as set forth in SEQ ID NO: 55, and VL-CDR3 as set forth in SEQ ID NO: 56;
Combination 1-4: VH-CDR1 as set forth in SEQ ID NO: 59, VH-CDR2 as set forth in SEQ ID NO: 60, VH-CDR3 as set forth in SEQ ID NO: 61, VL-CDR1 as set forth in SEQ ID NO: 62, VL-CDR2 as set forth in SEQ ID NO: 63, and VL-CDR3 as set forth in SEQ ID NO: 64;
Combination 1-5: VH-CDR1 as set forth in SEQ ID NO: 67, VH-CDR2 as set forth in SEQ ID NO: 68, VH-CDR3 as set forth in SEQ ID NO: 69, VL-CDR1 as set forth in SEQ ID NO: 70, VL-CDR2 as set forth in SEQ ID NO: 71, and VL-CDR3 as set forth in SEQ ID NO: 72;
Combination 1-6: VH-CDR1 as set forth in SEQ ID NO: 75, VH-CDR2 as set forth in SEQ ID NO: 76, VH-CDR3 as set forth in SEQ ID NO: 77, VL-CDR1 as set forth in SEQ ID NO: 78, VL-CDR2 as set forth in SEQ ID NO: 79, and VL-CDR3 as set forth in SEQ ID NO: 80;
Combination 1-7: VH-CDR1 as set forth in SEQ ID NO: 1, VH-CDR2 as set forth in SEQ ID NO: 83, VH-CDR3 as set forth in SEQ ID NO: 3, VL-CDR1 as set forth in SEQ ID NO: 84, VL-CDR2 as set forth in SEQ ID NO: 5, and VL-CDR3 as set forth in SEQ ID NO: 6.
In some embodiments of the present application, the combination 3 is selected from one or more of the following combinations:
Combination 3-1: VH-CDR1 as set forth in SEQ ID NO: 43, VH -CDR2 as set forth in SEQ ID NO: 44, VH-CDR3 as set forth in SEQ ID NO: 45, VL-CDR1 as set forth in SEQ ID NO: 46, VL-CDR2 as set forth in SEQ ID NO: 47, and VL-CDR3 as set forth in SEQ ID NO: 48;
Combination 3-2: VH-CDR1 as set forth in SEQ ID NO: 19, VH-CDR2 as set forth in SEQ ID NO: 20, VH-CDR3 as set forth in SEQ ID NO: 21, VL-CDR1 as set forth in SEQ ID NO: 22, VL-CDR2 as set forth in SEQ ID NO: 23, and VL-CDR3 as set forth in SEQ ID NO: 24;
Combination 3-3: VH-CDR1 as set forth in SEQ ID NO: 19, VH-CDR2 as set forth in SEQ ID NO: 85, VH-CDR3 as set forth in SEQ ID NO: 21, VL-CDR1 as set forth in SEQ ID NO: 22, VL-CDR2 as set forth in SEQ ID NO: 23, and VL-CDR3 as set forth in SEQ ID NO: 24.

In some embodiments of the present application, the antibody has one or more of the following technical features:
(1) the antibody having a heavy chain constant region and a light chain constant region independently derived from a species selected from human, mouse, rabbit, sheep, cattle, horse, pig, dog, cat, camel, donkey, deer, mink, chicken, duck or goose; and,
(2) the constant region of the antibody or antigen-binding fragment thereof has a sequence of the constant region selected from any one of IgG1, IgG2, IgG3, IgG4, IgA, IgM, IgE and IgD; and
(3) the constant region of the antibody has a sequence of the constant region selected from any one of κ light chain constant region and λ light chain constant region.

In some embodiments of the present application, the antibody has a combination selected from the following combinations of a heavy chain variable region and a light chain variable region:
(1) a heavy chain variable region as set forth in SEQ ID NO: 7 and a light chain variable region as set forth in SEQ ID NO: 9;
(2) a heavy chain variable region as set forth in SEQ ID NO: 41 and a light chain variable region as set forth in SEQ ID NO: 42;
(3) a heavy chain variable region as set forth in SEQ ID NO: 49 and a light chain variable region as set forth in SEQ ID NO: 50;
(4) a heavy chain variable region as set forth in SEQ ID NO: 17 and a light chain variable region as set forth in SEQ ID NO: 18;
(5) a heavy chain variable region as set forth in SEQ ID NO: 25 and a light chain variable region as set forth in SEQ ID NO: 27;
(6) a heavy chain variable region as set forth in SEQ ID NO: 57 and a light chain variable region as set forth in SEQ ID NO: 58;
(7) a heavy chain variable region as set forth in SEQ ID NO: 65 and a light chain variable region as set forth in SEQ ID NO: 66;
(8) a heavy chain variable region as set forth in SEQ ID NO: 73 and a light chain variable region as set forth in SEQ ID NO: 74;
(9) a heavy chain variable region as set forth in SEQ ID NO: 81 and a light chain variable region as set forth in SEQ ID NO: 82;
(10) a heavy chain variable region as set forth in SEQ ID NO: 8 and a light chain variable region as set forth in SEQ ID NO: 10;
(11) a heavy chain variable region as set forth in SEQ ID NO: 26 and a light chain variable region as set forth in SEQ ID NO: 27;
(12) a heavy chain variable region as set forth in SEQ ID NO: 28 and a light chain variable region as set forth in SEQ ID NO: 33;
(13) a heavy chain variable region as set forth in SEQ ID NO: 28 and a light chain variable region as set forth in SEQ ID NO: 34;
(14) a heavy chain variable region as set forth in SEQ ID NO: 29 and a light chain variable region as set forth in SEQ ID NO: 33;
(15) a heavy chain variable region as set forth in SEQ ID NO: 29 and a light chain variable region as set forth in SEQ ID NO: 34;
(16) a heavy chain variable region as set forth in SEQ ID NO: 30 and a light chain variable region as set forth in SEQ ID NO: 33;
(17) a heavy chain variable region as set forth in SEQ ID NO: 31 and a light chain variable region as set forth in SEQ ID NO: 33;
(18) a heavy chain variable region as set forth in SEQ ID NO: 31 and a light chain variable region as set forth in SEQ ID NO: 34;
(19) a heavy chain variable region as set forth in SEQ ID NO: 32 and a light chain variable region as set forth in SEQ ID NO: 33;
(20) a heavy chain variable region as set forth in SEQ ID NO: 32 and a light chain variable region as set forth in SEQ ID NO: 34;
(21) a heavy chain variable region as set forth in SEQ ID NO: 93 and a light chain variable region as set forth in SEQ ID NO: 101;
(22) a heavy chain variable region as set forth in SEQ ID NO: 94 and a light chain variable region as set forth in SEQ ID NO: 101;
(23) a heavy chain variable region as set forth in SEQ ID NO: 95 and a light chain variable region as set forth in SEQ ID NO: 101;
(24) a heavy chain variable region as set forth in SEQ ID NO: 96 and a light chain variable region as set forth in SEQ ID NO: 101;
(25) a heavy chain variable region as set forth in SEQ ID NO: 97 and a light chain variable region as set forth in SEQ ID NO: 101;
(26) a heavy chain variable region as set forth in SEQ ID NO: 98 and a light chain variable region as set forth in SEQ ID NO: 101;
(27) a heavy chain variable region as set forth in SEQ ID NO: 99 and a light chain variable region as set forth in SEQ ID NO: 101; and
(28) A heavy chain variable region as set forth in SEQ ID NO: 100 and a light chain variable region as set forth in SEQ ID NO: 101.

In a second aspect of the present application, a recombinant protein is provided, comprising:
one or more of the heavy chain complementarity determining regions and light chain complementarity determining regions as defined in the first aspect; and
a tag fragment that facilitates the expression and/or purification of the heavy chain complementary determining regions and the light chain complementary determining regions.

In some embodiments of the present application, the recombinant protein further includes the heavy chain constant region or the light chain constant region as defined in the first aspect, or a combination thereof.

In a third aspect of the present application, a CAR construct is provided, wherein the CAR construct has a scFV domain having the heavy chain variable region and the light chain variable region as defined in the first aspect.

In a fourth aspect of the present application, a nucleic acid is provided, wherein the nucleic acid encodes the antibody as desribed in the first aspect, the recombinant protein as described in the second aspect, or the CAR construct as described in the third aspect.

In a fifth aspect of the present application, a vector is provided, wherein the vector contains the nucleic acid as described in the fourth aspect.

In some embodiments of the present application, the vector is selected from a bacterial plasmid, a bacteriophage, a yeast plasmid, a plant cell virus, a mammalian cell virus such as adenovirus or retrovirus, or a combination thereof.

In a sixth aspect of the present application, a host cell is provided, wherein the host cell contains the nucleic acid as described in the fourth aspect or the vector as described in the fifth aspect.

In some embodiments of the present application, the host cell is CHO cells, COS cells, NSO cells, HeLa cells, BHK cells or HEK293 cells.

In a seventh aspect of the present application, a method for preparing the antibody as described in the first aspect, the recombinant protein as described in the second aspect, or the CAR construct as described in the third aspect is provided, the method comprising:
culturing the host cell as described in the sixth aspect, and isolating the antibody, recombinant protein or CAR construct from the obtained culture.

In the eighth aspect of the present application, a recombinant immune cell is provided, wherein the recombinant immune cell expresses the exogenous CAR construct as described in the third aspect.

In some embodiments of the present application, the immune cell is selected from one of NK cells and T cells.

In a ninth aspect of the present application, use of the antibody as described in the first aspect, the recombinant protein as described in the second aspect, the CAR construct as described in the third aspect, or the recombinant immune cell as described in the eighth aspect in manufacturing a medicament for treating ROR1-related disease or a product for diagnosing ROR1-related disease is provided.

In some embodiments of the present application, the ROR1-related disease is a tumor, an autoimmune disease, a metabolism-related disease or an infectious disease.

In some embodiments of the present application, the tumor is breast cancer, lung cancer, pancreatic cancer, ovarian cancer, prostate cancer, colorectal cancer, gastric cancer, liver cancer, esophageal cancer, kidney cancer, brain glioma, bladder cancer, prostate cancer, endometrial cancer, cervical cancer, leukemia, lymphoma, bone marrow cancer or angiosarcoma.

The inflammation is rheumarthritis, osteoarthritis, gout, Reiter's syndrome, psoriatic arthropathy, tuberculous arthritis, glomerulonephritis, systemic lupus erythematosus, Crohn's disease, ulcerative colitis, acute lung injury, chronic obstructive pulmonary disease or idiopathic pulmonary fibrosis.

The autoimmune disease is rheumatoid arthritis, ankylosing spondylitis, systemic lupus erythematosus, Sjögren's syndrome or systemic vasculitis, ulcerative colitis, type I diabetes, psoriasis, or multiple sclerosis.

The metabolic-related disease is diabetes, diet-induced obesity or fat inflammation.

The infectious disease is a disease caused by bacterial infection or by viral infection.

In some embodiments of the present application, the product for diagnosing ROR1-related disease is a diagnostic reagent, a test strip, a test plate or a test kit.

In a tenth aspect of the present application, an antibody-drug conjugate is provided, wherein the antibody-drug conjugate includes:
an antibody selected from the antibody as defined in the first aspect; and,
a conjugant coupled to the antibody.

In some embodiments of the present application, the antibody-drug conjugate has one or more of the following technical features:
the conjugant is selected from a detectable label, a cytotoxic drug, a cytokine, a radionuclide, an enzyme or a combination thereof; and,
the antibody is conjugated to the conjugant via a chemical linkage or a linker.

In some embodiments of the present application, the antibody-drug conjugate has one or more of the following technical features:
the antibody-drug conjugate has a structure as shown in the following molecular formula: 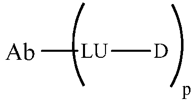 wherein
Ab is the antibody,
LU is a linker,
D is a cytotoxic drug;
the linker comprises a sulfydryl-specific active reactive group; and
the cytotoxic drug is a microtubule-targeted drug, a DNA-targeted drug or a topoisomerase inhibitor.

In an eleventh aspect of the present application, a pharmaceutical composition is provided, comprising:
the antibody as described in the first aspect, the recombinant protein as described in the second aspect, the CAR construct as described in the third aspect, the recombinant immune cell as described in the eighth aspect, the antibody-drug conjugate as described in the tenth aspect, or a combination thereof; and
a pharmaceutically acceptable carrier.

In a twelfth aspect of the present application, a product for diagnosing ROR1-related disease is provided, wherein
the product is a test plate coated with the antibody or antigen-binding fragment thereof as described in the first aspect, the recombinant protein as described in the second aspect, the CAR construct as described in the third aspect, the recombinant immune cell as described in the eighth aspect, or a combination thereof; or
the product is a test kit comprising a primary antibody and a secondary antibody for detecting ROR1, wherein the primary antibody is the antibody as defined in the first aspect.

In a thirteenth aspect of the present application, a method for detecting ROR1 for non-diagnostic purposes is provided, comprising:
providing a sample to be tested, and
mixing the sample to be tested with the antibody as described in the first aspect for reaction, detecting the reaction product, and determining the presence of ROR1 in the sample to be tested based on the test result.

In some embodiments of the present application, determining the presence of ROR1 in the sample to be tested based on the test result includes:
when an antigen-antibody complex is detected in the reaction product, it is determined that the sample to be tested contains ROR1; or
when no antigen-antibody complex is detected in the reaction product, it is determined that the sample to be tested contains no ROR1.

Details of one or more embodiments of the present application are set forth in the following description. The further features, objects, and advantages of the present application will become apparent from the specification and the claims.

### BRIEF DESCRIPTION OF THE DRAWINGS

To better clarify technical solutions of embodiments of the present application or the conventional technologies, drawings used in descriptions of embodiments of the present application or the conventional technologies are briefly introduced hereinafter. Apparently, the described drawings merely illustrate the embodiments of the present application. Those skilled in the art can obtain other drawings based on these drawings without any creative efforts.

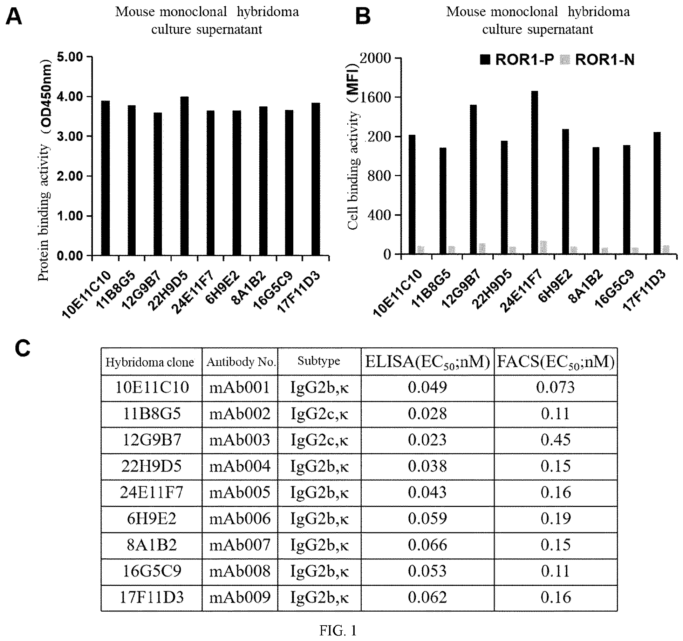

FIG. 1 shows the discovery of the anti-human ROR1 antibody in Example 1 of the present application; FIG. 1A shows enzyme-linked immunosorbent assay (ELISA) for testing the activity of binding of culture supernatants of a series of original anti-human ROR1 monoclonal antibodies (original hybridoma) to the purified human ROR1 protein extracellular segment (ROR1-ECD); FIG. 1B shows detection of the activity of binding of the culture supernatants of original anti-human ROR1 monoclonal antibodies (original hybridoma) against human ROR1-high-expressing MDA-MB-231 (ROR1-P) and ROR1-low-expressing MDA-MB-453 (ROR1-N) breast cancer cells by fluorescence-activated cell sorter (FACS); FIG. 1C shows the subtype identification of 9 monoclonal antibodies (mAb001, mAb002, mAb003, mAb004, mAb005, mAb006, mAb007, mAb008, mAb009) after purification, and EC₅₀ values of binding affinity of the 9 monoclonal antibodies against ROR1-ECD protein and MDA-MB-231 cells,;
FIG. 2 shows the SDS-PAGE diagram of 9 human-mouse chimeric antibodies mAb001c, mAb002c, mAb003c, mAb004c, mAb005c, mAb006c, mAb007c, mAb008c and mAb009c purified in Example 3 of the present application;
FIG. 3 shows the binding affinity (Binding affinity EC₅₀) of human-mouse chimeric antibodies mAb001c, mAb002c, mAb003c, mAb004c, mAb005c, mAb006c, mAb007c, mAb008c and mAb009c against ROR1-ECD measured by ELISA in Example 4 of the present application;
FIG. 4 shows the binding affinity (Binding affinity EC₅₀) of site-directed mutants mAb001c and mAb005c against ROR1-ECD measured by ELISA in Example 4 of the present application;
FIG. 5 shows the analysis and comparison of the expression levels of ROR1 mRNA (ratio to β-actin) in various tumor cell lines (lung cancer, breast cancer, colon cancer, blood tumors, liver cancer, gastric cancer) and 30 normal human tissues in Example 5 of the present application;
FIG. 6 shows the analysis of the expression levels of ROR1 mRNA in highly invasive and highly metastatic Basal-type/TNBC versus Luminal-type breast cancer cell lines from the Cancer Cell Line Encyclopedia (CCLE) database in Example 5 of the present application;
FIG. 7 shows the Western blot test for measuring the expression level of ROR1 protein in different lung cancer cell lines in Example 6 of the present application;
FIG. 8 shows the Western blot test for measuring the expression level of ROR1 protein in Basal-type/TNBC and Luminal-type breast cancer cell lines in Example 6 of the present application;
FIG. 9 shows the binding levels of mAb001c (5 µg/mL) against ROR1 on the surface of ROR1-highly expressed (NCI-H446, HT-29, MDA-MB-231, HCC1187, HCC827, or NCI-H226) or lowly expressed (MDA-MB-453, or MCF-7) tumor cells in Example 7 of the present application;
FIG. 10 shows the test results of binding affinity (EC₅₀) of the chimeric antibodies mAb001c, mAb002c, mAb003c, mAb004c, mAb005c, mAb006c, mAb007c, mAb008c and mAb009c in Example 8 of the present application against ROR1 on the surface of MDA-MB-231 cells; in this test, 2 × 10⁵ cells were mixed with the antibody at the indicated gradient of concentrations, and the MFI was measured after incubation for 1 hour;
FIG. 11 shows the test results of binding affinity (EC₅₀) of the chimeric antibodies mAb001c, mAb002c, mAb003c, mAb004c, mAb005c, mAb006c, mAb007c, mAb008c and mAb009c against ROR1 on the surface of NCI-H226 cells in Example 8 of the present application; in this test, 2 × 10⁵ cells were mixed with the antibody at the indicated gradient of concentrations, and the MFI was measured after incubation for 1 hour;
FIG. 12 shows the binding affinity (Binding affinity EC₅₀) of site-directed mutants mAb001c and mAb005c against MDA-MB-231 measured by FACS in Example 8 of the present application;
FIG. 13 shows the test results of binding affinity (EC₅₀) of the chimeric antibodies mAb001c, mAb002c, mAb003c, mAb004c, mAb005c, mAb006c, mAb007c, mAb008c and mAb009c against ROR1 transfected on the surface of CHO cells in Example 9 of the present application; in this test, 2 × 10⁵ cells were mixed with the human-mouse chimeric antibody at the indicated gradient of concentrations, and the MFI was measured after incubation for 1 hour;
FIG. 14 shows the test results of binding affinity (EC₅₀) of the chimeric antibodies mAb001c, mAb002c, mAb003c, mAb004c, mAb005c, mAb006c, mAb007c, mAb008c and mAb009c against ROR1 (with Ig-like domain removed) transfected on the surface of CHO cells in Example 9 of the present application; in this test, 2 × 10⁵ cells were mixed with the antibody at the indicated gradient of concentrations, and the MFI was measured after incubation for 1 hour;
FIG. 15 shows the test results of binding affinity (EC₅₀) of the chimeric antibodies mAb001c, mAb002c, mAb003c, mAb004c, mAb005c, mAb006c, mAb007c, mAb008c and mAb009c against ROR1 (with Frizzled domain removed) transfected on the surface of CHO cells in Example 9 of the present application; in this test, 2 × 10⁵ cells were mixed with the antibody at the indicated gradient of concentrations, and the MFI was measured after incubation for 1 hour;
FIG. 16 shows the test results of binding affinity (EC₅₀) of the chimeric antibodies mAb001c, mAb002c, mAb003c, mAb004c, mAb005c, mAb006c, mAb007c, mAb008c and mAb009c against ROR1 (with Kringle domain removed) transfected on the surface of CHO cells in Example 9 of the present application; in this test, 2 × 10⁵ cells were mixed with the antibody at the indicated gradient of concentrations, and the MFI was measured after incubation for 1 hour;
FIG. 17 shows the in vivo anti-tumor activity tested for the ROR1 chimeric antibody in Example 10 of the present application; in the in vivo test, ROR1-high-expressing NCI-H226 lung cancer cells were mixed with 50 µg of antibody and then inoculated subcutaneously to the back of nude mice, and the mice were observed 2 to 3 times per week with the tumor volume and mouse weight measured;
FIG. 18 shows internalization into intracellular lysosomes caused by binding mAb001c, mAb002c, mAb004c, and mAb005c to MDA-MB-231 cells in Example 11 of the present application; the antibody (5 µg/mL) was incubated with the cells at 4°C for 1 hour or at 37°C for 4 hours, and then observed under a laser confocal microscope;
FIG. 19 shows the hydrophobic interaction chromatography (HIC)-HPLC profiles of the antibody mAb001c and the antibody-drug conjugate mAb001c-vcMMAE in Example 12 of the present application;
FIG. 20 shows the size exclusion chromatography (SEC)-HPLC profiles of the antibody mAb001c and the antibody-drug conjugate mAb001c-vcMMAE in Example 12 of the present application;
FIG. 21 shows the test results of the in vitro anti-proliferative activity (IC₅₀) of mAb001c-vcMMAE on ROR1 high-expressing cell lines (MDA-MB-231, HCC1187, HT-29, NCI-H446, HCC827, and NCI-N87) and ROR1 low-expressing cell line (MCF-7) in Example 13 of the present application;
FIG. 22 shows the in vivo anti-tumor efficacy of mAb001c-vcMMAE (5 mg/kg) on MDA-MB-231 breast cancer tumor model in Example 14 of the present application;
FIG. 23 shows the in vivo anti-tumor efficacy of mAb001c-vcMMAE (5 mg/kg, 2.5 mg/kg) on HT-29 colon cancer tumor model in Example 14 of the present application;
FIG. 24 shows the binding affinity (Binding affinity EC₅₀) of the humanized antibody series Hu001-2, 3, 5, 6, 8, 11, 12, 14, and 15 based on mAb001c against ROR1-ECD measured by ELISA in Example 17 of the present application;
FIG. 25 shows the binding affinity (Binding affinity EC₅₀) of the humanized antibody series Hu005-35, 40, 41, 42, 43, 44, 45, and 46 based on mAb005c against ROR1-ECD measured by ELISA in Example 17 of the present application;
FIG. 26 shows the binding affinity (Binding affinity EC₅₀) of the humanized antibody series Hu001-2 and Hu005-46 against human ROR1-ECD and human ROR2-ECD measured by ELISA, in Example 18 of the present application;
FIG. 27 shows the binding affinity (Binding affinity EC₅₀) of the humanized antibody series Hu001-2, 3, 5, 6, 8, 11, 12, 14, and 15 based on mAb001c and the humanized antibody series Hu005-35, 40, 41, 42, 43, 44, 45, and 46 based on mAb005c against MDA-MB-231 measured by FACS in Example 19 of the present application;
FIG. 28 shows the binding affinity (Binding affinity EC₅₀) of the humanized antibody series Hu001-2, 3, 5, 6, 8, 11, 12, 14, and 15 based on mAb001c and the humanized antibody series Hu005-35, 40, 41, 42, 43, 44, 45, and 46 based on mAb005c against NCI-H226 measured by FACS in Example 19 of the present application;
FIG. 29 shows internalization into intracellular lysosomes caused by binding Hu001-02, Hu001-03, Hu005-44, and Hu005-46 to MDA-MB-231 cells in Example 11 of the present application; the antibody (5 µg/mL) was incubated with the cells at 4°C for 1 hour or at 37°C for 4 hours, and then observed under a laser confocal microscope;
FIG. 30 shows the endocytosis curves of the humanized antibodies Hu001-02, Hu001-03, Hu005-44, and Hu005-46 in Example 21 of the present application, plotted at 0, 30 min, 60 min, 120 min, and 240 min for which they bind to MDA-MB-231 cells;
FIG. 31 shows the endocytosis curves of the humanized antibodies Hu001-02, and Hu005-46 in Example 21 of the present application, plotted at 0, 30 min, 60 min, 120 min, and 240 min for which they bind to NCI-N87 cells;
FIG. 32 shows the endocytosis curves of the humanized antibodies Hu001-02, and Hu005-46 in Example 21 of the present application, plotted at 0, 30 min, 60 min, 120 min, and 240 min for which they bind to NCI-H446 cells;
FIG. 33 shows the hydrophobic interaction chromatography (HIC)-HPLC profiles of the humanized antibody Hu001-2 and the antibody-drug conjugate Hu001-2-MMAE in Example 22 of the present application;
FIG. 34 shows the size exclusion chromatography (SEC)-HPLC profiles of the humanized antibody Hu001-2 and the antibody-drug conjugate Hu001-2-MMAE in Example 22 of the present application;
FIG. 35 shows the hydrophobic interaction chromatography (HIC)-HPLC profiles of the humanized antibody Hu001-3 and the antibody-drug conjugate Hu001-3-MMAE in Example 22 of the present application;
FIG. 36 shows the size exclusion chromatography (SEC)-HPLC profiles of the humanized antibody Hu001-3 and the antibody-drug conjugate Hu001-3-MMAE in Example 22 of the present application;
FIG. 37 shows the hydrophobic interaction chromatography (HIC)-HPLC profiles of the humanized antibody Hu005-44 and the antibody-drug conjugate Hu005-44-MMAE in Example 22 of the present application;
FIG. 38 shows the size exclusion chromatography (SEC)-HPLC profiles of the humanized antibody Hu005-44 and the antibody-drug conjugate Hu005-44-MMAE in Example 22 of the present application;
FIG. 39 shows the hydrophobic interaction chromatography (HIC)-HPLC profiles of the humanized antibody Hu005-46 and the antibody-drug conjugate Hu005-46-MMAE in Example 22 of the present application;
FIG. 40 shows the size exclusion chromatography (SEC)-HPLC profiles of the humanized antibody Hu005-46 and the antibody-drug conjugate Hu005-46-MMAE in Example 22 of the present application;
FIG. 41 shows the hydrophobic interaction chromatography (HIC)-HPLC profiles of the positive reference antibody UC961 and the antibody-drug conjugate UC961-MMAE in Example 22 of the present application;
FIG. 42 shows the size exclusion chromatography (SEC)-HPLC profiles of the positive reference antibody UC961 and the antibody-drug conjugate UC961-MMAE in Example 22 of the present application;
FIG. 43 shows the binding affinity (Binding affinity EC₅₀) of the humanized antibodies Hu001-2, Hu001-3, Hu005-44, and Hu005-46 and the antibody conjugates Hu001-2-MMAE, Hu001-3-MMAE, Hu005-44-MMAE, and Hu005-46-MMAE against ROR1-ECD measured by ELISA in Example 23 of the present application;
FIG. 44 shows the binding affinity (Binding affinity EC₅₀) of the humanized antibodies Hu001-2, Hu001-3, Hu005-44, and Hu005-46 and the antibody conjugates Hu001-2-MMAE, Hu001-3-MMAE, Hu005-44-MMAE, and Hu005-46-MMAE against MDA-MB-231 measured by FACS in Example 24 of the present application;
FIG. 45 shows the binding affinity (Binding affinity EC₅₀) of the humanized antibodies Hu001-2, Hu001-3, Hu005-44, and Hu005-46 and the antibody conjugates Hu001-2-MMAE, Hu001-3-MMAE, Hu005-44-MMAE, and Hu005-46-MMAE against NCI-H226 measured by FACS in Example 24 of the present application;
FIG. 46 shows the test results of the in vitro anti-proliferative activity (IC₅₀) Hu001-2-MMAE, Hu001-3-MMAE, Hu005-44-MMAE, Hu005-46-MMAE and UC961-MMAE on SK-BR-3 cells in Example 25 of the present application;
FIG. 47 shows the test results of the in vitro anti-proliferative activity (IC₅₀) of Hu001-2-MMAE, Hu001-3-MMAE, Hu005-44-MMAE, Hu005-46-MMAE and UC961-MMAE on MCF-7 cells in Example 25 of the present application;
FIG. 48 shows the test results of the in vitro anti-proliferative activity (IC₅₀) of Hu001-2-MMAE, Hu001-3-MMAE, Hu005-44-MMAE, Hu005-46-MMAE and UC961-MMAE on MDA-MB-231 cells in Example 25 of the present application;
FIG. 49 shows the test results of the in vitro anti-proliferative activity (IC₅₀) of Hu001-2-MMAE, Hu001-3-MMAE, Hu005-44-MMAE, Hu005-46-MMAE and UC961-MMAE on HCC1187 cells in Example 25 of the present application;
FIG. 50 shows the test results of the in vitro anti-proliferative activity (IC₅₀) of Hu001-2-MMAE, Hu001-3-MMAE, Hu005-44-MMAE, Hu005-46-MMAE and UC961-MMAE on HT-29 cells in Example 25 of the present application;
FIG. 51 shows the test results of the in vitro anti-proliferative activity (IC₅₀) of Hu001-2-MMAE, Hu001-3-MMAE, Hu005-44-MMAE, Hu005-46-MMAE and UC961-MMAE on SK-CO-1 cells in Example 25 of the present application;
FIG. 52 shows the test results of the in vitro anti-proliferative activity (IC₅₀) of Hu001-2-MMAE, Hu001-3-MMAE, Hu005-44-MMAE, Hu005-46-MMAE and UC961-MMAE on Colo-678 cells in Example 25 of the present application;
FIG. 53 shows the test results of the in vitro anti-proliferative activity (IC₅₀) of Hu001-2-MMAE, Hu001-3-MMAE, Hu005-44-MMAE, Hu005-46-MMAE and UC961-MMAE on HCC827 cells in Example 25 of the present application;
FIG. 54 shows the test results of the in vitro anti-proliferative activity (IC₅₀) of Hu001-2-MMAE, Hu001-3-MMAE, Hu005-44-MMAE, Hu005-46-MMAE and UC961-MMAE on NCI-H446 cells in Example 25 of the present application;
FIG. 55 shows the test results of the in vitro anti-proliferative activity (IC₅₀) of Hu001-2-MMAE, Hu001-3-MMAE, Hu005-44-MMAE, Hu005-46-MMAE and UC961-MMAE on PA-1 cells in Example 25 of the present application;
FIG. 56 shows the test results of the in vitro anti-proliferative activity (IC₅₀) of Hu001-2-MMAE, Hu001-3-MMAE, Hu005-44-MMAE, Hu005-46-MMAE and UC961-MMAE on NCI-N87 cells in Example 25 of the present application;
FIG. 57 shows the direct correlation of the cytotoxicity (IC₅₀ value) of Hu001-2-MMAE, Hu001-3-MMAE, Hu005-44-MMAE, Hu005-46-MMAE, and UC961-MMAE with of ROR1 expression level of the tested cells in Example 25, indicating target-specific cytotoxicity;
FIG. 58 shows the in vivo antitumor efficacy of Hu001-2-MMAE, Hu001-3-MMAE, Hu005-44-MMAE, and Hu005-46-MMAE (5 mg/kg, or 2.5 mg/kg) on MDA-MB-231 triple-negative breast cancer tumor model in Example 26 of the present application;
FIG. 59 shows the in vivo antitumor efficacy of Hu001-2-MMAE, Hu005-46-MMAE, and UC961-MMAE (5 mg/kg, or 2.5 mg/kg) on PA-1 ovarian cancer tumor model in Example 26 of the present application;
FIG. 60 shows the in vivo antitumor efficacy of Hu001-2-MMAE, Hu005-46-MMAE, and UC961-MMAE (5 mg/kg, or 2.5 mg/kg) on NCI-N87 gastric cancer tumor model in Example 26 of the present application;
FIG. 61 shows the in vivo antitumor efficacy of Hu001-2-MMAE, and UC961-MMAE (5 mg/kg, or 2.5 mg/kg) on HCC1187 triple-negative breast cancer tumor model in Example 26 of the present application.

### DETAILED DESCRIPTION

The present application is described in further detail below in conjunction with the accompanying drawings, embodiments and examples. It is understood that these embodiments and examples are only for illustrating the present application and are not intended to limit the scope of the present application, and that they are provided for the purpose of enabling a more thorough and comprehensive understanding of the disclosure of the present application. It is also understood that the present application can be realized in many different forms, and is not limited to the embodiments and examples described herein. Those skilled in the art can make various changes or modifications without departing from the connotation of the present application, and the equivalent forms obtained also fall within the protection scope of the present application. Furthermore, in the description below, a great deal of specific detail is given in order to provide a fuller understanding of the present application, and it is understood that the present application can be implemented without one or more of these details.

Unless otherwise defined, all technical and scientific terms used herein have the same meaning as commonly understood by those skilled in the art belonging to the present application. The terms used herein in the specification of the present application are used only for the purpose of describing embodiments and examples and are not intended to limit the present application.

### Terminology

Unless otherwise stated or there is a contradiction, terms or phrases used herein have the following meanings.

As used herein, the terms "and/or" and "or/and" means any one of two or more relevant listed items can be selected, as well as any and all combinations of the relevant listed items, including combinations of any two of the relevant listed items, any more of the relevant listed items, or all of the relevant listed items. It is noted that when at least three items are connected by combinations of at least two conjunctions selected from "and/or" and "or/and", it is understood that, in the present application, the technical solution undoubtedly includes technical solutions that are all connected by "logical AND" and also undoubtedly includes technical solutions that are all connected by "logical OR". For example, "A and/or B" includes three parallel solutions A, B and A+B. For another example, the technical solution "A, and/or B, and/or C, and/or D" includes any one of A, B, C, and D (i.e., technical solutions connected by "logical OR"), as well as any and all combinations of A, B, C, D, i.e., any two or three of A, B, C, and D, and also the combination of A, B, C, and D (i.e., technical solutions connected by "logical AND").

In the present application, unless otherwise specified, "a plurality of", "multiple", "more", "poly-", etc. refer to a quantity greater than or equal to 2. For example, "one or more" means a quantity of one, or more than or equal to two.

As used herein, "a combination thereof", "any combination thereof', "any combination manner thereof', and the like include all suitable combinations of any two or more of the listed items.

Herein, the "suitable" referred to in "suitable combination manner", "suitable manner", "any suitable manner", and the like, is based on being able to implement the technical solutions of the present application, solve the technical problems of the present application, and realize the expected technical effects of the present application.

Herein, "preferred", "preferably", "better", and "appropriate" are only for describing an embodiment or example with better effect, and should not be understood as constituting a limitation on the protection scope of the present application.

In the present application, "further", "even further", "particularly" and the like are for descriptive purposes to indicate differences in content, but should not be construed as limiting the protection scope of the present application.

In the present application, "optionally", and "optional" means either with or without, i.e., either of the two parallel solutions of "with" or "without". If there are more than one "optionally" in a technical solution, each "optionally" is independent of the other, if not otherwise specified and if there are no contradictions or mutual constraints.

In the present application, the terms "first", "second", "third", "fourth", etc., in such as "first aspect", "second aspect", "third aspect", "fourth aspect", etc., are used for descriptive purposes only and are not to be construed as indicating or implying relative importance or quantity, or as implicitly specifying the importance or quantity of the technical features indicated. Moreover, "first", "second", "third", "fourth" and the like only serve the purpose of non-exhaustive enumeration, and it should be understood that they do not constitute a closed-ended limitation of quantity.

In the present application, among the technical features described in an open-ended manner, an open-ended technical solution comprising the enumerated features is included as well as a closed technical solution consisting of the enumerated features.

The present application relates to numerical intervals (i.e., ranges of values), wherein, if not specifically stated, the optional distribution of values is considered continuous within the numerical intervals and includes the two numerical endpoints (i.e., the minimum value and the maximum value) of the range of values, as well as each of the numerical values between these two numerical endpoints. Unless otherwise noted, when the numerical range is directed only to integers within that numerical range, the two endpoint integers of that numerical range are included, as well as each integer between the two endpoints. Herein, it is equivalent to directly enumerate each of the integers. For example, t is an integer selected from 1-10, which means that t is any integer selected from the integer group consisting of 1, 2, 3, 4, 5, 6, 7, 8, 9 and 10. In addition, when multiple ranges are provided to describe the feature or characteristic, the ranges may be combined. In other words, unless otherwise indicated, the ranges disclosed herein should be understood to include any and all sub-ranges subsumed therein.

In the present application, unless otherwise specifically defined, the temperature parameters allow for not only constant temperature treatment, but also treatment within a certain temperature range. It should be understood that the constant temperature treatment allows for fluctuations within the precision range of the instrument, for example, allows for fluctuations within a range such as ±5°C, ±4°C, ±3°C, ±2°C, and ±1°C.

In the present application, %(w/w) and wt% both refer to weight percentage, %(v/v) refers to volume percentage, and %(w/v) refers to mass volume percentage.

All references mentioned in the present application are incorporated herein by reference as if each reference is separately incorporated herein by reference. The references involved in the present application are incorporated by their entireties for all purposes, unless they conflict with the application purpose and/or technical solution of the present application. When references are involved in the present application, the definitions of relevant technical features, terms, nouns, phrases, etc. in the references are also incorporated. When references are involved in the present application, the examples and preferred methods of the cited relevant technical features can also be incorporated into this application by reference, but only to the extent that the present application can be implemented. It is understood that when the cited contents conflict with the description in this application, this application shall prevail or the cited contents shall be modified adaptively according to the description of the present application.

### Preparation of antibody

Fragments of the DNA molecules for the antibodies or antigen-binding fragments thereof of the present application can be obtained by conventional techniques, such as by methods such as PCR amplification or genomic library screening. In addition, the sequences coding the light chain and the heavy chain can be fused together to form a single-chain antibody. Once the relevant sequence information is obtained, the relevant sequence fragments can be obtained in large quantities by recombinant methods. This is usually done by cloning it into a vector, transferring the vector into a cell, and then isolating the relevant sequence fragments from the proliferated host cells by conventional methods.

In addition, the relevant sequence fragments may also be synthesized by artificial synthesis methods, particularly when the fragment length is relatively short. Typically, a long sequence fragment can be obtained by synthesizing multiple small fragments first and then ligating these fragments.

At present, DNA sequences encoding the antibodies (or fragments or derivatives thereof) of the present application may be obtained completely by chemical synthesis. The DNA sequences may then be introduced into various existing DNA molecules (or such as vectors) and cells known in the art. In addition, mutations may also be introduced into the protein sequences of the present application by chemical synthesis.

The present application also relates to vectors comprising the above-mentioned appropriate DNA sequence and an appropriate promoter or control sequence. These vectors can be used to transform appropriate host cells to enable them to express the protein.

The host cells may be prokaryotic cells, such as bacterial cells, lower eukaryotic cells such as yeast celsl, or higher eukaryotic cells, such as mammalian cells. Preferred animal cells include, but are not limited to, CHO-S, and HEK-293 cells.

Typically, the transformed host cells are cultured under conditions suitable for the expression of the antibody of the present application, and then purified using conventional immunoglobulin purification steps such as conventional separation and purification means well known to those skilled in the art, for example, protein A-Sepharose, hydroxyapatite chromatography, gel electrophoresis, dialysis, ion exchange chromatography, hydrophobic chromatography, molecular sieve chromatography, affinity chromatography, or the like, to obtain the antibodies of the present application.

The resulting monoclonal antibodies may be identified by conventional means. For example, the binding specificity of monoclonal antibodies may be determined by immunoprecipitation or by an in vitro binding assay, such as radioimmunoassay (RIA) or enzyme-linked immunoabsorbent assay (ELISA). The binding affinity of the monoclonal antibodies may, for example, be determined by the Scatchard analysis of Munson et al , Anal Biochem, 107: 220 (1980).

The antibodies of the present application may be expressed intracellularly, on the cell membrane, or secreted extracellularly. If necessary, the recombinant proteins may be isolated and purified using various separation methods by exploiting their physical, chemical, and other properties. These methods are well-known to those skilled in the art. Examples of such methods include, but are not limited to, conventional renaturation treatment, treatment with protein precipitating agents (salting-out methods), centrifugation, osmotic lysis, ultrasonication, ultracentrifugation, molecular sieve chromatography (gel filtration), adsorption chromatography, ion-exchange chromatography, high-performance liquid chromatography (HPLC), and other liquid chromatography techniques, and a combination thereof.

### Antibody-drug conjugate (ADC)

The present application also provides an antibody-drug conjugate (ADC). Typically, the antibody-drug conjugate comprises the antibody and an effector molecule, wherein the antibody is coupled to the effector molecule, preferably via chemical coupling. Preferably, the effector molecule is a therapeutically active drug. In addition, the effector molecule may be one or more of a toxic protein, a chemotherapeutic drug, a small-molecule drug or a radionuclide.

The antibody and the effector molecule may be coupled by a coupling agent. Examples of the coupling agent may be any one or more of a non-selective coupling agent, a coupling agent utilizing a carboxyl group, a peptide chain, and a coupling agent utilizing a disulfide bond. The non-selective coupling agent refers to a compound that results in a linkage between an effector molecule and an antibody via a covalent bond, such as glutaraldehyde, etc. The coupling agent utilizing a carboxyl group may be any one or more of cis-aconitic anhydride coupling agents (such as cis-aconitic anhydride) and acyl hydrazone coupling agents (the coupling site is acyl hydrazone).

Certain residues on an antibody (such as Cys or Lys, etc.) are used to link a variety of functional groups, including imaging agents (such as chromophores and fluorophores), diagnostic agents (such as MRI contrast agents and radioisotopes), stabilizers (such as poly(ethylene glycol)) and therapeutic agents. An antibody can be coupled to a functional agent to form an antibody-functional agent conjugate. The functional agent (e.g. a drug, a detection reagent, or a stabilizer) is coupled (covalently linked) to an antibody. The functional agent may be linked to an antibody either directly or indirectly via a linker.

Typical coupling manners suitable for the present application include both K-Lock and C-Lock couplings. In the K-Lock coupling, a drug molecule is coupled to the lysine (K) residue in an antibody sequence; and in the C-Lock coupling, a drug molecule is coupled to the cysteine (C) residue in an antibody sequence.

Antibodies can be coupled to drugs to form antibody-drug conjugates (ADCs). Typically, the ADC contains a linker between the drug and the antibody. The linker can be a cleavable or a non-cleavable linker. Cleavable linkers are generally susceptible to cleavage under intracellular conditions such as at the target site, thereby facilitating the release of the drug from the antibody. Suitable cleavable linkers include, for example, enzyme cleavable linkers including peptide-based linkers cleavable by an intracellular protease, such as lysosomal or endosomal proteases or sugar linkers for example, glucuronide-containing linkers cleavable by a glucuronidase. Peptide-based linkers may include, for example, a dipeptide, such as valine-citrulline (val-cit) phenylalanine-lysine (phe-lys) or valine-alanine (val-ala). Other suitable cleavable linkers include, for example, pH-sensitive linkers (e.g., linkers hydrolyzable at a pH of less than 5.5, such as a hydrazone linker) and linkers cleavable under reducing conditions (e.g., disulfide linkers). Non-cleavable linkers typically release drugs under conditions where the antibody is proteolytically hydrolyzed.

Prior to attachment to the antibody, the linker have a reactive functional group capable of reacting with certain amino acid residues and attachment is achieved via the reactive group. Thiol-specific reactive groups are preferred and include, for example, maleimides; haloacetamides (e.g., iodo-, bromo- or chloro-); haloesters (e.g., iodo-, bromo- or chloro-); halomethyl ketones (e.g., iodo-, bromo- or chloro-); benzylic halides (e.g., iodo-, bromo-, or chloro-); vinyl sulfones; pyridyl disulfides; mercury derivatives such as 3,6-bis-(mercurimethyl)dioxane with counterions such as acetate, chloride or nitrate; and polymethylene dimethyl sulfide thiosulfonates. The linker may include, for example, a maleimide attached to the antibody via a thio-succinimide linkage.

The drug may be any cytotoxic, cytostatic or immunosuppressive drug. In embodiments wherein a linker links the antibody and the drug, the drug has a functional group that can form a bond with the linker. For example, the drug can have an amino group, a carboxyl group , a thiol group, a hydroxyl group, or a ketone group that can form a bond with the linker. In aspects wherein the drug is directly attached to the linker, the drug have a reactive functional group prior to attachment to the antibody.

Useful drugs include, for example, antitubulin agents, DNA minor groove binders, DNA replication inhibitors, alkylating agents, antibiotics, antifolates, antimetabolites, chemotherapy sensitizers, topoisomerase inhibitors, vinca alkaloids, or the like. Particularly useful examples of cytotoxic drugs include, for example, DNA minor groove binders, DNA alkylating agents, and tubulin inhibitors. Exemplary cytotoxic drugs include, for example, auristatins, camptothecins, duocarmycins, etoposides, maytansines and maytansinoids (e.g., DM1 and DM4), taxanes, benzodiazepines or benzodiazepine-containing drugs (e.g., pyrrolo[1,4]-benzodiazepines (PBDs), indolinobenzodiazepines, and oxazolidinobenzodiazepines) and vinca alkaloids, 7-ethyl-10-hydroxycamptothecin (SN38), exatecan and analogs thereof, etc.

In the present application, a drug-linker can be used to form an ADC in a single step. In other embodiments, a bifunctional linker compound can be used to form an ADC in a two-step or multi-step process. For example, the cysteine residue is reacted with the reactive moiety of a linker in a first step, and a functional group on the linker is reacted with a drug to form the ADC in a subsequent step.

Generally, a functional group on the linker is selected for specific reaction with a suitable reactive group on the drug moiety. As a non-limiting example, an azide-based moiety can be used for specific reaction with a reactive alkyne group on the drug moiety. The drug is covalently bound to the linker via 1,3-dipolar cycloaddition between the azide and alkyne. Other useful functional groups include, for example, ketones and aldehydes (suitable for reaction with hydrazides and alkoxyamines); phosphines (suitable for reaction with azides); isocyanates and isothiocyanates (suitable for reaction with amines and alcohols); and activated esters such as N-hydroxysuccinimidyl esters (suitable for reaction with amines and alcohols). These and other linking strategies, as described, for example, in Bioconjugate Techniques, 2nd Ed. (Elsevier), are well known to those of skill in the art. One of skill in the art will appreciate that when a complementary pair of reactive functional groups is chosen for selective reaction of the drug moiety to the linker, each member of the complementary pair can be employed on either the linker or the drug.

### Drug

As used herein, the term "drug" refers broadly to any compound possessing a desired biological activity and contains a reactive functional group available for preparing the conjugate of the present application. The desired biological activity includes activity useful in the diagnosis, cure, mitigation, treatment, or prevention of a disease in human or other animal. Thus, the term "drug" encompasses compounds identified in the official national pharmacopeia as well as e.g., official Homeopathic Pharmacopeia of the United States, or official National Formulary, or any supplements thereof, provided they possess the necessary reactive functional groups. Exemplary drugs are set forth in the Physician's Desk Reference (PDR) and in the Orange Book maintained by the U.S. Food and Drug Administration (FDA). It is understood that, as new drugs are continually discovered and developed, these drugs shall also be incorporated into the "drug" of the drug conjugates of the present application.

### First aspect of the present application

The present application provides an antibody capable of specifically binding to ROR1, the antibody including a heavy chain variable region and a light chain variable region:
the heavy chain variable region including heavy chain complementarity determining regions selected from one or more sets of the following heavy chain complementarity determining regions or derivative fragments thereof:
   i) VH-CDR1 as set forth in SEQ ID NO: 1, VH-CDR2 as set forth in SEQ ID NO: 2, and VH-CDR3 as set forth in SEQ ID NO: 3;
   ii) VH-CDR1 as set forth in SEQ ID NO: 11, VH-CDR2 as set forth in SEQ ID NO: 12, and VH-CDR3 as set forth in SEQ ID NO: 13; and
   iii) VH-CDR1 of SEQ ID NO.19, VH-CDR2 of SEQ ID NO.20, and VH-CDR3 of SEQ ID NO.21; and/or,
the light chain variable region including light chain complementarity determining regions selected from one or more sets of the following light chain complementarity determining regions or derivative fragments thereof:
   i') VL-CDR1 as set forth in SEQ ID NO: 4, VL-CDR2 as set forth in SEQ ID NO: 5, and VL-CDR3 as set forth in SEQ ID NO: 6;
   ii ') VL-CDR1 as set forth in SEQ ID NO: 14, VL-CDR2 as set forth in SEQ ID NO: 15, and VL-CDR3 as set forth in SEQ ID NO: 16; and
   iii ') VL-CDR1 as set forth in SEQ ID NO: 22, VL-CDR2 as set forth in SEQ ID NO: 23, and VL-CDR3 as set forth in SEQ ID NO: 24;
wherein the derivative fragments have no more than 6 amino acid substitutions with respect to the corresponding complementary determining regions and are capable of retaining EC₅₀ of 0.009 nM to 0.05 nM for ROR1-ECD and EC₅₀ of 0.15 nM to 1.1 nM for tumor cell ROR1.

In an embodiment, the derivative fragments are formed by amino acid substitution at no more than 6 sites with respect to the corresponding complementary determining regions, and retains the same biological activity as the corresponding complementary determining regions. For example, the derivative fragments are substituted at 1, 2, 3, 4, 5 or 6 sites with respect to the corresponding complementary determining regions, and one amino acid may be substituted with another amino acid, or one amino acid may be substituted with multiple (e.g., 2) amino acids.

In the present application, a derivative fragment (conservative variant) refers to a polypeptide formed by substituting 1, 2, or 3 amino acids in the amino acid sequence of the antibody of the present application with amino acids of similar or analogous properties. These conservative variant polypeptides are preferably produced by amino acid substitution according to Table 1.

**Table 1**

| Original residue | Representive substitution | Preferred substitution |
|---|---|---|
| Ala (A) | Val; Leu; Ile | Val |
| Arg (R) | Lys; Gln; Asn | Lys |
| Asn (N) | Gln; His; Lys; Arg | Gln |
| Asp (D) | Glu | Glu |
| Cys (C) | Ser | Ser |
| Gln (Q) | Asn | Asn |
| Glu (E) | Asp | Asp |
| Gly (G) | Pro; Ala | Ala |
| His (H) | Asn; Gln; Lys; Arg | Arg |
| Ile (I) | Leu; Val; Met; Ala; Phe | Leu |
| Leu (L) | Ile; Val; Met; Ala; Phe | Ile |
| Lys (K) | Arg; Gln; Asn | Arg |
| Met (M) | Leu; Phe; Ile | Leu |
| Phe (F) | Leu; Val; Ile; Ala; Tyr | Leu |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala | Leu |

In an embodiment, the affinity EC₅₀ of the derivative fragments for ROR1 (such as the extracellular domain of human ROR1 protein, ROR1-ECD) is 0.02 nM to 0.05 nM, optionally 0.02 nM to 0.046 nM, or optionally 0.02 nM to 0.028 nM.

In another preferred example, the affinity EC₅₀ of the derivative fragments for ROR1 on the surface of tumor cells is 0.06 nM to 0.08 nM, optionally 0.06 nM to 0.073 nM, or optionally 0.06 nM to 0.067 nM.

For antibodies as used herein, "antibodies" and "immunoglobulins" are usually heterotetrameric glycoproteins of about 150,000 daltons, which share the same structure features, composed of two identical light (L) chains and two identical heavy (H) chains. Each light chain is linked to a heavy chain by a covalent disulfide bond, while the number of disulfide linkages varies among the heavy chains of different immunoglobulin isotypes. Each heavy and light chain also has regularly spaced intrachain disulfide bridges. Each heavy chain has at one end a variable domain (VH) followed by a number of constant domains. Each light chain has a variable domain at one end (VL) and a constant domain at its other end; the constant domain of the light chain is aligned with the first constant domain of the heavy chain, and the light-chain variable domain is aligned with the variable domain of the heavy chain. Particular amino acid residues form an interface between the light- and heavy- chain variable domains.

As used herein, the term "variable" refers to the fact that certain portions of the variable domains differ extensively in sequence among antibodies, rendering the binding and specificity of each particular antibody for its particular antigen. However, the variability is not evenly distributed throughout the variable domains of antibodies. It is concentrated in three segments called complementarity-determining regions (CDRs) or hypervariable regions both in the light-chain and the heavy-chain variable domains. The more highly conserved portions of variable domains are called the framework region (FR). The variable domains of native heavy and light chains each comprise four FR regions, largely adopting a β-sheet configuration, connected by three CDRs, which form connecting loops, and in some cases forming part of, the β-sheet structure. The CDRs in each chain are held together in close proximity by the FRs and, with the CDRs from the other chain, contribute to the formation of the antigen-binding site of antibodies (see Kabat et al., NIHPubl No.91-3242, Vol. I, pages 647-669 [1991]). The constant domains are not involved directly in binding an antibody to an antigen, but exhibit various effector functions, such as participation of the antibody in antibody dependent cellular cytotoxicity.

The "light chains" of antibodies (immunoglobulins) from any vertebrate species can be assigned to one of two clearly distinct types, called kappa (κ) and lambda (λ), based on the amino acid sequences of their constant domains. Depending on the amino acid sequence of the constant domain of their heavy chains, immunoglobulins can be assigned to different classes. There are five major classes of immunoglobulins: IgA, IgD, IgE, IgG, and IgM, and several of these may be further divided into subclasses (isotypes), e.g., IgGl, IgG2, IgG3, IgG4, IgA, and IgA2. The heavy-chain constant domains that correspond to the different classes of immunoglobulins are called α, δ, e, y, and µ, respectively. The subunit structures and three-dimensional configurations of different classes of immunoglobulins are well known to those skiled in the art.

In general, the antigen-binding properties of an antibody can be described by three specific regions located in the variable regions of the heavy and light chains, referred as complementarity determining regions (CDRs), which are spaced by four framework regions (FRs) whose amino acid sequences are relatively conservative and do not directly participate in binding reactions. These CDRs form a loop structure and are spatially close to each other by the β-sheets formed by the FRs therebetween, and the CDRs on the heavy chain and the CDRs on the corresponding light chain constitute the antigen-binding site of the antibody. The amino acids that constitute the FR or CDR regions can be determined by comparing the amino acid sequences of antibodies of the same type.

The present application includes not only intact antibodies, but also fragments of antibodies with immunological activity or recombinant proteins formed by antibodies and other sequences. Therefore, the present application also includes fragments, derivatives and analogs of the antibodies.

In the present application, the antibodies include those derived from animals (e.g., mouse, rabbit, sheep, cattle, horse, pig, dog, cat, camel, donkey, deer, mink, chicken, duck or goose), chimeric, humanized or fully human antibodies, prepared by techniques well known to those skilled in the art. Recombinant antibodies, such as chimeric and humanized monoclonal antibodies, comprising both human and non-human portions, which can be made using standard recombinant DNA techniques, are useful antibodies. A chimeric antibody is a molecule in which different portions are derived from different animal species, such as those having a variable region derived from a murine monoclonal and human immunoglobulin constant regions. (See, e.g., Cabilly et ah, U.S. Pat. No. 4,816,567; and Boss et ah, U.S. Pat. No. 4,816,397, which are incorporated herein by reference in their entirety.) Humanized antibodies refer to antibody molecules derived from non-human species having one or more complementarity determining regions (CDRs) from the non-human species and framework regions from a human immunoglobulin molecule. (See, e.g., Queen, U.S. Pat. No. 5,585,089, which is incorporated herein by reference in its entirety.) Such chimeric and humanized monoclonal antibodies can be produced by DNA recombinant techniques known in the art. The antibody in the present application can be a chimeric antibody, a humanized antibody, a CDR-grafted and/or modified antibody targeting human ROR1.

In the present application, the antibody may be monospecific, bispecific, trispecific, or of greater multispecificity. At least one of its antigen-binding fragments comprises the heavy chain complementary determining region and the light chain complementary determining region as defined in the present application.

In an embodiment, the heavy chain complementarity determining regions of the heavy chain variable region are selected from one or more sets of the following heavy chain complementarity determining regions:
i) VH-CDR1 having a sequence represented by general formula D-Y-N-Xa1-H, VH-CDR2 having a sequence represented by general formula Y-I-N-P-N-Xa2-Xa3-Xa4-T-Xa5-Y-N-Q-K-F-Xa6-G, and VH-CDR3 having a sequence represented by general formula R-Xa7- Xa8- Xa9- Xa10- Xa11-Xa12-Xa13-D-Xa14; wherein Xa1 is I, M or L, Xa2 is N or H, Xa3 is D or G, Xa4 is A, N or G, Xa5 is S, N or T, Xa6 is Q, K or E, Xa7 is V or G, Xa8 is R or Y, Xa9 is T or G, Xa10 is S or G, Xa11 is S, T or G, Xa12 is G or Y, Xa13 is L, F or AM, and Xa14 is D, F or Y;
ii) VH-CDR1 as set forth in SEQ ID NO: 11, VH-CDR2 as set forth in SEQ ID NO: 12, and VH-CDR3 as set forth in SEQ ID NO: 13; and
iii) VH-CDR1 as set forth in SEQ ID NO: 19, VH-CDR2 having a sequence represented by general formula T-I-S-D-Xb1-G-S-Y-T-Y-Y-P-D-Xb2-Xb3-K-G, and VH-CDR3 as set forth in SEQ ID NO: 21; wherein Xb1 is A or G, Xb2 is S or N, and Xb3 is V or E; and/or
the light chain variable region including light chain complementarity determining regions selected from one or more sets of the following light chain complementarity determining regions:
i') VL-CDR1 having a sequence represented by general formula Xa1'-S-S-Xa2'-Xa3'-I-Xa4'-H-T-N-Xa5'-N-T-Y-L-E, VL-CDR2 having a sequence represented by general formula K-V-Xa6'-N-R-F-S, and VL-CDR3 having a sequence represented by general formula F-Q-G-S-Xa7'-Xa8'- P-Y-T; wherein Xa1' is R, K or T, Xa2' is H or Q, Xa3' is I, N or S, Xa4' is V or L, Xa5' is A or G, Xa6' is F or S, Xa7' is R or L, and Xa8' is F or V;
ii') VL-CDR1 as set forth in SEQ ID NO: 14, VL-CDR2 as set forth in SEQ ID NO: 15, and VL-CDR3 as set forth in SEQ ID NO: 16; and
iii') VL-CDR1 having a sequence represented by general formula K-A-S-Q-S-V-S-F-Xb1'-G-T-S-L-M-H, VL-CDR2 as set forth in SEQ ID NO: 23, and VL-CDR3 as set forth in SEQ ID NO: 24; wherein Xb1' is A or P.

In an embodiment, in the heavy chain complementary determining regions as shown in i):
Xa1 is L, Xa2 is H, Xa3 is D, Xa4 is A or G, Xa5 is S or T, Xa6 is Q, Xa7 is V, Xa8 is R, Xa9 is T, Xa10 is G, Xa11 is T, Xa12 is G, Xa13 is L or F, and Xa14 is D or Y; or,
Xa1 is I or M, Xa2 is N, Xa3 is G, Xa4 is N or G, Xa5 is S, N or T, Xa6 is K or E, Xa7 is G, Xa8 is Y, Xa9 is G, Xa10 is S, Xa11 is S or G, Xa12 is Y, Xa13 is AM, and Xa14 is F or Y

In an embodiment, in the light chain complementary determining regions as shown in i):
Xa1' is R or K, Xa2' is H, Xa3' is N, Xa4' is V or L, Xa5' is A or G, Xa6' is S, Xa7' is R, and Xa8' is F; or,
Xa1' is R or T, Xa2' is H or Q, Xa3' is I or S, Xa4' is V, Xa5' is G, Xa6' is F or S, Xa7' is R or L, and Xa8' is F or V.

In an embodiment, the antibody has one or more of the following combinations of heavy chain complementary determining regions and light chain complementary determining regions:
Combination 1: the heavy chain complementary determining regions as shown in i) or derivative fragments thereof, and the light chain complementary determining region as shown in i') or derivative fragments thereof;
Combination 2: the heavy chain complementary determining regions as shown in ii) or derivative fragments thereof, and the light chain complementary determining region as shown in ii') or derivative fragments thereof;
Combination 3: the heavy chain complementary determining regions as shown in iii) or derivative fragments thereof, and the light chain complementary determining region as shown in iii') or derivative fragments thereof.

In an embodiment, the antibody has one or more of the following technical features:
the combination 1 is selected from one or more of the following combinations:
   Combination 1-1: VH-CDR1 as set forth in SEQ ID NO: 1, VH-CDR2 as set forth in SEQ ID NO: 2, VH-CDR3 as set forth in SEQ ID NO: 3, VL-CDR1 as set forth in SEQ ID NO: 4, VL-CDR2 as set forth in SEQ ID NO: 5, and VL-CDR3 as set forth in SEQ ID NO: 6;
   Combination 1-2: VH-CDR1 as set forth in SEQ ID NO: 35, VH-CDR2 as set forth in SEQ ID NO: 36, VH-CDR3 as set forth in SEQ ID NO: 37, VL-CDR1 as set forth in SEQ ID NO: 38, VL-CDR2 as set forth in SEQ ID NO: 39, and VL-CDR3 as set forth in SEQ ID NO: 40;
   Combination 1-3: VH-CDR1 as set forth in SEQ ID NO: 51, VH-CDR2 as set forth in SEQ ID NO: 52, VH-CDR3 as set forth in SEQ ID NO: 53, VL-CDR1 as set forth in SEQ ID NO: 54, VL-CDR2 as set forth in SEQ ID NO: 55, and VL-CDR3 as set forth in SEQ ID NO: 56;
   Combination 1-4: VH-CDR1 as set forth in SEQ ID NO: 59, VH-CDR2 as set forth in SEQ ID NO: 60, VH-CDR3 as set forth in SEQ ID NO: 61, VL-CDR1 as set forth in SEQ ID NO: 62, VL-CDR2 as set forth in SEQ ID NO: 63, and VL-CDR3 as set forth in SEQ ID NO: 64;
   Combination 1-5: VH-CDR1 as set forth in SEQ ID NO: 67, VH-CDR2 as set forth in SEQ ID NO: 68, VH-CDR3 as set forth in SEQ ID NO: 69, VL-CDR1 as set forth in SEQ ID NO: 70, VL-CDR2 as set forth in SEQ ID NO: 71, and VL-CDR3 as set forth in SEQ ID NO: 72;
   Combination 1-6: VH-CDR1 as set forth in SEQ ID NO: 75, VH-CDR2 as set forth in SEQ ID NO: 76, VH-CDR3 as set forth in SEQ ID NO: 77, VL-CDR1 as set forth in SEQ ID NO: 78, VL-CDR2 as set forth in SEQ ID NO: 79, and VL-CDR3 as set forth in SEQ ID NO: 80;
   Combination 1-7: VH-CDR1 as set forth in SEQ ID NO: 1, VH-CDR2 as set forth in SEQ ID NO: 83, VH-CDR3 as set forth in SEQ ID NO: 3, VL-CDR1 as set forth in SEQ ID NO: 84, VL-CDR2 as set forth in SEQ ID NO: 5, and VL-CDR3 as set forth in SEQ ID NO: 6; and
the combination 3 is selected from one or more of the following combinations:
   Combination 3-1: VH-CDR1 as set forth in SEQ ID NO: 43, VH -CDR2 as set forth in SEQ ID NO: 44, VH-CDR3 as set forth in SEQ ID NO: 45, VL-CDR1 as set forth in SEQ ID NO: 46, VL-CDR2 as set forth in SEQ ID NO: 47, and VL-CDR3 as set forth in SEQ ID NO: 48;
   Combination 3-2: VH-CDR1 as set forth in SEQ ID NO: 19, VH-CDR2 as set forth in SEQ ID NO: 20, VH-CDR3 as set forth in SEQ ID NO: 21, VL-CDR1 as set forth in SEQ ID NO: 22, VL-CDR2 as set forth in SEQ ID NO: 23, and VL-CDR3 as set forth in SEQ ID NO: 24;
   Combination 3-3: VH-CDR1 as set forth in SEQ ID NO: 19, VH-CDR2 as set forth in SEQ ID NO: 85, VH-CDR3 as set forth in SEQ ID NO: 21, VL-CDR1 as set forth in SEQ ID NO: 22, VL-CDR2 as set forth in SEQ ID NO: 23, and VL-CDR3 as set forth in SEQ ID NO: 24.

In an embodiment, the antibody has one or more of the following technical features:
(1) the antibody having a heavy chain constant region and a light chain constant region independently derived from a species selected from human, mouse, rabbit, sheep, cattle, horse, pig, dog, cat, camel, donkey, deer, mink, chicken, duck or goose;
(2) the constant region of the antibody has a sequence of the constant region selected from any one of IgG1, IgG2, IgG3, IgG4, IgA, IgM, IgE and IgD; and,
(3) the constant region of the antibody has a sequence of the constant region selected from any one of κ light chain constant region and λ light chain constant region.

In an embodiment, the antibody has one or more combinations selected from the following combinations of a heavy chain variable region and a light chain variable region:
a heavy chain variable region as set forth in SEQ ID NO: 7 and a light chain variable region as set forth in SEQ ID NO: 9;
a heavy chain variable region as set forth in SEQ ID NO: 41 and a light chain variable region as set forth in SEQ ID NO: 42;
a heavy chain variable region as set forth in SEQ ID NO: 49 and a light chain variable region as set forth in SEQ ID NO: 50;
a heavy chain variable region as set forth in SEQ ID NO: 17 and a light chain variable region as set forth in SEQ ID NO: 18;
a heavy chain variable region as set forth in SEQ ID NO: 25 and a light chain variable region as set forth in SEQ ID NO: 27;
a heavy chain variable region as set forth in SEQ ID NO: 57 and a light chain variable region as set forth in SEQ ID NO: 58;
a heavy chain variable region as set forth in SEQ ID NO: 65 and a light chain variable region as set forth in SEQ ID NO: 66;
a heavy chain variable region as set forth in SEQ ID NO: 73 and a light chain variable region as set forth in SEQ ID NO: 74;
a heavy chain variable region as set forth in SEQ ID NO: 81 and a light chain variable region as set forth in SEQ ID NO: 82;
a heavy chain variable region as set forth in SEQ ID NO: 8 and a light chain variable region as set forth in SEQ ID NO: 10;
a heavy chain variable region as set forth in SEQ ID NO: 26 and a light chain variable region as set forth in SEQ ID NO: 27;
a heavy chain variable region as set forth in SEQ ID NO: 28 and a light chain variable region as set forth in SEQ ID NO: 33;
a heavy chain variable region as set forth in SEQ ID NO: 28 and a light chain variable region as set forth in SEQ ID NO: 34;
a heavy chain variable region as set forth in SEQ ID NO: 29 and a light chain variable region as set forth in SEQ ID NO: 33;
a heavy chain variable region as set forth in SEQ ID NO: 29 and a light chain variable region as set forth in SEQ ID NO: 34;
a heavy chain variable region as set forth in SEQ ID NO: 30 and a light chain variable region as set forth in SEQ ID NO: 33;
a heavy chain variable region as set forth in SEQ ID NO: 31 and a light chain variable region as set forth in SEQ ID NO: 33;
a heavy chain variable region as set forth in SEQ ID NO: 31 and a light chain variable region as set forth in SEQ ID NO: 34;
a heavy chain variable region as set forth in SEQ ID NO: 32 and a light chain variable region as set forth in SEQ ID NO: 33;
a heavy chain variable region as set forth in SEQ ID NO: 32 and a light chain variable region as set forth in SEQ ID NO: 34;
a heavy chain variable region as set forth in SEQ ID NO: 93 and a light chain variable region as set forth in SEQ ID NO: 101;
a heavy chain variable region as set forth in SEQ ID NO: 94 and a light chain variable region as set forth in SEQ ID NO: 101;
a heavy chain variable region as set forth in SEQ ID NO: 95 and a light chain variable region as set forth in SEQ ID NO: 101;
a heavy chain variable region as set forth in SEQ ID NO: 96 and a light chain variable region as set forth in SEQ ID NO: 101;
a heavy chain variable region as set forth in SEQ ID NO: 97 and a light chain variable region as set forth in SEQ ID NO: 101;
a heavy chain variable region as set forth in SEQ ID NO: 98 and a light chain variable region as set forth in SEQ ID NO: 101;
a heavy chain variable region as set forth in SEQ ID NO: 99 and a light chain variable region as set forth in SEQ ID NO: 101; and
A heavy chain variable region as set forth in SEQ ID NO: 100 and a light chain variable region as set forth in SEQ ID NO: 101.

In an embodiment, the antibody is an original mouse antibody mAb001, mAb002, mAb003, mAb004, mAb005, mAb006, mAb007, mAb008, or mAb009.

In an embodiment, the antibody is a human-mouse chimeric antibody mAb001c, mAb001c_v1, mAb002c, mAb003c, mAb004c, mAb005c, mAb005c_v1, mAb006c, mAb007c, mAb008c, or mAb009c.

In an embodiment, the antibody is a humanized antibody Hu001-2, Hu001-3, Hu001-5, Hu001-6, Hu001-8, Hu001-11, Hu001-12, Hu001-14, Hu001-14, Hu001-15, Hu005-35, Hu005-40, Hu005-41, Hu005-42, Hu005-43, Hu005-44, Hu005-45, or Hu005-46.

The antibody provided in the first aspect of the present application has one or more features selected from the following group:
a) inhibiting the biological activity of ROR1;
b) specifically binding to ROR1-ECD protein or ROR1 on the surface of tumor cells;
c) binding to ROR1on tumor cell so as to rapidly mediate target endocytosis;
d) inhibiting tumor cell migration or metastasis;
f) inhibiting tumor growth and improving the anti-tumor efficacy of combination therapy;
g) being able to reduce the emergence of anti-tumor therapy resistance.

The present application provides three major categories of antibodies targeting ROR1 with high specificity and high affinity, which can be used in combination and can be used for constructing CAR constructs, recombinant immune cells containing CAR constructs, antibody-drug conjugates, etc., as well as for (a) preparing a detection reagent, a detection plate or a kit; and/or (b) preparing a medicament for preventing and/or treating ROR1-related diseases.

### Second aspect of the present application

The present application provides a recombinant protein, comprising:
one or more of the heavy chain complementarity determining regions and light chain complementarity determining regions as defined in the first aspect; and
a tag fragment that facilitates the expression and/or purification of the heavy chain complementary determining regions and the light chain complementary determining regions.

In some embodiments of the present application, the recombinant protein further includes the heavy chain constant region or the light chain constant region as defined in the first aspect, or a combination thereof.

In the present application, the tag sequence includes but is not limited to 6His tag.

In the present application, the recombinant protein (or polypeptide) includes but is not limited to a fusion protein.

In the present application, the recombinant protein may be a monomer, a dimer, or a polymer.

### Third aspect of the present application

The present application provides a CAR construct, wherein the CAR construct has a scFV domain having the heavy chain variable region and the light chain variable region as defined in the first aspect.

It can be understood that the scFV domain is a specific binding region of ROR1.

### Fourth aspect of the present application

The present application provides a nucleic acid, wherein the nucleic acid encodes the antibody as desribed in the first aspect, the recombinant protein as described in the second aspect, or the CAR construct as described in the third aspect.

### Fifth aspect of the present application

The present application provides a vector, wherein the vector contains the nucleic acid as described in the fourth aspect.

In the present application, the vector includes but is not limited to a bacterial plasmid, a bacteriophage, a yeast plasmid, a plant cell virus, a mammalian cell virus such as adenovirus or retrovirus, or a combination thereof.

### Sixth aspect of the present application

The present application provides a host cell, wherein the host cell contains the nucleic acid as described in the fourth aspect or the vector as described in the fifth aspect.

In the present application, the nucleic acid can be integrated into a genome of the host cell.

In the present application, the host cell includes but are not limited to CHO cells, COS cells, NSO cells, HeLa cells, BHK celsl or HEK293 cells.

### Seventh aspect of the present application

The present application provides a method for preparing the antibody as described in the first aspect, the recombinant protein as described in the second aspect, or the CAR construct as described in the third aspect, the method comprising:
culturing the host cell as described in the sixth aspect, and isolating the antibody or antigen-binding fragment thereof, recombinant protein or CAR construct from the obtained culture.

It is understood that the culturing is carried out under appropriate culture conditions, including but not limited to appropriate culture medium, appropriate temperature, appropriate period, etc.

With reference to the definition in the first aspect, the antibody of the present application may be a chimeric antibody or a humanized antibody.

In some embodiments, the method for preparing the chimeric antibody comprises:
cloning the nucleotide as described in the fourth aspect of the present application into an expression vector containing a human antibody constant region, and transfecting the expression vector into animal cells to express the chimeric antibody.

In some embodiments, the method for preparing the humanized antibody comprises:
introducing the nucleic acid as described in the fourth aspect of the present application into a template containing the FR region of a human antibody, cloning the template into an expression vector containing a human antibody constant region, and transfecting the expression vector into animal cells to express the humanized antibody.

### Eighth aspect of the present application

The present application provides a recombinant immune cell, wherein the recombinant immune cell expresses the exogenous CAR construct as described in the third aspect.

In the present application, the immune cell may be selected from the group comprising but not limited to NK cells and T cells.

In the present application, the immune cell may be derived from human or other mammals (such as mouce).

### Ninth aspect of the present application

The present application provides use of the antibody or antigen-binding fragment thereof as described in the first aspect, the recombinant protein as described in the second aspect, the CAR construct as described in the third aspect, or the recombinant immune cell as described in the eighth aspect in manufacturing a medicament for treating drug-resistant tumors, a medicament for treating ROR1-related disease or a product for diagnosing ROR1-related disease.

In this application, treatment includes prevention, management, auxiliary treatment, etc.

In some embodiments of the present application, the ROR1-related disease is a ROR1 high-expressing tumor, autoimmune disease, metabolism-related disease or infectious disease.

In the present application, the tumor may be a solid tumor or a blood cancer.

In the present application, the ROR1 high-expressing tumor refers to a ratio (L1/L0) of ROR1 transcript and/or protein level in tumor tissue (L1) to ROR1 transcript and/or protein level in normal tissue (L2) greater than or equal to 2, preferably greater than or equal to 3.

In some embodiments of the present application, the tumor is breast cancer, lung cancer, pancreatic cancer, ovarian cancer, prostate cancer, colorectal cancer, gastric cancer, liver cancer, esophageal cancer, kidney cancer, brain glioma, bladder cancer, prostate cancer, endometrial cancer, cervical cancer, leukemia, lymphoma, bone marrow cancer or angiosarcoma.

The inflammation is rheumarthritis, osteoarthritis, gout, Reiter's syndrome, psoriatic arthropathy, tuberculous arthritis, glomerulonephritis, systemic lupus erythematosus, Crohn's disease, ulcerative colitis, acute lung injury, chronic obstructive pulmonary disease or idiopathic pulmonary fibrosis.

The autoimmune disease is rheumatoid arthritis, ankylosing spondylitis, systemic lupus erythematosus, Sjögren's syndrome or systemic vasculitis, ulcerative colitis, type I diabetes, psoriasis, or multiple sclerosis.

The metabolic-related disease is diabetes, diet-induced obesity or fat inflammation.

The infectious disease is a disease caused by bacterial infection or a disease caused by viral infection.

In the present application, the medicament may be in the form of an antibody-drug conjugate or a pharmaceutical composition, which is used for treating tumors with high expression of ROR1, tumor migration or tumor resistance. In some embodiments, the medicament may be used for:
(a) specifically binding to to ROR1 on the tumor cells and/or the immune/stromal cells in tumor microenvironment;
(b) inhibiting the biological function of overactivated ROR1 in tumors/tumor microenvironment;
(c) inhibiting tumor cell migration or metastasis;
(d) inhibiting tumor growth and improving the anti-tumor efficacy of combination therapy;
(e) antibody-dependent cell- mediated cytotoxicity (ADCC).

In some embodiments, the tumor resistance includes: resistance to tumor immunotherapy drugs, resistance to tumor targeted therapy drugs, resistance to conventional tumor chemotherapy, and insusceptibility to radiotherapy.

In some embodiments, the product for diagnosing ROR1-related disease is a diagnostic reagent, a test strip, a test plate or a test kit. The test reagent, test plate or test kit may be used for:
detecting ROR1 protein in a sample;
detecting endogenous ROR1 protein in tumor cells;
detecting tumor cells expressing ROR1 protein.

In the present application, the antibody-drug conjugate provided in the following tenth aspect or the pharmaceutical composition provided in the eleventh aspect can target a specific cell population and bind to a specific cell surface protein (antigen), thereby releasing the drug in an active form into the cell through endocytosis of the conjugate or drug penetration. Therefore, the antibody-drug conjugate or pharmaceutical composition of the present application can be used to treat target diseases and can be administered to subjects in a therapeutically effective amount via a suitable route. Subjects in need of treatment may be patients who are at risk or suspected of having a disease related to the activity or expression of a specific antigen. Such patients can be identified by routine physical examinations.

Conventional methods, known to those skilled in the art of medicine, can be used to administer the antibody-drug conjugate or the pharmaceutical composition to subjects. The mode of administration depends on the type or the site of disease to be treated. For exaample, the antibody-drug conjugate or the pharmaceutical composition can be administered orally, parenterally, by inhalation spray, topically, rectally, nasally, buccally, vaginally or via implantation. The term "parenteral" as used herein includes subcutaneous, intravenous, intramuscular, intraarticular, intraarterial, intrasynovial, intrasternal, intrathecal, intralesional or intracranial injection, or infusion techniques.

### Tenth aspect of the present application

The present application provides an antibody-drug conjugate, wherein the antibody-drug conjugate includes:
an antibody selected from the antibody as defined in the first aspect; and,
a conjugant coupled to the antibody.

In the present application, the conjugant may be selected from the group comprising but not limited to a detectable label, a cytotoxic drug, a cytokine, a radionuclide, and an enzyme.

The term "cytotoxic drug" refers to a substance that inhibits or prevents the expression activity of cells, function of cells and/or causes destruction of cells. The term includes radioactive isotopes, chemotherapeutic agents, and toxins such as small molecule toxins or enzymatically active toxins of bacterial, fungal, plant or animal origin, including fragments and/or variants thereof, including but not limited to auristatins (e.g., auristatin E, auristatin F, MMAE and MMAF), auromycins, maytansinoids, ricin, ricin A-chain, combrestatin, duocarmycins, dolostatins, doxorubicin, daunorubicin, taxol, cispiatin, cc1065, ethidium bromide, mitomycin, etoposide, tenoposide, vincristine, vinblastine, colchicine, dihydroxy anthracin dione, actinomycin, diphtheria toxin, Pseudomonas exotoxin (PE) A, PE40, abrin, abrin A chain, modeccin A chain, alpha-sarcin, gelonin, mitogellin, retstrictocin, phenomycin, enomycin, curicin, crotin, calicheamicin, Sapaonaria officinalis inhibitor, and glucocorticoid and other chemotherapeutic agents, as well as radioisotopes such as At211, I131, I125, Y90, Re186, Re188, Sm153, Bi212 or 213, P32 and radioactive isotopes of Lu including Lu177. Antibodies may also be coupled to an anti-cancer pro-drug activating enzyme capable of converting the pro-drug to its active form.

Optionally, the cytotoxic drug is selected from compounds with high cytotoxicity, preferably monomethyl auristatin, calicheamicin, maytansinoids, or a combination thereof; more preferably selected from: monomethyl auristatin-E (MMAE), monomethyl auristatin-D (MMAD), monomethyl auristatin-F (MMAF), or a combination thereof.

Optionally, the cytotoxic drug is selected from cytotoxic drugs used for cancer treatment, or proteins or polypeptides with desired biological activity, such as a toxin, for example abrin, ricin A, Pseudomonas exotoxin, and diphtheria toxin; other suitable proteins include tumor necrosis factor, α-interferon, β-interferon, neurogenic growth factor, platelet-derived growth factor, tissue-type plasminogen activator, and biological response modifiers, such as lymphokines, interleukin-1 (IL-1), interleukin-2 (IL-2), interleukin-6 (IL-6), granulocyte macrophage colony stimulating factor (GM-CSF), granulocyte colony stimulating factor, or other growth factors.

A cytotoxic drug is maytansine or maytansinoids. Maytansine inhibits cell proliferation by inhibiting the formation of microtubules from tubulins. Maytansinoids are derivatives of maytansine. Both maytansine and maytansinoids are highly cytotoxic, but they have a greatly limited clinical use in cancer therapy due to poor selectivity for tumors. However, a high cytotoxicity enables them to be preferred drug moieties in antibody-drug conjugates. The structureof deacetyl-maytansine is provided below.

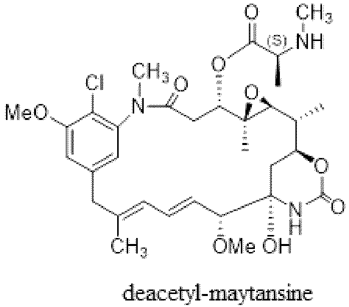

A cytotoxic drug is auristatins. Auristatins are synthetic analogues of Dolastatin 10, which is a polypeptide isolated from marine mollusk Aplysia and found to have biological activity. Dolastatin 10 inhibits tubulin polymerization by binding to tubulin at the same domain as vincristine. Dolastitin 10, auristatin PE, and auristatin E are all linear peptides containing four amino acids, three of which are unique to the dolastatin class compounds, and a C-terminal amide group. Two representative auristatins, monomethyl auristatin E (MMAE) and monomethyl auristatin F (MMAF), are preferred drug moiety candidates for ADCs. 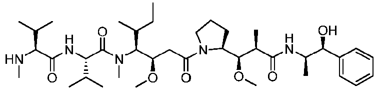 Monomethyl Auristatin E(MMAE) 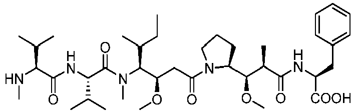 Monomethyl Auristatin F(MMAF) 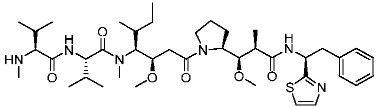 Monomethyl Dolastatin 10(MMAD)

A cytotoxic drug is pyrrolo [2,1-c][1,4] benzodiazepines (PBDs) or PBD dimers. PBDs are a class of natural products produced by Streptomyces species with a unique characteristic of forming non-distortive covalent adducts in DNA minor groove, specifically at a purine-guanine-purine sequence. There is a growing interest in using PBDs as part of a small-molecule strategy for targeting and locking DNA sequences and also as novel anticancer and antibacterial agents. The dimer, which is obtained by linking two PBD units together through their C8/C8'-hydroxyl functionalities via a flexible alkylene linker, has increased biological activity. PBD dimers are believed to lead to sequence-selective DNA damage such as interstrand cross-linking of the inverted 5'-Pu-GATC-Py-3' sequence, which mainly accounts for their biological activity. These compounds have been shown to be highly potent cytotoxic agents and good drug candidates for ADCs.

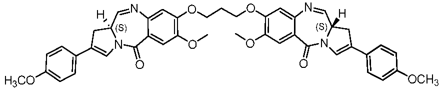

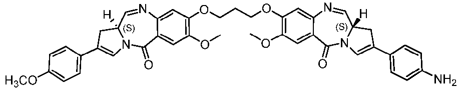

### PBD dimers

A cytotoxic drug is PNU-159682 derivatives. PNU-159682 is a main active metabolite of Nemorubicin in human liver microsomes, with an activity 3000 times larger compared to MMDX or doxorubicin.

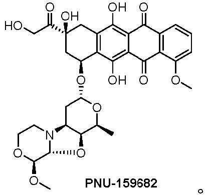

The cytotoxic drug is not limited to above-mentioned categories and include all those could be used in ADCs, especially those capable of coordinating with a linker through an amide bond of the linker, such as through a basic amine (primary or second amine) possessed.

In the present application, the cytotoxic drug may be classed into a microtubule-targeted drug, a DNA-targeted drug or a topoisomerase inhibitor.

In the present application, the antibody can be coupled to the conjugant via a chemical linkage or a linker (but not limited thereto).

In the present application, the linker comprises a sulfydryl-specific active reactive group. The sulfydryl-specific active reactive group may be selected from the group comprising but not limited to 4-(N-maleimidomethyl)cyclohexane-1-carboxylic acid succinate, maleimidocaproyl, 6-maleimidocaproyl-valine-citrulline-p-aminobenzyloxycarbonyl and disubstituted maleimide linkers. Optionally, the linker is selected from 6-maleimidocaproyl-valine-citrulline-p-aminobenzyloxycarbonyl (MC-val-cit-PAB), 6-maleimidocaproyl-alanine-phenylalanine-p-aminobenzyloxycarbonyl (MC-ala-phe-PAB), maleimidopropionyl-valine-citrulline-p-aminobenzyloxycarbonyl (MP-val-cit-PAB), maleimidopropionyl-alanine-phenylalanine-p-aminobenzyloxycarbonyl (MP-ala-phe-PAB), N-succinimidyl 4-(2-pyridylthio)pentanoate (SPP), N-succinimidyl 4-(N-maleimidomethyl)cyclohexane-1-carboxylate (SMCC), 4-(2-pyridyldithio)butanoic acid N-hydrosuccinimide ester (SPDB) or N-succinimidyl (4-iodo-acetyl)aminobenzoate (SIAB). In an embodiment, the linker is 6-maleimidocaproyl-valine-citrulline-p-aminobenzyloxycarbonyl (MC-val-cit-PAB).

In the present application, the antibody-drug conjugate has a structure as shown in the following molecular formula: 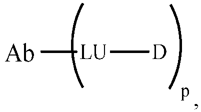 wherein
Ab is the antibody,
LU is a linker,
D is a cytotoxic drug.

Optionally, p is selected from 1-10, for example, 1, 2, 3, 4, 5, 6, 7, 8, 9, 10. In some embodiments, p is a value from 1 to 8.

The embodiments of the present application further provides a method for preparing an ADC, which may further comprise: binding an antibody to a drug-linker compound under conditions sufficient to form an antibody-drug conjugate (ADC). In certain embodiments, the method according to the present application comprises: binding an antibody to a bifunctional linker compound under conditions sufficient to form an antibody-linker conjugate. In these embodiments, the method according to the present invention further comprises: binding the antibody-linker conjugate toa drug moiety under conditions sufficient to covalently link the drug moiety to the antibody via the linker.

The antibody-drug conjugate of the present application can be delivered and administered by conventional methods in the art. For example, it can be introduced into cells by using liposomes, hydrogels, cyclodextrins, biodegradable nanocapsules, or bioadhesive microspheres.

### Eleventh aspect of the present application

The present application provides a pharmaceutical composition, comprising:
the antibody as described in the first aspect, the recombinant protein as described in the second aspect, the CAR construct as described in the third aspect, or the recombinant immune cell as described in the eighth aspect, the antibody-drug conjugate as described in the tenth aspect, or a combination thereof; and
a pharmaceutically acceptable carrier.

In some embodiments, the administration of the pharmaceutical composition includes, but is not limited to, oral, rectal administration, parenteral (intravenous, intramuscular or subcutaneous) injection, topical administration, and inhalation.

In some embodiments, the pharmaceutical composition can be administered orally, by enema or parenterally.

In some embodiments, the administration regimenof the pharmaceutical composition can be intermittent administration, periodic administration, continuous administration or long-term administration.

Solid dosage forms for oral administration include capsules, tablets, pills, powders, and granules. In such solid dosage forms, the active ingredient is admixed with at least one common inert excipient (or carrier) such as sodium citrate or dicalcium phosphate or (a) fillers or extenders, for example, starches, lactose, sucrose, glucose, mannitol, and silicic acid; (b) binders, for example, hydroxymethyl cellulose, alignates, gelatin, polyvinylpyrrolidone, sucrose, and acacia; (c) humectants, for example, glycerol; (d) disintegrating agents, for example, agar, calcium carbonate, potato or tapioca starch, alginic acid, certain complex silicates, and sodium carbonate; (e) solution retarders, for example paraffin; (f) absorption accelerators, for example, quaternary ammonium compounds; (g) wetting agents, for example, cetyl alcohol and glycerol monostearate; (h) adsorbents, for example, kaolin; and (i) lubricants, for example, talc, calcium stearate, magnesium stearate, solid polyethylene glycols, sodium lauryl sulfate, or mixtures thereof. In the case of capsules, tablets, and pills, the dosage forms may also comprise buffering agents . Solid dosage forms such as tablets, dragees, capsules, pills, and granules can be prepared with coatings and shells, such as enteric coatings and others well-known in the art. They may contain opacifying agents, and can also be of such composition that they release the active ingredient or compounds in a certain part of the intestinal tract in a delayed manner. Examples of embedding components which can be used are polymeric substances and waxes. The active ingredients can also be in micro-encapsulated form, if appropriate, with one or more of the above-mentioned excipients .

Liquid dosage forms for oral administration include pharmaceutically acceptable emulsions, solutions, suspensions, syrups, and elixirs. In addition to the active ingredients, the liquid dosage forms may contain inert diluents commonly used in the art, such as water or other solvents, solubilizing agents and emulsifiers, for example, ethyl alcohol, isopropyl alcohol, ethyl carbonate, ethyl acetate, propyleneglycol, 1, 3-butyleneglycol, dimethylformamide, oils, in particular, cottonseed oil, groundnut oil, corn germ oil, olive oil, castor oil and sesame oil, or mixtures of these substances, and the like. Besides such inert diluents, the composition can also include adjuvants, such as wetting agents, emulsifying and suspending agents, sweetening, flavoring, and perfuming agents. Suspensions may contain suspending agents, in particular, for example, ethoxylated isostearyl alcohols, polyoxyethylene sorbitol, sorbitan esters, microcrystalline cellulose, aluminum metahydroxide,agar, or mixtures of these substances.

Liquid dosage forms for parenteral injection may include physiologically acceptable sterile aqueous or anhydrous solutions, dispersions, suspensions or emulsions, and sterile powders for redissolving into sterile injectable solutions or dispersions. Suitable aqueous or non-aqueous carriers, diluents, solvents or excipients include vegetable oils, dimethylacetamide, dimethylformamide, ethyl lactate, ethyl carbonate, isopropyl myristate, water, ethanol, polyols, and suitable mixtures thereof. For intravenous injection, water-soluble antibodies can be administered by drip method, whereby administrating a pharmaceutical preparation containing the antibody and a physiologically acceptable excipient by infusion. Physiologically acceptable excipients may include, for example, 5% glucose, 0.9% saline, Ringer's solution or other suitable excipients. For intramuscular formulations, for example, sterile formulations in the form of a suitable soluble salt of an antibody, pharmaceutical excipient such as water for injection, 0.9% saline, or 5% glucose solution can be used for dissolution and administration.

Dosage forms for topical administration include ointments, powders, patches, sprays, and inhalants.

The pharmaceutical composition of the present application is prepared with active component admixed under sterile conditions with a pharmaceutically acceptable carrier and any preservatives, buffers, or propellants as may be required.

The "active ingredient" in the above-mentioned pharmaceutical preparation of this aspect refers to the ingredient in the pharmaceutical composition that can play the role of "drug". It is understood that the drugs of the embodiments of the present application can be admixed with different pharmaceutically acceptable carriers to prepare suitable clinical dosage forms, which include but are not limited to the dosage forms as described above.

### Twelfth aspect of the present application

The present application provides a product for diagnosing ROR1-related disease, wherein the product is a test plate coated with the antibody or antigen-binding fragment thereof as described in the first aspect, the recombinant protein as described in the second aspect, the CAR construct as described in the third aspect, or the recombinant immune cell as described in the eighth aspect, or a combination thereof.

The product is a test kit comprising a primary antibody and a secondary antibody for detecting ROR1, wherein the primary antibody is the antibody as defined in the first aspect.

In the present application, the test plate may be, but is not limited to, an immunochromatographic plate, and may also be a test paper. The test plate may include a substrate (base plate, base pad or support plate) and chromatography paper, etc.

In the present application, the test kit may include at least two receptacles, one for placing the antibody as described in the first aspect; and the other for placing a secondary antibody against the antibody.

In some embodiments, the test kit may further include instructions for use, a buffer, or the like.

### Thirteenth aspect of the present application

The present application provides a method for detecting ROR1, comprising:
providing a sample to be tested, and
mixing the sample to be tested with the antibody as described in the first aspect for reaction, detecting the reaction product, and determining the presence of ROR1 in the sample to be tested based on the test result.

In some embodiments of the present application, determining the presence of ROR1 in the sample to be tested based on the test result includes:
when the antigen-antibody complex is detected in the reaction product, it is determined that the sample to be tested contains ROR1; or
when no antigen-antibody complex is detected in the reaction product, it is determined that the sample to be tested contains no ROR1.

In the present application, the detection method may be for diagnostic purposes or non-diagnostic purposes.

In the present application, the sample to be tested (sample) includes cells, tissue samples and biopsy specimens. The term "biopsy" used in the present application should include all types of biopsies known to those skilled in the art. Therefore, the biopsy used in the present application can include, for example, an excision sample of a tumor, a tissue sample prepared by endoscopic methods or puncture or needle biopsy of an organ.

The sample to be tested used in this application includes fixed or preserved cell or tissue samples.

### Fourteenth aspect of the present application

The present application provides a method for treating a ROR1-related disease, the method comprising:
administering to a subject an effective amount of the antibody as described in the first aspect of the present application, the recombinant immune cell as described in the eighth aspect, the antibody-drug conjugate as described in the tenth aspect, the pharmaceutical composition as described in the eleventh aspect, or a combination thereof.

The ROR1-related disease refers to the ninth aspect.

In some embodiments, the method further comprises: administering other drugs or treatment methods to the subject for combined treatment.

In some embodiments, the other drugs or treatment methods include, but are not limited to: anti-tumor immunotherapy drugs, tumor targeted drugs, tumor chemotherapy drugs, and tumor radiotherapy.

In some embodiments, the anti-tumor immunotherapy drugs include but are not limited to: PD-1, and PD-L1 monoclonal antibodies.

In some embodiments, the administration includes, but is not limited to, oral, rectal administration, parenteral (intravenous, intramuscular or subcutaneous) injection, topical administration, and inhalation.

In the present application, the "subject" is an animal, which may be a human or other non-human mammal. The subjects include, but are not limited to, drug consumers and patients with diseases, disorders and/or symptoms. The term "mammal" in the present application mainly refers to warm-blooded vertebrate mammals, including but not limited to: cats, dogs, rabbits, bears, foxes, wolves, monkeys, deer, mice (such as rats, mice), pigs, cattle, sheep, horses, humans, etc., preferably primates, more preferably humans.

### Fifteenth aspect of the present application

The present application provides a method for inhibiting tumor cell growth and migration, the method comprising: administering to a subject an effective amount of the antibody as described in the first aspect of the present application, the recombinant immune cell as described in the eighth aspect, the antibody-drug conjugate as described in the tenth aspect, the pharmaceutical composition as described in the eleventh aspect, or a combination thereof.

In some embodiments, the method further comprises: administering other drugs or treatment methods to the subject for combined treatment.

The "other drugs or treatment methods", "anti-tumor immunotherapy drugs" and "subjects" are defined as in the fourteenth aspect.

### Sixteenth aspect of the present application

The present application provides a method for inhibiting tumor growth in a model animal, the method comprising: administering to a model animal an effective amount of the antibody as described in the first aspect of the present application, the recombinant immune cell as described in the eighth aspect, the antibody-drug conjugate as described in the tenth aspect, the pharmaceutical composition as described in the eleventh aspect, or a combination thereof.

In some embodiments, the method further comprises: administering other drugs or treatment methods to the subject for combined treatment.

The "other drugs or treatment methods", and "anti-tumor immunotherapy drugs" are defined as in the fourteenth aspect.

In the above fourteenth, fifteenth and sixteenth aspects of the present application, the term "effective amount" mentioned in the present application refers to the dosage of the component corresponding to the term to achieve treatment, prevention, mitigation and/or alleviation of the specific disease, disorder or symptom in a subject. In the present application, if not specifically limited, it refers to the dosage to achieve treatment, prevention, mitigation and/or alleviation of ROR1 high-expressing diseases, conditions and/or symptoms. In the process of administering the drug to the subject, a safe and effective amount suitable for the subject is to be administered. Of course, the specific dosage should also be determined based on factors such as the route of administration and the patient's health status, which are all within the skills of a skilled physician. Taking the antibody-drug conjugate described in the present application as an example, its effective amount may vary with the mode of administration and the severity of the disease to be treated. The selection of the preferred effective amount can be determined by a person of ordinary skill in the art according to various factors (for example, through clinical trials). The factors include, but are not limited to: pharmacokinetic parameters of the bifunctional antibody-drug conjugate, such as bioavailability, metabolism, half-life, etc.; the severity of the disease to be treated in the patient, the patient's weight, the immune status in the patient, the route of administration, etc. Generally, desired effects can be obtained when the antibody-drug conjugate of the present application is administered at a dose of about 0.0001 mg/kg to 50 mg/kg animal body weight (preferably 0.001 mg/kg to10 mg/kg animal body weight) per day. For example, several divided doses may be administered per day, or the dose may be reduced proportionally, depending on the urgency of the treatment condition.

The present application is further illustrated by the following examples. It is appreciated that these examples are only intended to illustrate the invention, but not to limit the scope of the present application. For the experimental methods in the following examples, they are performed under routine conditions, e.g., those described by Sambrook. et al., in Molecule Clone: A Laboratory Manual, New York: Cold Spring Harbor Laboratory Press, 1989, or as instructed by the manufacturers, unless otherwise specified. Unless otherwise stated, percentages and parts are percentages by weight and parts by weight. The cell lines are conventional commercial products or purchased from ATCC, and the plasmids are all commercial products.

In the following specific embodiments, the measured parameters of raw material components may have slight deviations within the range of weighing accuracy unless otherwise specified. For temperature and time parameters, acceptable deviations caused by instrument test accuracy or operation accuracy are allowed.

In the following examples, the applicant unexpectedly obtained 9 anti-ROR1 monoclonal antibodies through extensive and in-depth research with a large number of screenings, respectively named mAb001 to mAb009. Human IgG1, κ was selected as the backbone to construct human-mouse chimeric antibodies, respectively named mAb001c to mAb009c. After further testing the above antibodies, the results obtained are as follows:
1) the chimeric antibodies can bind to the ROR1-ECD antigen with high specificity, with their EC₅₀ values all ≤0.02nM as determined by ELISA;
2) the chimeric antibodies exhibited extremely high binding affinity for multiple ROR1 high-expressing tumor cells, with their EC₅₀ of 0.15 nM to 1.1 nM as determined by FACS. Considering the similarity of the antibody CDR region sequences, thebinding activity of the antibody against antigen and the uniqueness of the binding epitope, mAb001c and mAb005c were selected for subsequent development;
3) a series of humanized antibodies designed based on mAb001c exhibited higher ROR1 protein binding affinity and cell binding affinity, with their EC₅₀ of 0.010 nM to 0.015 nM as determined by ELISA, and 0.19nM to 0.62nM as determined by FACS;
4) the chimeric antibody exhibited a significant anti-tumor effect in vivo and no obvious toxic and side effects on mammals themselves;
5) a series of humanized antibodies designed based on mAb005c exhibited higher ROR1 protein binding affinity and cell binding affinity, with their EC₅₀ of 0.009 nM to 0.02 nM as determined by ELISA, and 0.12 nM to 0.22 nM as determined by FACS;
6) the chimeric antibody and humanized antibody both exhibited excellent endocytosis properties;
7) the chimeric antibody-drug conjugates and humanized antibody-drug conjugates (ADC) exhibited excellent anti-proliferative activity, that is, exhibited no obvious toxic and side effects on ROR1-low-expressing cells in vitro but extremely high killing effects on ROR1-high-expressing tumor cells, with the killing activity correlated to the ROR1 expression level. Also, the chimeric antibody ADC and humanized antibody ADC drugs exhibited excellent anti-tumor activity in vivo against tumor models with different ROR1 expression levels.

The representative meanings of the sequences involved in the present application are as follows:

**Table 2**

| Sequence No. | Sequence Name | Sequence No. | Sequence Name |
|---|---|---|---|
| SEQ ID NO.1 | mAb001 HCDR1 | SEQ ID NO.52 | mAb006 HCDR2 |
| SEQ ID NO.2 | mAb001 HCDR2 | SEQ ID NO.53 | mAb006 HCDR3 |
| SEQ ID NO.3 | mAb001 HCDR3 | SEQ ID NO.54 | mAb006 LCDR1 |
| SEQ ID NO.4 | mAb001 LCDR1 | SEQ ID NO.55 | mAb006 LCDR2 |
| SEQ ID NO.5 | mAb001 LCDR2 | SEQ ID NO.56 | mAb006 LCDR3 |
| SEQ ID NO.6 | mAb001 LCDR3 | SEQ ID NO.57 | mAb006-VH |
| SEQ ID NO.7 | mAb001-VH | SEQ ID NO.58 | mAb006-VL |
| SEQ ID NO.8 | mAb001-VH(DG_DA) | SEQ ID NO.59 | mAb007 HCDR1 |
| SEQ ID NO.9 | mAb001-VL | SEQ ID NO.60 | mAb007 HCDR2 |
| SEQ ID NO.10 | mAb001-VL(NG_NA) | SEQ ID NO.61 | mAb007 HCDR3 |
| SEQ ID NO.11 | mAb004 HCDR1 | SEQ ID NO.62 | mAb007 LCDR1 |
| SEQ ID NO.12 | mAb004 HCDR2 | SEQ ID NO.63 | mAb007 LCDR2 |
| SEQ ID NO.13 | mAb004 HCDR3 | SEQ ID NO.64 | mAb007 LCDR3 |
| SEQ ID NO.14 | mAb004 LCDR1 | SEQ ID NO.65 | mAb007-VH |
| SEQ ID NO.15 | mAb004 LCDR2 | SEQ ID NO.66 | mAb007-VL |
| SEQ ID NO.16 | mAb004 LCDR3 | SEQ ID NO.67 | mAb008 HCDR1 |
| SEQ ID NO.17 | mAb004-VH | SEQ ID NO.68 | mAb008 HCDR2 |
| SEQ ID NO.18 | mAb004-VL | SEQ ID NO.69 | mAb008 HCDR3 |
| SEQ ID NO.19 | mAb005 HCDR1 | SEQ ID NO.70 | mAb008 LCDR1 |
| SEQ ID NO.20 | mAb005 HCDR2 | SEQ ID NO.71 | mAb008 LCDR2 |
| SEQ ID NO.21 | mAb005 HCDR3 | SEQ ID NO.72 | mAb008 LCDR3 |
| SEQ ID NO.22 | mAb005 LCDR1 | SEQ ID NO.73 | mAb008-VH |
| SEQ ID NO.23 | mAb005 LCDR2 | SEQ ID NO.74 | mAb008-VL |
| SEQ ID NO.24 | mAb005 LCDR3 | SEQ ID NO.75 | mAb009 HCDR1 |
| SEQ ID NO.25 | mAb005-VH | SEQ ID NO.76 | mAb009 HCDR2 |
| SEQ ID NO.26 | mAb005-VH(DG_DA) | SEQ ID NO.77 | mAb009 HCDR3 |
| SEQ ID NO.27 | mAb005-VL | SEQ ID NO.78 | mAb009 LCDR1 |
| SEQ ID NO.28 | mAb001_VHg0 | SEQ ID NO.79 | mAb009 LCDR2 |
| SEQ ID NO.29 | mAb001_VHg1 | SEQ ID NO.80 | mAb009 LCDR3 |
| SEQ ID NO.30 | mAb001_VHg2 | SEQ ID NO.81 | mAb009-VH |
| SEQ ID NO.31 | mAb001_VHg3 | SEQ ID NO.82 | mAb009-VL |
| SEQ ID NO.32 | mAb001_VHg4 | SEQ ID NO.83 | mAb001_VH-CDR2 (DG_DA) |
| SEQ ID NO.33 | mAb001_VLg1 | SEQ ID NO.84 | mAb001_VL- CDR1(NG_NA) |
| SEQ ID NO.34 | mAb001_VLg2 | SEQ ID NO.85 | mAb005_VH- CDR2-(DG_DA) |
| SEQ ID NO.35 | mAb002 HCDR1 | SEQ ID NO.86 | Extracellular domain of human ROR1 protein (ROR1-ECD) |
| SEQ ID NO.36 | mAb002 HCDR2 | SEQ ID NO.87 | UC-961-VH |
| SEQ ID NO.37 | mAb002 HCDR3 | SEQ ID NO.88 | UC-961-VL |
| SEQ ID NO.38 | mAb002 LCDR1 | SEQ ID NO.89 | ROR1 extracellular segment sequence (including transmembrane) |
| SEQ ID NO.39 | mAb002 LCDR2 | SEQ ID NO.90 | ROR1 extracellular segment with Ig-like domain removed |
| SEQ ID NO.40 | mAb002 LCDR3 | SEQ ID NO.91 | ROR1 extracellular segment with Frizzled domain removed |

| SEQ ID NO.41 | mAb002 -VH | SEQ ID NO.92 | ROR1 extracellular segment with Kringle domain removed |
|---|---|---|---|
| SEQ ID NO.42 | mAb002 -VL | SEQ ID NO.93 | mAb005_VHg8 |
| SEQ ID NO.43 | mAb003 HCDR1 | SEQ ID NO.94 | mAb005_VHg13 |
| SEQ ID NO.44 | mAb003 HCDR2 | SEQ ID NO.95 | mAb005_VHg14 |
| SEQ ID NO.45 | mAb003 HCDR3 | SEQ ID NO.96 | mAb005_VHg15 |
| SEQ ID NO.46 | mAb003 LCDR1 | SEQ ID NO.97 | mAb005_VHg16 |
| SEQ ID NO.47 | mAb003 LCDR2 | SEQ ID NO.98 | mAb005_VHg17 |
| SEQ ID NO.48 | mAb003 LCDR3 | SEQ ID NO.99 | mAb005_VHg18 |
| SEQ ID NO.49 | mAb003-VH | SEQ ID NO.100 | mAb005_VHg19 |
| SEQ ID NO.50 | mAb003-VL | SEQ ID NO.101 | mAb005_VLg22 |
| SEQ ID NO.51 | mAb006 HCDR1 | | |

### Example 1. Discovery and preparation of monoclonal antibodies targeting human ROR1

### Step 1) preparing hybridoma cells

First, extracellular domain of human ROR1 protein (ROR1-ECD) was prepared as a backup antigen. Referring to amino acids from position 30 to 403 of NP_005003.2 from NCBI, a C-terminus polyhistidine-tagged antigen was obtained by using gene cloning technology and a mammalian vector expression system. The specific amino acid sequence is as follows (SEQ ID NO. 86):

QETELSVSAELVPTSSWNISSELNKDSYLTLDEPMNNITTSLGQTAELHCKVSGNPPPTIR
WFKNDAPVVQEPRRLSFRSTIYGSRLRIRNLDTTDTGYFQCVATNGKEVVSSTGVLFVKFGPPPTASPG
YSDEYEEDGFCQPYRGIACARFIGNRTVYMESLHMQGEIENQITAAFTMIGTSSHLSDKCSQFAIPSLCH
YAFPYCDETSSVPKPRDLCRDECEILENVLCQTEYIFARSNPMILMRLKLPNCEDLPQPESPEAANCIRI
GIPMADPINKNHKCYNSTGVDYRGTVSVTKSGRQCQPWNSQYPHTHTFTALRFPELNGGHSYCRNPG
NQKEAPWCFTLDENFKSDLCDIPACDSKDSKEKNKMEAHHHHHHHHHH

Then, the human ROR1 extracellular domain protein expressed and prepared in HEK293T cells was used to immunize SJL mice at a dosage of 50 µg/mouse to prepare immune spleen cells; mouse myeloma cells (SP2/0) and feeder cells were prepared in good time for fusion. After the above three types of cells were prepared, the immune spleen cells and SP2/0 cells were fused by an electric fusion instrument (BEX, Model: LF301), resuspended in HAT complete medium containing feeder cells, inoculated into 96-well plates for culture, and screened for positive wells by ELISA/FACS.

Finally, the cells in the positive wells were cloned, cultured by the limiting dilution method, and screened for the cells with high titer, good morphology and monoclonal growth by ELSIA or FASCS for further subclone screening until the screened positive clone rate reached 100%. Then, the cell line can be expanded and cultured, and then a library can be constructed.

### Step 2) Preparing and purifying mouse monoclonal antibodies targeting human ROR1:

After the hybridoma cells selected in step ① were expanded and cultured in roller bottles for 14 days, the cell culture supernatant was collected and filtered through a 0.45µm filter membrane. The obtained culture supernatant was added to a pre-equilibrated Protein A affinity resin column (GE, Cat# 17-1279-02) at a constant rate and the column was equilibrated with 0.1M Tris-HCl (PH=8.0, containing 1.5M NaCl).

The equilibrium column was then eluted with 0.1 M sodium citrate buffer. The eluate was collected and desalted, sterilized using 0.22 µm filter membrane, quantified by UV280, and subjected to SDS-PAGE electrophoresis, SEC-HPLC and endotoxin detection.

The purified monoclonal antibodies were aliquoted and stored at -80°C for later use.

### Step 3) Determining the biological activity and specificity of the mouse monoclonal antibody targeting human ROR1:

After repeated screening, the 9 selected hybridoma monoclonal antibodies were determined for their biological activity and targeting specificity.

As shown in FIG. 1A, the supernatants of the monoclonal cell culture were tested by enzyme-linked immunosorbent assay (ELISA), showing that All 9 monoclones could specifically bind to the purified human ROR1-ECD protein.

As shown in FIG. 1B, the supernatant of monoclonal cell culture was tested by fluorescence-activated cell sorter (FACS), showing that all 9 monoclones could specifically bind to the human ROR1- high-expressing MDA-MB-231 cells (ROR1-P), but had no obvious binding activity to the ROR1-low-expressing MDA-MB-453 cells (ROR1-N).

As shown in FIG. 1C, the purified monoclonal antibody samples were used for subtype testing, and mAb001, mAb004 to mAb009 were all identified as IgG2b/k, and mAb002 and mAb003 were IgG2c/k.

Then, the nine purified monoclonal antibodies were serially diluted and tested for the binding activity against purified ROR1-ECD protein by ELISA, showing EC₅₀ values of 0.049 nM, 0.028 nM, 0.023 nM, 0.038 nM, 0.043 nM, 0.059 nM, 0.066 nM, 0.053 nM, and 0.062 nM, respectively. The EC₅₀ values of ROR1 binding activity against MDA-MB-231 cells tested by FACS were 0.073 nM, 0.11 nM, 0.45 nM, 0.15 nM, 0.16 nM, 0.19 nM, 0.15 nM, 0.11 nM, and 0.16 nM, respectively.

### Example 2. Monoclonal antibody sequencing and identification of complementarity determining regions (CDRs)

Based on excellent specificity and affinity, mAb001, mAb002, mAb003, mAb004, mAb005, mAb006, mAb007, mAb008, and mAb009 were preferentially selected for antibody sequencing and identification. The heavy chain (VH) and light chain (VL) variable region fragments were amplified by 5 ' RACE and conventional PCR technology, and cloned into a vector. Information of the following heavy chain variable region (VH) and light chain variable region (VL) amino acid sequences and the complementary determining region (CDR) were obtained by conventionally sequencing and analysis on the Kabat database (http://www.bioinf.org.uk). The underlined sequences are the CDR-1/2/3 amino acid sequences.

### (1) mAb001

**SEQ ID NO.1 (mAb001 HCDR1):** DYNLH
**SEQ ID NO.2 (mAb001 HCDR2) :**YINPNHDGTSYNQKFQG
**SEQ ID NO.3 (mAb001 HCDR3):** RVRTGTGLDD
**SEQ ID NO.4 (mAb001 LCDR1):** RSSHNIVHTNGNTYLE
**SEQ ID NO.5 (mAb001 LCDR2):** KVSNRFS
**SEQ ID NO.6 (mAb001 LCDR3):** FQGSRFPYT
**SEQ ID NO.7 (mAb001-VH):** 
EVRLQQSGPVLVKPGASVKMSCKASGYTFTDYNLHWVKQSHRQSLEWIGYINPNHDG
TSYNQKFQGKATLTVDRSSSTAYMEFRSLTSEDSAVYYCARRVRTGTGLDDWGQGTTLTVSS
**SEQ ID NO.9 (mAb001-VL):**

DVLMTQSPLSLPVSLGDQASISCRSSHNIVHTNGNTYLEWYLQKPGQSPKLLIYKVSNR
FSGVPDRFSGSGSGTYFTLTISRVEAEDLGVYYCFQGSRFPYTFGGGTKLEIK

### (2) mAb002

**SEQ ID NO.35 (mAb002 HCDR1):** DYNLH
**SEQ ID NO.36 (mAb002 HCDR2) :** YINPNNGGTTYNQKFKG
**SEQ ID NO.37 (mAb002 HCDR3):** RVRTGTGFDY
**SEQ ID NO.38 (mAb002 LCDR1):** KSSHNILHTNGNTYLE
**SEQ ID NO.39 (mAb002 LCDR2):** KVSNRFS
**SEQ ID NO.40 (mAb002 LCDR3):** FQGSRFPYT
**SEQ ID NO.41 (mAb002 heavy chain variable region (VH)sequence):** 
EVQLQQSGPELVKPGASVKMSCKTSGYTFTDYNLHWVKQSHRRSLEWIGYINPNNGGT
TYNQKFKGKATLTVDKSSSTAYMELRSLTSEDSAVYYFVRRVRTGTGFDYWGQGTTLTVSS
**SEQ ID NO.42 (mAb002 light chain variable region (VL) sequence):**

EVLMTQTPLSLPVSLGDQASISCKSSHNILHTNGNTYLEWYLQKPGQSPKLLIYKVSNR
FSGVPDRFSGSGSGTDFTLTISRVEAEDLGVYYCFQGSRFPYTFGGGTKLEIK

### mAb003

**SEQ ID NO.43 (mAb003 HCDR1):** SYAMS
**SEQ ID NO.44 (mAb003 HCDR2) :**TISDGGSYTYYPDSVKG
**SEQ ID NO.45 (mAb003 HCDR3):** GGYYYNLYAMDY
**SEQ ID NO.46 (mAb003 LCDR1):** KASQSVSFAGTSLMH
**SEQ ID NO.47 (mAb003 LCDR2):** RASNLEA
**SEQ ID NO.48 (mAb003 LCDR3):** QQSGEYPWT
**SEQ ID NO.49 (mAb003 heavy chain variable region** (**VH**) sequence): 
EVQLVESGGGLVKPGGSLKLSCAASGFTFSSYAMSWVRQTPEKRLEWVATISDGGSYTY
YPDSVKGRFTISRDNAKNNLYLQMSHLKSEDTDMYYCVRGGYYYNLYAMDYWGQGTSVTVSS
**SEQ ID NO.50 (mAb003 light chain variable region (VL) sequence):**

DIVLTQSPASLAVSLGQRAIISCKASQSVSFAGTSLMHWYQQKPGQQPKLLIYRASNLEA
GVPTRFSGSGSRADFTLNIHPVEEDDAATYYCQQSGEYPWTFGGGTKLEIK

### mAb004

**SEQ ID NO.11 (mAb004 HCDR1):** TYAMH
**SEQ ID NO.12 (mAb004 HCDR2):** RIRSKSSNFATYYADSVRD
**SEQ ID NO.13 (mAb004 HCDR3):** GYGPSYFSLDY
**SEQ ID NO.14 (mAb004 LCDR1):** RSSQSLVHSNGNTYLH
**SEQ ID NO.15 (mAb004 LCDR2):** KVSNRFS
**SEQ ID NO.16 (mAb004 LCDR3):** SQSTHAIYT
**SEQ ID NO.17 (mAb004-VH):** 
EVQLVESGGGLVQPKGSLKLSCAASGFTFNTYAMHWVRQAPGKGLEWVARIRSKSSNF
ATYYADSVRDRFTISRDDSQSMLYLQMNNLKTEDTAMYYCVRGYGPSYFSLDYWGQGTSVTVSS
**SEQ ID NO.18 (mAb004-VL):**

DVVMTQTPLSLPVSLGDQASISCRSSQSLVHSNGNTYLHWYLQKPGQSPKLLIYKVSNR
FSGVPDRFSGSGSGTDFTLKINRVEAEDLGVYFCSQSTHAIYTFGGGTKLEIK

### mAb005

**SEQ ID NO.19 (mAb005 HCDR1):** SYAMS
**SEQ ID NO.20 (mAb005 HCDR2):** TISDGGSYTYYPDNEKG
**SEQ ID NO.21 (mAb005 HCDR3):** GGYYYNLYAMDY
**SEQ ID NO.22 (mAb005 LCDR1):** KASQSVSFPGTSLMH
**SEQ ID NO.23 (mAb005 LCDR2):** RASNLEA
**SEQ ID NO.24 (mAb005 LCDR3):** QQSREYPWT
**SEQ ID NO.25 (mAb005-VH):** 
EVQLVESGGGLVKPGGSLKLSCAASGFTFNSYAMSWVRQTPEKRLEWVATISDGGSYTYYPDNEKG
RFTISRDNAKNNLYLQMSHLKSEDTAIYYCVRGGYYYNLYAMDYWGQGTSVTVSS
**SEQ ID NO.27 (mAb005-VL):**

DIVLTQSPASLAVSLGQRAIISCKASQSVSFPGTSLMHWYQQKPGQQPKLLIYRASNLEA
GVPTRFSGSGSRTDFTLNIHPVEEDDTATYYCQQSREYPWTFGGGTQLEIK

### mAb006

**SEQ ID NO.51 (mAb006 HCDR1):** DYNIH
**SEQ ID NO.52 (mAb006 HCDR2):** YINPNNGGTTYNQKFKG
**SEQ ID NO.53 (mAb006 HCDR3):** RGYGSSYAMDY
**SEQ ID NO.54 (mAb006 LCDR1):** RSSHIIVHTNGNTYLE
**SEQ ID NO.55 (mAb006 LCDR2):** KVSNRFS
**SEQ ID NO.56 (mAb006 LCDR3):** FQGSRVPYT
**SEQ ID NO.57 (mAb006-VH):** 
EVQLQQSGPELVKPGASLKMSCKASGYTFPDYNIHWVKQSHGKSLEWIGYINPNNGGT
TYNQKFKGKATLTVSKSSSTAYMELRSLTSEDSAVYSCARRGYGSSYAMDYWAQGTSVTVSS
**SEQ ID NO.58 (mAb006-VL):**

DVLMTQSPLSLPVSLGDQASISCRSSHIIVHTNGNTYLEWYLQKPGQSPKLLIYKVSNRF
SGVPDRFSGSGSGTDFTLKISRVEAEDLGVYYCFQGSRVPYTFGGGTKLEIK

### mAb007

**SEQ ID NO.59 (mAb007 HCDR1):** DYNMH
**SEQ ID NO.60 (mAb007 HCDR2):** YINPNNGGTSYNQKFKG
**SEQ ID NO.61 (mAb007 HCDR3):** RGYGSGYAMDY
**SEQ ID NO.62 (mAb007 LCDR1):** RSSQSIVHTNGNTYLE
**SEQ ID NO.63 (mAb007 LCDR2):** KVFNRFS
**SEQ ID NO.64 (mAb007 LCDR3):** FQGSLFPYT
**SEQ ID NO.65 (mAb007-VH):** 
EVQLQQSGPELVKPGASVKMSCKASGYTFTDYNMHWVKQSHGKSLEWIGYINPNNGG
TSYNQKFKGKATLTVNKSSSTAYMELRSLTSDDSAVYYCARRGYGSGYAMDYWGQGTSVTVSS
**SEQ ID NO.66 (mAb007-VL):**

DVLMTQTPLSLPVSLGDQASISCRSSQSIVHTNGNTYLEWYLQNPGQSPKLLIYKVFNR
FSGVPDRFSGSGSGTDFTLKISRVEAEDLGVYYCFQGSLFPYTFGGGTKLEIK

### mAb008

**SEQ ID NO.67 (mAb008 HCDR1):** DYNMH
**SEQ ID NO.68 (mAb008 HCDR2):** YINPNNGGTTYNQKFKG
**SEQ ID NO.69 (mAb008 HCDR3):** RGYGSGYAMDY
**SEQ ID NO.70 (mAb008 LCDR1):** TSSQSIVHTNGNTYLE
**SEQ ID NO.71 (mAb008 LCDR2):** KVSNRFS
**SEQ ID NO.72 (mAb008 LCDR3):** FQGSLFPYT
**SEQ ID NO.73 (mAb008-VH):** 
EVQLQQSGPELVKPGASVKMSCKASEYTFTDYNMHWVKQSHGKSLEWIGYINPNNGG
TTYNQKFKGKATLTVNKSSSTAYMELRSLTSDDSAVYYCARRGYGSGYAMDYWGQGASVTVSS
**SEQ ID NO.74 (mAb008-VL):**

DVLLTQTPLSLPVSLGDRASISCTSSQSIVHTNGNTYLEWYLQNPGQSPKLLIYKVSNRF
SGVPDRFSGSGSGTDFTLKISRVEAEDLGVYYCFQGSLFPYTFGGGTKLEIK

### mAb009

**SEQ ID NO.75 (mAb009 HCDR1):** DYNIH
**SEQ ID NO.76 (mAb009 HCDR2):** YINPNNGNTNYNQKFEG
**SEQ ID NO.77 (mAb009 HCDR3):** RGYGSSYAMDF
**SEQ ID NO.78 (mAb009 LCDR1):** RSSHIIVHTNGNTYLE
**SEQ ID NO.79 (mAb009 LCDR2):** KVSNRFS
**SEQ ID NO.80 (mAb009 LCDR3):** FQGSRVPYT
**SEQ ID NO.81 (mAb009-VH):** 
EVQLQQSGPELVKPGASLKLSCKASGYTFPDYNIHWVKQSHGKSLEWIGYINPNNGNTN
YNQKFEGKATLTVSKSSSTAYMELRSLTSDDSAVYSCARRGYGSSYAMDFWGQGTSVTVSS
**SEQ ID NO.82 (mAb009-VL):**

DVLMTQTPLSLPVSLGNQASISCRSSHIIVHTNGNTYLEWYLQKPGQSPKLLIYKVSNRF
SGVPDRFSGSGSGTDFTLKISRVEAEDLGVYYCFQGSRVPYTFGGGTKLEIK

**Table 3**

| Monoclonal antibody | CDR | Sequence No. | Sequence |
|---|---|---|---|
| mAb001 | H CDR-1 | SEQ ID NO.1 | DYNLH |
| | H CDR-2 | SEQ ID NO.2 | YINPNHDGTSYNQKFQG |
| | H CDR-3 | SEQ ID NO.3 | RVRTGTGLDD |
| | L CDR-1 | SEQ ID NO.4 | RSSHNIVHTNGNTYLE |
| | L CDR-2 | SEQ ID NO.5 | KVSNRFS |
| | L CDR-3 | SEQ ID NO.6 | FQGSRFPYT |
| mAb002 | H CDR-1 | SEQ ID NO.35 | DYNLH |
| | H CDR-2 | SEQ ID NO.36 | YINPNNGGTTYNQKFKG |
| | H CDR-3 | SEQ ID NO.37 | RVRTGTGFDY |
| | L CDR-1 | SEQ ID NO.38 | KSSHNILHTNGNTYLE |
| | L CDR-2 | SEQ ID NO.39 | KVSNRFS |
| | L CDR-3 | SEQ ID NO.40 | FQGSRFPYT |
| mAb003 | H CDR-1 | SEQ ID NO.43 | SYAMS |
| | H CDR-2 | SEQ ID NO.44 | TISDGGSYTYYPDSVKG |
| | H CDR-3 | SEQ ID NO.45 | GGYYYNLYAMDY |
| | L CDR-1 | SEQ ID NO.46 | KASQSVSFAGTSLMH |
| | L CDR-2 | SEQ ID NO.47 | RASNLEA |
| | L CDR-3 | SEQ ID NO.48 | QQSGEYPWT |
| mAb004 | H CDR-1 | SEQ ID NO.11 | TYAMH |
| | H CDR-2 | SEQ ID NO.12 | RIRSKSSNFATYYADSVRD |
| | H CDR-3 | SEQ ID NO.13 | GYGPSYFSLDY |
| | L CDR-1 | SEQ ID NO.14 | RSSQSLVHSNGNTYLH |
| | L CDR-2 | SEQ ID NO.15 | KVSNRFS |
| | L CDR-3 | SEQ ID NO.16 | SQSTHAIYT |
| mAb005 | H CDR-1 | SEQ ID NO.19 | SYAMS |
| | H CDR-2 | SEQ ID NO.20 | TISDGGSYTYYPDNEKG |
| | H CDR-3 | SEQ ID NO.21 | GGYYYNLYAMDY |
| | L CDR-1 | SEQ ID NO.22 | KASQSVSFPGTSLMH |
| | L CDR-2 | SEQ ID NO.23 | RASNLEA |
| | L CDR-3 | SEQ ID NO.24 | QQSREYPWT |
| mAb006 | H CDR-1 | SEQ ID NO.51 | DYNIH |
| | H CDR-2 | SEQ ID NO.52 | YINPNNGGTTYNQKFKG |
| | H CDR-3 | SEQ ID NO.53 | RGYGSSYAMDY |
| | L CDR-1 | SEQ ID NO.54 | RSSHIIVHTNGNTYLE |
| | L CDR-2 | SEQ ID NO.55 | KVSNRFS |
| | L CDR-3 | SEQ ID NO.56 | FQGSRVPYT |
| mAb007 | H CDR-1 | SEQ ID NO.59 | DYNMH |
| | H CDR-2 | SEQ ID NO.60 | YINPNNGGTSYNQKFKG |
| | H CDR-3 | SEQ ID NO.61 | RGYGSGYAMDY |
| | L CDR-1 | SEQ ID NO.62 | RSSQSIVHTNGNTYLE |
| | L CDR-2 | SEQ ID NO.63 | KVFNRFS |
| | L CDR-3 | SEQ ID NO.64 | FQGSLFPYT |
| mAb008 | H CDR-1 | SEQ ID NO.67 | DYNMH |
| | H CDR-2 | SEQ ID NO.68 | YINPNNGGTTYNQKFKG |
| | H CDR-3 | SEQ ID NO.69 | RGYGSGYAMDY |
| | L CDR-1 | SEQ ID NO.70 | TSSQSIVHTNGNTYLE |
| | L CDR-2 | SEQ ID NO.71 | KVSNRFS |
| | L CDR-3 | SEQ ID NO.72 | FQGSLFPYT |
| mAb009 | H CDR-1 | SEQ ID NO.75 | DYNIH |
| | H CDR-2 | SEQ ID NO.76 | YINPNNGNTNYNQKFEG |
| | H CDR-3 | SEQ ID NO.77 | RGYGSSYAMDF |
| | L CDR-1 | SEQ ID NO.78 | RSSHIIVHTNGNTYLE |
| | L CDR-2 | SEQ ID NO.79 | KVSNRFS |
| | L CDR-3 | SEQ ID NO.80 | FQGSRVPYT |

### Example 3. Preparation of human-mouse chimeric antibodies

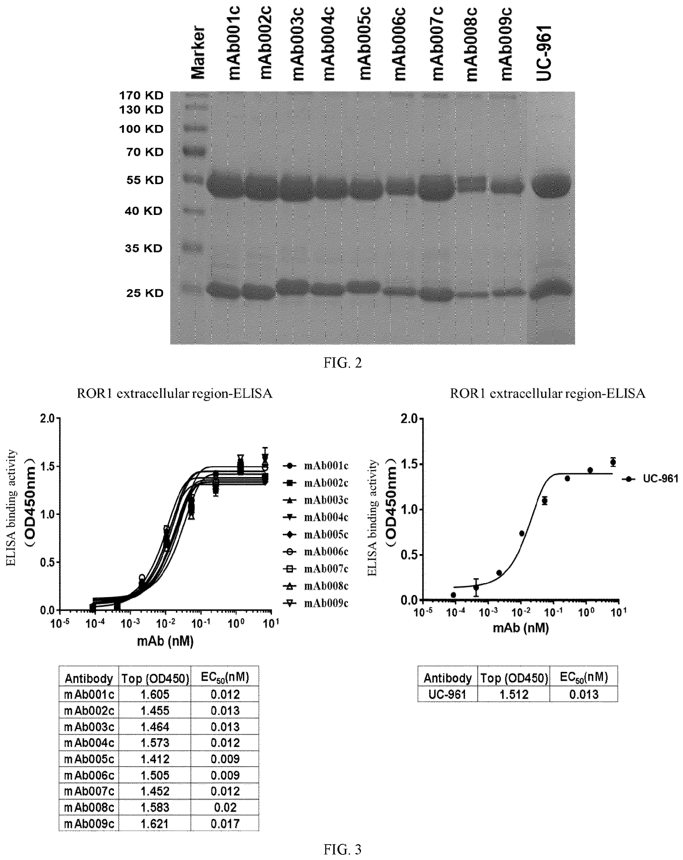

By gene recombination technology, 9 sets of nucleic acids encoding variable region fragments (see SEQ ID NO.7, SEQ ID NO.41, SEQ ID NO.49, SEQ ID NO.17, SEQ ID NO.25, SEQ ID NO.57, SEQ ID NO.65, SEQ ID NO.73, SEQ ID NO.81, SEQ ID NO.9, SEQ ID NO.42, SEQ ID NO.50, SEQ ID NO.18, SEQ ID NO.27, SEQ ID NO.58, SEQ ID NO.66, SEQ ID NO.74, SEQ ID NO.82) were cloned into a vector containing human IgG1 heavy chain constant region and Kappa chain constant region. After confirming by sequencing, the constructed chimeric antibodies were expressed and purified using a mammalian HEK-293T cell expression system (see FIG. 2). The obtained human-mouse chimeric antibodies were designated as mAb001c, mAb002c, mAb003c, mAb004c, mAb005c, mAb006c, mAb007c, mAb008c, mAb009c, respectively. UC-961 disclosed in the invention patent application US20150232569A1 was prepared in the same manner as a control.

The variable region sequence of the antibody contains several unfavorable amino acids, which were modified by site-directed mutagenesis. The amino acid sequences of the heavy chain variable region (VH) and light chain variable region (VL) after site-directed mutagenesis are listed below ("_" shows the CDR amino acid sequence).
**SEQ ID NO.8** mAb001_VH(DG_DA) EVRLQQSGPVLVKPGASVKMSCKASGYTFTDYNLHWVKQSHRQSLEWIGYI 
NPNHDATSYNQKFQGKATLTVDRSSSTAYMEFRSLTSEDSAVYYCARRVRTGTGLDDWG
QGTTLTVSS
**SEQ ID NO.10** mAb001 VL(NG NA) 
DVLMTQSPLSLPVSLGDQASISCRSSHNIVHTNANTYLEWYLQKPGQSPKLLI
YKVSNRFSGVPDRFSGSGSGTYFTLTISRVEAEDLGVYYCFQGSRFPYTFGGGTKLEIK
**SEQ ID NO.26** mAb005_VH(DG_DA) 
EVQLVESGGGLVKPGGSLKLSCAASGFTFNSYAMSWVRQTPEKRLEWVATIS
DAGSYTYYPDNEKGRFTISRDNAKNNLYLQMSHLKSEDTAIYYCVRGGYYYNLYAMDY
WGQGTSVTVSS
**SEQ ID NO.83:** YINPNHDATSYNOKFOG
**SEQ ID NO.84:** RSSHNIVHTNANTYLE
**SEQ ID NO.85:** TISDAGSYTYYPDNEKG

The site-directed mutagenesis (PTM) obtained by matching the above template for site-directed mutagenesis was cloned into the hIgG1,κ vector to obtain the corresponding chimeric antibody mutant with the site-directed mutagenesis.

The numbers of the above-mentioned human-mouse chimeric antibodies and the corresponding human-mouse chimeric antibody mutants, and the sequence numbers of the heavy chain variable regions and light chain variable regions of the human-mouse chimeric antibodies are summarized in Table 4.

**Table-4: Human-mouse chimeric antibodies and their mutants**

| Antibody Name | VH (heavy chain variable region) | VL (light chain variable region) |
|---|---|---|
| mAb001c | SEQ ID NO.7 | SEQ ID NO.9 |
| mAb001c_v1 | SEQ ID NO.8 | SEQ ID NO.10 |
| mAb002c | SEQ ID NO.41 | SEQ ID NO.42 |
| mAb003c | SEQ ID NO.49 | SEQ ID NO.50 |
| mAb004c | SEQ ID NO.17 | SEQ ID NO.18 |
| mAb005c | SEQ ID NO.25 | SEQ ID NO.27 |
| mAb005c_v1 | SEQ ID NO.26 | SEQ ID NO.27 |
| mAb006c | SEQ ID NO.57 | SEQ ID NO.58 |
| mAb007c | SEQ ID NO.65 | SEQ ID NO.66 |
| mAb008c | SEQ ID NO.73 | SEQ ID NO.74 |
| mAb009c | SEQ ID NO.81 | SEQ ID NO.82 |
| UC-961 | SEQ ID NO.87 | SEQ ID NO.88 |

### Example 4 ELISA determination of affinity of human-mouse chimeric antibody to human ROR1 protein

The purified human ROR1 protein extracellular domain (ROR1-ECD) was diluted to 1 µg/mL in a coating solution, and coated on ELISA plate at 100 µL/well at 4°C overnight. After washing excess antigen off, and the plate was blocked with 1% BSA at room temperature for 2 h, then 3-fold gradient dilutions of human-mouse chimeric antibody were added at 100 L/well, and incubated at room temperature for 2 h. After washing unbound human-mouse antibody off, anti-mouse secondary antibody labeled with horseradish peroxidase at appropriate concentration was added at 100 µL/well and incubated at room temperature for 1 h. After washing unbound secondary antibody off, TMB color development solution was added to react for about 10min, 2N H₂SO₄ was added at 50 µL/well to terminate the color development reaction, then the absorbance was measured at 450nm, and the data was analyzed.

The test results are shown in FIG. 3. mAb001c, mAb002c, mAb003c, mAb004c, mAb005c, mAb006c, mAb007c, mAb008c, and mAb009c had strong affinity for human ROR1-ECD, with EC₅₀ of 0.012nM, 0.013nM, 0.013nM, 0.012nM, 0.009nM, 0.009nM, 0.012nM, 0.02nM, and 0.017nM, respectively. Among them, the binding activity of mAb001c, mAb004c, mAb005c, mAb006c, and mAb007c was slightly stronger than that of UC-961 (EC₅₀ is 0.013nM);

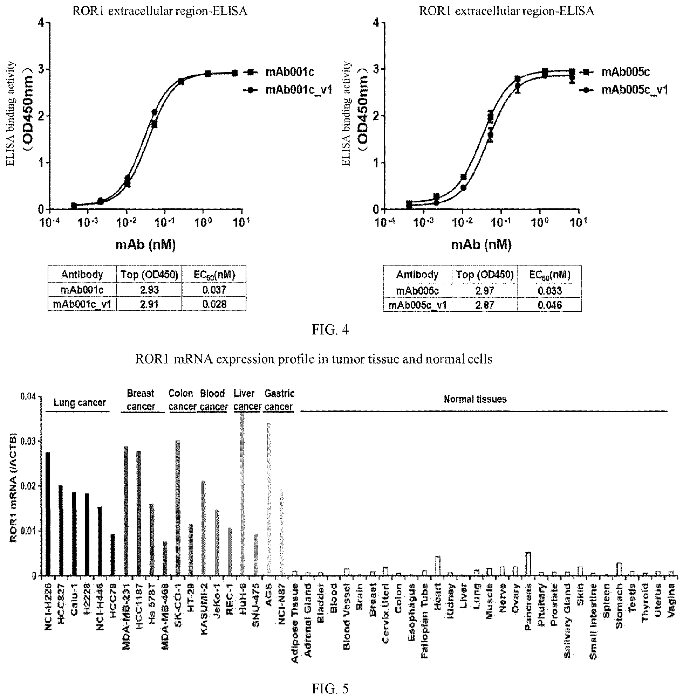

The test results are shown in FIG. 4. The human-mouse chimeric antibody mutants of mAb001c and mAb005c also had a strong affinity for human ROR1-ECD, with EC₅₀ of 0.028 nM and 0.046 nM, respectively.

### Example 5. Analysis of gene expression database of human tumor and normal tissue

By downloading the gene expression information from the CCLE (Cancer cell line encyclopedia) database and the GTEX (human normal tissue) database, the expression of ROR1 mRNA levels was analyzed in tumor cell line groups (such as lung cancer, breast cancer, colon cancer, blood tumors, liver cancer, and gastric cancer) relative to 30 normal human tissues.

The results are shown in FIG. 5. Compared with the CCLE database and the GTEX database, the average ROR1 mRNA expression level in highly invasive breast cancer, lung cancer, colon cancer, blood tumor, liver cancer and gastric cancer cell lines was significantly higher than that of normal tissues, indicating that ROR1 has an excellent therapeutic window in tumors and normal tissues. The antibody targeting ROR1 in the present application will have significant therapeutic effects and broad application prospects in the diagnosis, prevention and treatment of highly invasive breast cancer, lung cancer, colon cancer, blood tumor, liver cancer and gastric cancer.

In this example, the expression level of ROR1 mRNA was also analyzed and compared for different molecular subtypes (e.g., luminal-type versus basal-type) breast cancers.

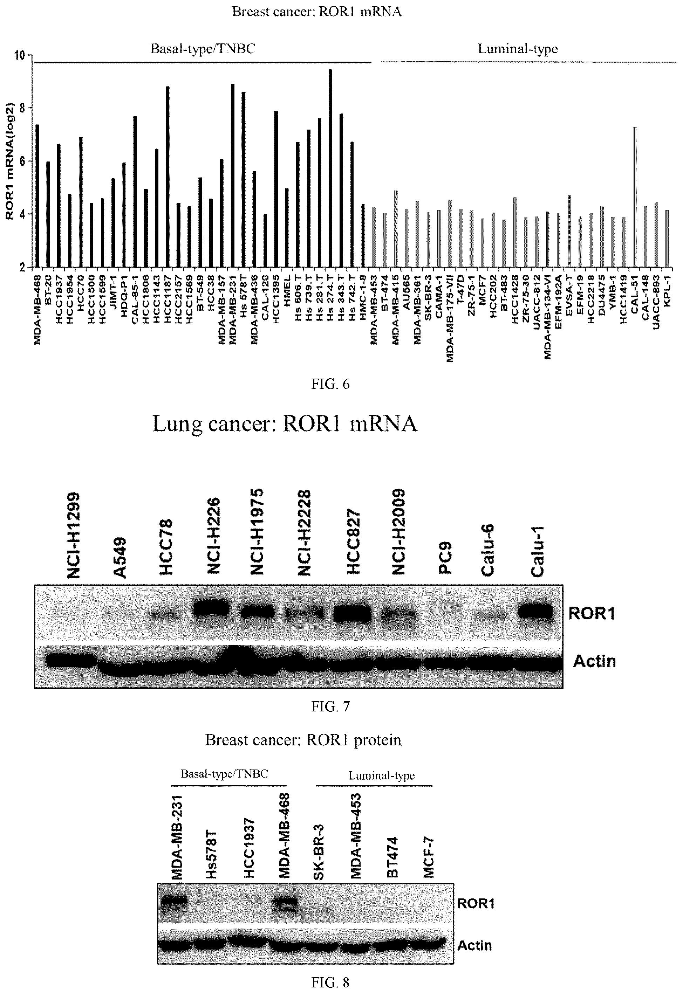

The results are shown in FIG. 6. Compared with luminal-type breast cancer cell lines, the average ROR1 mRNA expression level of highly invasive and metastatic basal-type breast cancer cell lines was higher with statistical significance. Given that basal-type breast cancer is the main source of "triple-negative" breast cancer in clinical practice, the antibody targeting ROR1 in the present application will have a more significant effect in the diagnosis, prevention and treatment of triple-negative breast cancer.

### Example 6. High expression of ROR1 protein in various tumor cells

Total cell protein was prepared for a variety of lung cancer cell lines (NCI-H1299, A549, HCC78, NCI-H226, NCI-H1975, NCI-H2228, HCC827, NCI-H2009, PC9, Calu-6, Calu-1) and breast cancer cell lines of different molecular types (MDA-MB-231, Hs578T, HCC1937, MDA-MB-468, SK-BR-3, MDA-MB-453, BT474, MCF-7). After accurate quantification, the expression level of ROR1 protein was tested by Western blot. The results are shown in FIGS. 7 and 8. ROR1 protein was abnormally activated and expressed in most of the lung cancer and highly invasive breast cancer (basal/triple negative) cell lines tested.

### Example 7. Specific binding of human-mouse chimeric antibody to ROR1 on the surface of tumor cells

ROR1-high-expressing lung cancer cells (NCI-H226, HCC827, NCI-H446), triple-negative breast cancer cells (MDA-MB-231, HCC1187), colon cancer HT-29 cells and ROR1-low-expressing breast cancer cells (MDA-MB-453, MCF-7) were used as target cells to determine the binding of human-mouse chimeric antibody mAb001c to ROR1 on the cell surface (taking mAb001c as an example, while other human-mouse chimeric antibodies were not listed). 2×10⁵ Tumor cells were mixed with the human-mouse chimeric antibody (at a final concentration of 5 µg/mL), then incubated at 4°C for 1 hour, washed twice with PBS to remove unbound primary antibody, added with 200 µL (2.5 µg/mL) PE-labeled secondary antibody and incubated at 4°C for 30 min, and washed twice with PBS to remove unbound secondary antibodies. Finally the cells were resuspended in 200 µL PBS and tested for the binding intensity (MFI) by fluorescence-activated cell sorter (FACS).

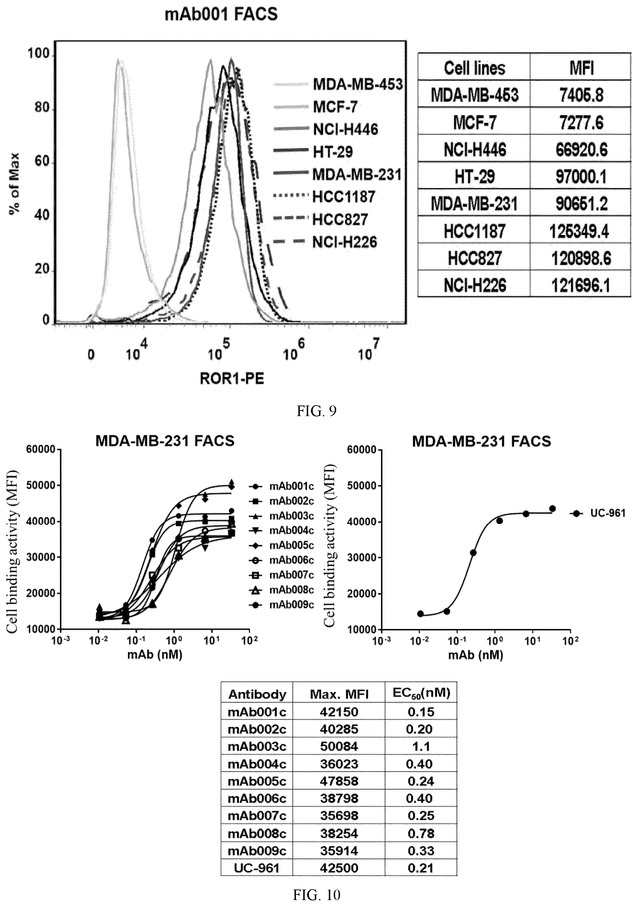

The test results are shown in FIG. 9. The human-mouse chimeric antibody mAb001c can specifically bind to ROR1-high-expressing tumor cells. The binding rate is ranked in order of fluorescence intensity: HCC1187, NCI-H226, HCC827, HT-29, MDA-MB-231, and NCI-H446. Weak binding fluorescence intensity was shown for ROR1-low-expressing tumor cells MDA-MB-453 and MCF-7. Comparing the binding rate (MFI) of HCC1187, NCI-H226, and HCC827 against the human-mouse chimeric antibody with the binding rate of MCF-7 against the human-mouse chimeric antibody, it can be concluded that the difference of binding rate for mAb001c was 17.2 times, 16.7 times, and 16.6 times, respectively.

### Example 8. Determination of the binding affinity of human-mouse chimeric antibodies to ROR1 on the surface of tumor cells

The triple-negative breast cancer cells MDA-MB-231 and non-small cell lung cancer NCI-H226 with high expression of ROR1 were used as target cells. 100 µL dilutions of the human-mouse chimeric antibody to be tested diluted 5- fold from 33.3 nM to 0.002 nM were used as the primary antibody and mixed with 2×10⁵ MDA-MB-231 or NCI-H226 suspended in 100 µL RPMI-1640 serum-free medium, respectively. Then, the cells were incubated at 4°C for 1 h, and the cells were washed twice with PBS to remove the unbound primary antibody. Then, the target cells were incubated with 200 µL PE-labeled secondary antibody at 2.5 µg/mL at 4°C for 30 min, and washed twice with PBS to remove the unbound secondary antibody. Finally, the cells were resuspended in 200 µL PBS, and tested for the binding affinity of the human-mouse chimeric antibody to be tested to ROR1 on cell surface by fluorescence-activated cell sorter(FACS).

The test results are shown in FIG. 10. mAb001c, mAb002c, mAb003c, mAb004c, mAb005c, mAb006c, mAb007c, mAb008c, and mAb009c had strong affinity for MDA-MB-231, with EC₅₀ of 0.15nM, 0.20nM, 1.1nM, 0.40nM, 0.24nM, 0.40nM, 0.25nM, 0.78nM, and 0.33nM, respectively. Among them, the binding activity of mAb001c is significantly better than that of UC-961 (EC₅₀ was 0.21nM), and the binding activity of mAb002, mAb005c, and mAb007c is comparable to that of UC-961.

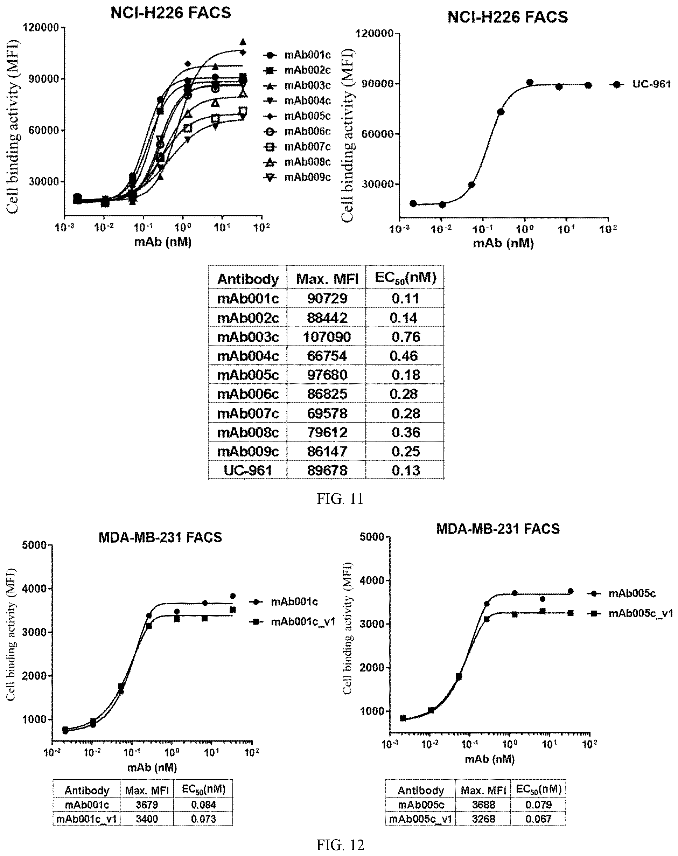

The test results are shown in FIG. 11. mAb001c, mAb002c, mAb003c, mAb004c, mAb005c, mAb006c, mAb007c, mAb008c, and mAb009c also had strong affinity for NCI-H226, with EC₅₀ of 0.11nM, 0.14nM, 0.76nM, 0.46nM, 0.18nM, 0.28nM, 0.28nM, 0.36nM, and 0.25nM, respectively. Among them, the binding activity of mAb001c is better than that of UC-961 (EC₅₀ was 0.13nM), and the binding activities of mAb002c and mAb005c are comparable to those of UC-961;

The test results are shown in FIG. 12. The mutants of mAb001c and mAb005c also had excellent binding affinity to MDA-MB-231. The EC₅₀ value of mAb001c_v1 was 0.073 nM; and the EC₅₀ value of mAb005c_v1 was 0.067 nM.

### Example 9. Determination of the binding activity of human-mouse chimeric antibodies to the extracellular segments of ROR1

The human ROR1 extracellular segment containing the transmembrane region (SEQ ID NO.89), the ROR1 extracellular segment with Ig-like domain removed (SEQ ID NO.90), the ROR1 extracellular segment with Frizzled domain removed (SEQ ID NO.91), and the ROR1 extracellular segment with Kringle domain removed (SEQ ID NO.92) were constructed by genetic engineering technology and each cloned into the pCDNA3.1 expression vector. The specific amino acid sequences are as follows:
**SEQ ID NO.89 ROR1 extracellular segment sequence (including transmembrane)** 
MHRPRRRGTRPPLLALLAALLLAARGAAAQETELSVSAELVPTSSWNISSELNKDSYLTL
DEPMNNITTSLGQTAELHCKVSGNPPPTIRWFKNDAPVVQEPRRLSFRSTIYGSRLRIRNLDTTDTGYF
QCVATNGKEVVSSTGVLFVKFGPPPTASPGYSDEYEEDGFCQPYRGIACARFIGNRTVYMESLHMQGE
IENQITAAFTMIGTSSHLSDKCSQFAIPSLCHYAFPYCDETSSVPKPRDLCRDECEILENVLCQTEYIFAR
SNPMILMRLKLPNCEDLPQPESPEAANCIRIGIPMADPINKNHKCYNSTGVDYRGTVSVTKSGRQCQP
WNSQYPHTHTFTALRFPELNGGHSYCRNPGNQKEAPWCFTLDENFKSDLCDIPACDSKDSKEKNKME
ILYILVPSVAIPLAIALLFFFICVCRNNQKSSSAPVQRQPKHVRGQNVEMSMLNAYKPKSKAKELPLSA
**SEQ ID NO.90 ROR1 extracellular segment with Ig-like domain removed** 
MHRPRRRGTRPPLLALLAALLLAARGAAAQETELSVSAELVPTSSWNISSELNKDSYLTL
DEPMNNITTVVSSTGVLFVKFGPPPTASPGYSDEYEEDGFCQPYRGIACARFIGNRTVYMESLHMQGEI
ENQITAAFTMIGTSSHLSDKCSQFAIPSLCHYAFPYCDETSSVPKPRDLCRDECEILENVLCQTEYIFARS
NPMILMRLKLPNCEDLPQPESPEAANCIRIGIPMADPINKNHKCYNSTGVDYRGTVSVTKSGRQCQPW
NSQYPHTHTFTALRFPELNGGHSYCRNPGNQKEAPWCFTLDENFKSDLCDIPACDSKDSKEKNKMEIL
YILVPSVAIPLAIALLFFFICVCRNNQKSSSAPVQRQPKHVRGQNVEMSMLNAYKPKSKAKELPLSA
**SEQ ID NO.91 ROR1 extracellular segment with Frizzled domain removed** 
MHRPRRRGTRPPLLALLAALLLAARGAAAQETELSVSAELVPTSSWNISSELNKDSYLTL
DEPMNNITTSLGQTAELHCKVSGNPPPTIRWFKNDAPVVQEPRRLSFRSTIYGSRLRIRNLDTTDTGYF
QCVATNGKEVVSSTGVLFVKFGPPPTASPGYSDEYEEDGPMADPINKNHKCYNSTGVDYRGTVSVTK
SGRQCQPWNSQYPHTHTFTALRFPELNGGHSYCRNPGNQKEAPWCFTLDENFKSDLCDIPACDSKDS
KEKNKMEILYILVPSVAIPLAIALLFFFICVCRNNQKSSSAPVQRQPKHVRGQNVEMSMLNAYKPKSKA
KELPLSA
**SEQ ID NO.92 ROR1 extracellular segment with Kringle domain removed**

MHRPRRRGTRPPLLALLAALLLAARGAAAQETELSVSAELVPTSSWNISSELNKDSYLTL
DEPMNNITTSLGQTAELHCKVSGNPPPTIRWFKNDAPVVQEPRRLSFRSTIYGSRLRIRNLDTTDTGYF
QCVATNGKEVVSSTGVLFVKFGPPPTASPGYSDEYEEDGFCQPYRGIACARFIGNRTVYMESLHMQGE
IENQITAAFTMIGTSSHLSDKCSQFAIPSLCHYAFPYCDETSSVPKPRDLCRDECEILENVLCQTEYIFAR
SNPMILMRLKLPNCEDLPQPESPEAANCIRIGIPMADPINKNKDSKEKNKMEILYILVPSVAIPLAIALLF
FFICVCRNNQKSSSAPVQRQPKHVRGQNVEMSMLNAYKPKSKAKELPLSA

CHO cells transfected with the above gene expression vectors were used as target cells (named CHO-ROR1 (extracellular segment), CHO-ROR1 (with Ig-like domain removed), CHO-ROR1 (with Frizzled domain removed), CHO-ROR1 (with Kringle domain removed). 100 µL dilutions of the human-mouse chimeric antibody to be tested diluted 5- fold from 33.3 nM to 0.002 nM or diluted 5- fold from 200 nM to 0.013 nM were used as the primary antibody and mixed with 2×10⁵ target cells suspended in 100 µL RPMI-1640 serum-free medium, respectively. Then, the cells were incubated at 4°C for 1 h, and the cells were washed twice with PBS to remove the unbound primary antibody. Then, the target cells were incubated with 200 µL PE-labeled secondary antibody at 2.5 µg/mL at 4°C for 30 min, and washed twice with PBS to remove the unbound secondary antibody. Finally, the cells were resuspended in 200 µL PBS, and tested for the binding affinity of the antibody to be tested to ROR1 on cell surface by fluorescence-activated cell sorter (FACS).

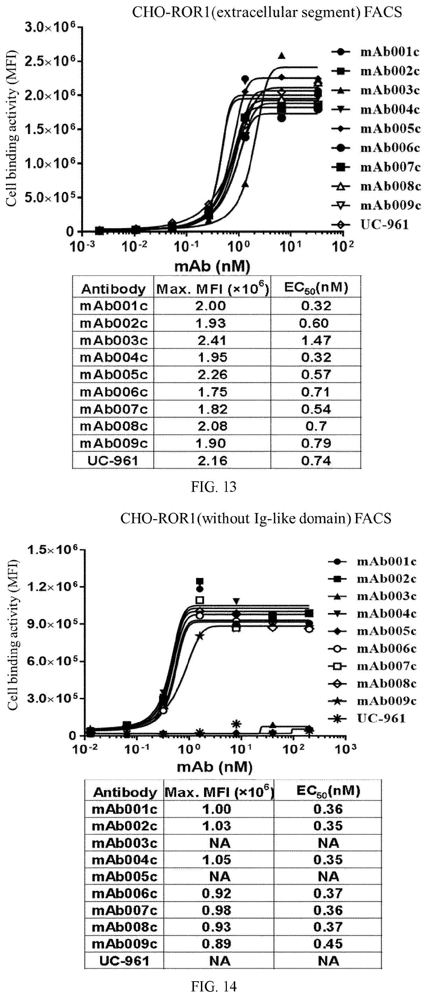

The test results are shown in FIG. 13. mAb001c, mAb002c, mAb003c, mAb004c, mAb005c, mAb006c, mAb007c, mAb008c, and mAb009c had excellent binding affinity to CHO-ROR1 (extracellular segment), with EC₅₀ of 0.32nM, 0.60nM, 1.47nM, 0.32nM, 0.57nM, 0.71nM, 0.54nM, 0.7nM and 0.79nM, respectively. Among them, the binding activity of mAb001c, mAb004c, mAb005c and mAb007c was significantly better than that of UC-961 (EC₅₀ was 0.74nM).

The test results are shown in FIG14. mAb001c, mAb002c, mAb004c, mAb006c, mAb007c, mAb008c, and mAb009c had excellent binding affinity to CHO-ROR1 (with Ig-like domain removed), with EC₅₀ of 0.36nM, 0.35nM, 0.35nM, 0.37nM, 0.36nM, 0.37nM, and 0.45nM, respectively. mAb003c and mAb005c, similar to UC-961, exhibited substantially no binding to CHO-ROR1 (with the Ig-like domain removed).

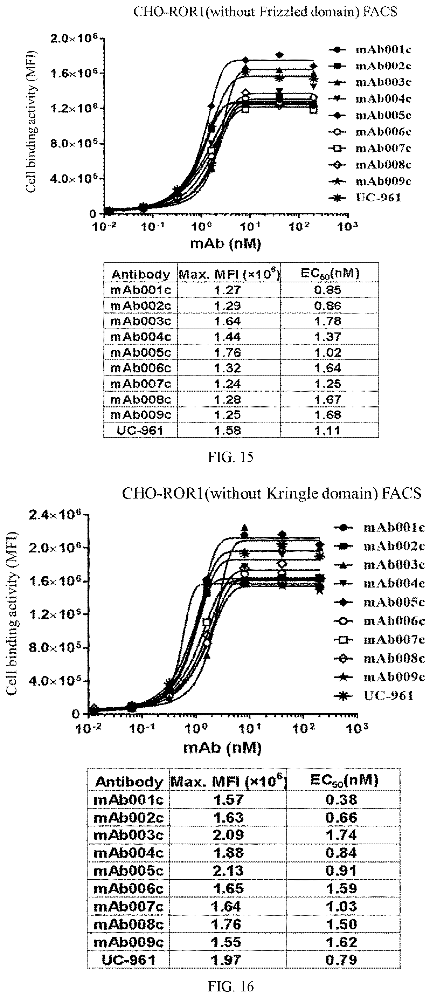

The test results are shown in FIG. 15. mAb001c, mAb002c, mAb003c, mAb004c, mAb005c, mAb006c, mAb007c, mAb008c, and mAb009c had excellent binding affinity to CHO-ROR1 (with Frizzled domain removed), with EC₅₀ of 0.85nM, 0.86nM, 1.78nM, 1.37nM, 1.02nM, 1.64nM, 1.25nM, 1.67nM, and 1.68nM, respectively. Among them, the binding activity of mAb001c, mAb002c, and mAb005c was significantly better than that of UC-961 (EC₅₀ was 1.11nM).

The test results are shown in FIG. 16. mAb001c, mAb002c, mAb003c, mAb004c, mAb005c, mAb006c, mAb007c, mAb008c, and mAb009c had excellent binding affinity to CHO-ROR1 (with Kringle domain removed), with EC₅₀ of 0.38nM, 0.66nM, 1.74nM, 0.84nM, 0.91nM, 1.59nM, 1.03nM, 1.50nM, and 1.62nM, respectively. Among them, the binding activity of mAb001c and mAb002c was significantly better than that of UC-961 (EC₅₀ was 0.79nM).

In summary, the results show that the chimeric antibodies mAb001c to mAb009c of this example had excellent binding affinity (EC₅₀ < 2 nM) for ROR1 transferred onto the cell surface; more importantly, the binding region of ROR1 for mAb001c, mAb002c, mAb004c, mAb006c, mAb007c, mAb008c, and mAb009c was a non-Ig-like domain (Frizzled or Kringle domain), which was significantly different from UC-961 (binding to the Ig-like domain as shown in US20150232569A1, which is consistent with the results of this example), and for mAb003c and mAb005c was the ROR1-Ig-like region, with the binding activity of mAb001c and mAb005c for ROR1 significantly better than that of UC-961. Therefore, the antibodies of this example have significant differentiation advantages over UC-961.

### Example 10. Antitumor activity of ROR1 human-mouse chimeric antibody in an immunodeficient mouse transplant tumor model

Immunodeficient nude mice (Balb /c-nude) were randomly divided into 4 groups. 50µL of cell suspension containing 5×10⁶ NCI-H226 cells was mixed with 50µL of the human-mouse chimeric antibody indicated at final concentration of 50µg/tumor. 100µL of matrix gel was mixed with the above 100µL cell/antibody mixture and inoculated into the subcutaneous part of the back of nude mice (n=6). hIgG was used as a negative control. The inhibitory effect of human-mouse antibody on subcutaneous tumor growth was observed regularly, and the body weight and tumor size of nude mice were measured 2-3 times a week. The tumor growth curve was plotted to evaluate the activity.

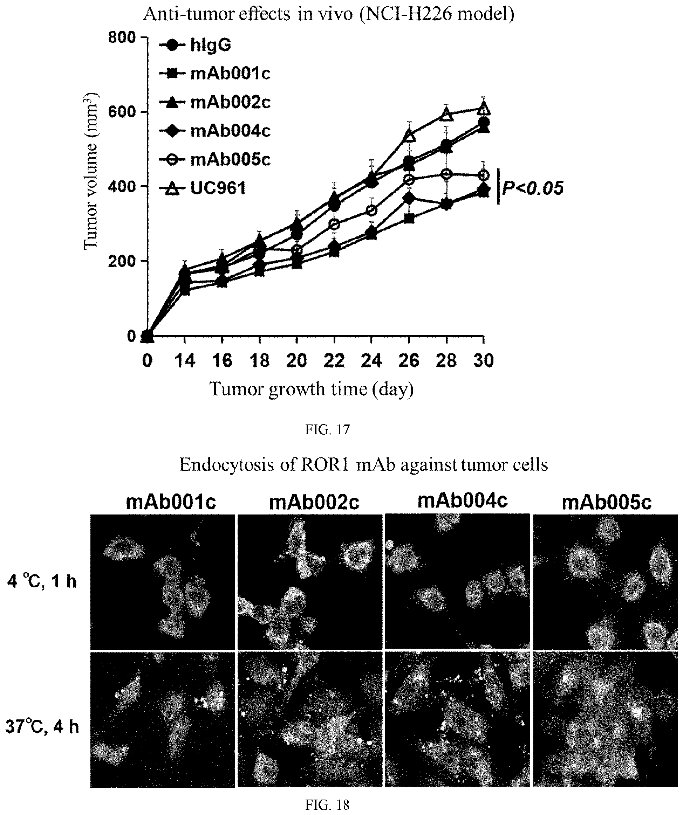

The results are shown in FIG. 17. The human-mouse chimeric antibodies mAb001c, mAb004c, and mAb005c can significantly inhibit the growth of NCI-H226 tumors in nude mice (vs hIgG group, P<0.05). The control drug UC-961 had basically no significant inhibitory activity on NCI-H226 tumors.

### Example 11 Binding of ROR1 human-mouse chimeric antibody to tumor cells resulting in internalization into intracellular lysosomes

The ROR1 high-expressing MDA-MB-231 cells at a density of 60% were plated in a laser confocal culture dish and cultured overnight. 5 µg/mL of ROR1 human-mouse chimeric antibodies mAb001c, mAb002c, mAb004c, and mAb005c were added and incubated at 37°C for 4 h and at 4°C for 1 h, respectively. The cells were washed three times with PBS to remove unbound human-mouse chimeric antibodies and fixed with 4% paraformaldehyde at room temperature for 30 min. The cells were washed three times with PBS and permeabilized with 0.4% TritonX-100 for 10 min. After washing three times with PBS, rabbit anti-human LAMP-2 antibody was added and incubated at 37°C for 1 h to mark the location of cell lysosomes. After unbound antibodies were washed off with PBS, R-PE-labeled goat anti-human and Alexa Fluor 488-labeled donkey anti-rabbit secondary antibodies were added and incubated at 37°C for 30 min. Unbound secondary antibody was washed away, and the cells were stained with DAPI for 10 min to mark the location of the cell nucleus. The antibody internalization was observed using a laser confocal microscope (20×).

The results are shown in FIG. 18. mAb001c, mAb002c, mAb004c, and mAb005c can be rapidly and significantly internalized into lysosomes by MDA-MB-231 cells. The results show that the human-mouse chimeric antibody of the present application is suitable for the preparation of antibody-drug conjugates (ADCs), suggesting that ROR1 ADCs will have good ADC drug properties and can be used for the prospect of broad-spectrum and highly specific targeted ROR1-positive tumor therapeutic drugs.

### Example 12. coupling andpreparation of mAb001c-vc-MMAE

The human-mouse chimeric antibody mAb001c (7.088 mg/ml) targeting ROR1 was placed in a 600 ml centrifuge bottle, and 50 mM PBS (pH=7.4) buffer was added to dilute the human-mouse chimeric antibody to a concentration of 5 mg/ml. A 100 mM EDTA aqueous solution was added to form 5% of the total volume of the reaction solution and mixed well by shaking, and 2 mg/ml TCEP aqueous solution was added for reduction of the human-mouse chimeric antibody at a molar ratio of TCEP to human-mouse chimeric antibody of 3:1. After mixing well by shaking, the mixture was placed on a cooling-type thermomixer for reaction at 37°C for 2 h. A solution of vcMMAE (purchased from Haoyuan Chemexpress, Shanghai) in DMSO was added at a final molar ratio of drug to human-mouse chimeric antibody of 8:1, and DMSO was supplemented to form 10% of the total volume of the reaction solution. After mixing well by shaking, the mixture was placed on the cooling-type thermomixer for reaction at 4°C for 1 h. The sample preservation buffer was replaced using an ultrafiltration tube (MWCO 30KD, manufactured by Millipore), and ultrafiltration was performed three times with 30 mM His-Hac buffer (pH 5.5) containing 10% DMSO, and further performed six times with 30 mM His-Hac (pH 5.5) without DMSO. The sample was sterilized through a 0.22 µm pore size filter and stored at -80 °C. The resulting antibody conjugate was named mAb001c-vcMMAE.

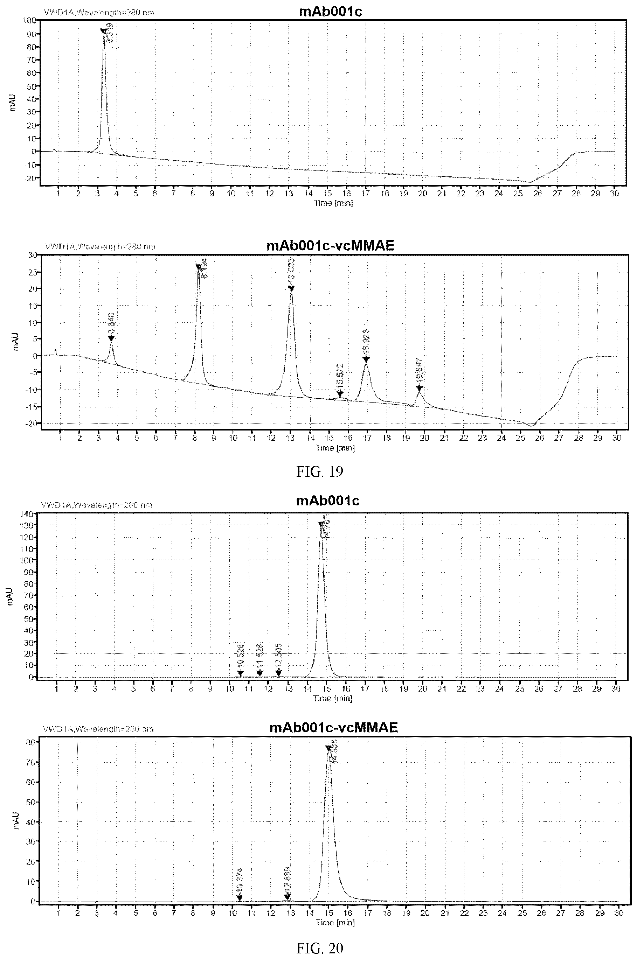

The results are shown in FIGS. 19 and 20. The HIC-HPLC (FIG. 19) and SEC-HPLC (FIG. 20) of the human-mouse chimeric antibody mAb001c and its antibody conjugate mAb001c-vc-MMAE both showed that after the coupling reaction, the human-mouse antibody mAb001c formed an antibody conjugate mAb001c-vc-MMAE with a relatively high purity (>98%). The molecular weight of the conjugate was consistent with the expected value, with the average DAR value of about 3.81.

### Example 13 In vitro anti-proliferative activity of ROR1 human-mouse chimeric antibody drug conjugate against ROR1-high-expressing tumor cells

The cell lines used in this example, including MCF-7, MDA-MB-231, NCI-N87, NCI-H446, HT-29, HCC827, and HCC1187 cells, were purchased from Cobioer Biosciences, Nanjing, and Yuchi Biology, Shanghai, and cultured according to the corresponding instructions. The above cells in logarithmic growth phase were inoculated into 96-well cell culture plates at a density of 250 to 1500 cells per well (depending on the growth rate of different cells) at 150 µL/well, and cultured at 37°C, 5% CO₂ for about 3 to 5 hours. Then, mAb001c-vcMMAE at different concentrations were added, each drug concentration in 2 to 4 replicates, as well as the corresponding vehicle control and blank control wells. After 5 to 6 days of action (depending on the cell growth rate, to ensure a sufficient number of cell divisions), the culture medium was removed and CCK8 reaction solution (purchased from MCE, cat# HY-K0301) was added at 100 µL/well. The reaction was carried out at 37°C until the expected color depth. The cell viability (OD450 nm) of each group was measured, and the cell survival rate was calculated according to the following formula: survival rate = (OD drug-OD blank)/(OD control-OD blank) × 100%. The above data were analyzed by GraphPad Prism 5 software, and the IC₅₀ values of the above ROR1 antibody-drug conjugates for different cell lines were calculated.

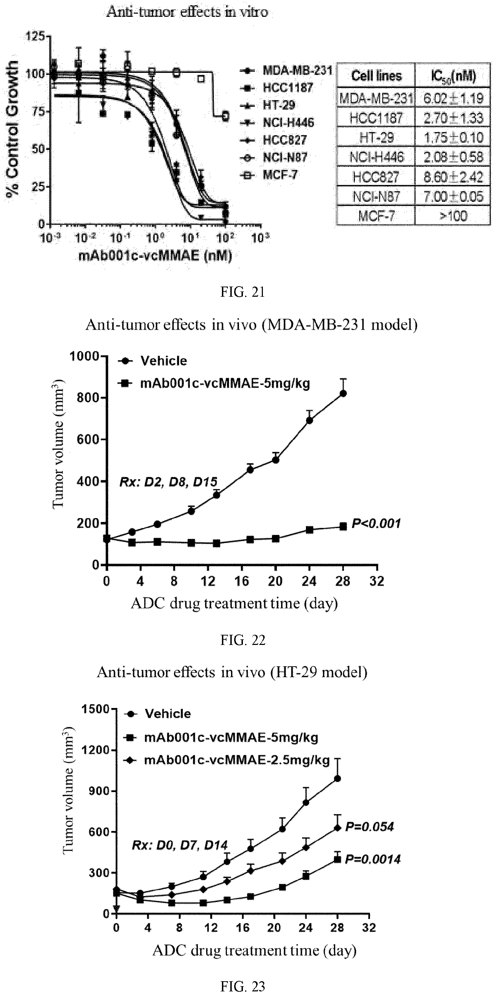

As shown in FIG. 21, mAb001C-vcMMAE had no significant inhibitory effect on the proliferation of ROR1-low-expressing MCF-7 cells (IC₅₀ >100nM), but showed strong inhibitory effect on ROR1-high-expressing MDA-MB-231, NCI-N87, NCI-H446, HT-29, HCC827, and HCC1187 cells (IC₅₀ <10nM).

Overall, the cytotoxicity (IC₅₀ value) of the ROR1-ADC indicated that the ROR1-ADC had cytotoxic activity directly correlated with the ROR1 expression level of the tested cells, and thus was determined to be ROR1 target-specific cytotoxic.

### Example 14 In vivo antitumor activity of ROR1 human-mouse chimeric antibody drug conjugate

200 µL of cell matrix gel suspension containing 5×10⁶ MDA-MB-231 and HT-29 were inoculated subcutaneously on the back of immunodeficient mice (NCG or Balb/c-nude). When the tumor volume grew to 100 to 200 mm³, the mice were randomly divided into groups (n=10) according to the tumor volume and nude mouse weight. The mice were dosed at doses of 5 mg/kg and 2.5 mg/kg once a week through the tail vein for a total of 3 weeks, as well as a group with PBS set as a negative control. The tumor volume and nude mouse weight were measured and recorded 2 to 3 times a week to plot the tumor growth curve. The formula for calculating tumor volume (V) is: V=1/2×a×b², where a and b represent the length and width of the tumor, respectively.

As shown in FIG. 22, 5 mg/kg of mAb001c-vcMMAE can completely inhibit the growth of MDA-MB-231 tumors with high expression of ROR1 (P<0.001), and no significant tumor growth recovery was observed after 2 weeks of drug withdrawal.

As shown in FIG. 23, mAb001c-vcMMAE significantly and dose-dependently inhibited the growth of HT-29 tumors when administered at 5 mg/kg and 2.5 mg/kg (P = 0.0014, P = 0.054).

### Example 15 Comparison of the present ROR1 human-mouse chimeric antibody with conventional technology

The heavy chain and light chain variable region sequences (VH/VL) of the UC-961 antibody disclosed in the invention patent application number US20150232569A1 were artificially synthesized and cloned into a vector containing the human IgG1 heavy chain constant region and the Kappa chain constant region. After comfirmed by sequencing, UC-961 was expressed and purified in the Expi-293F cell system. The experimental conditions for antibody preparation were consistent with those in Examples 3 and 15.
**UC-961-heavy chain variable region (VH) SEQ ID NO. 87** 
QVQLQESGPGLVKPSQTLSLTCTVSGYAFTAYNIHWVRQAPGQGLEWMGSFDPYDGGS
SYNQKFKDRLTISKDTSKNQVVLTMTNMDPVDTATYYCARGWYYFDYWGHGTLVTVSS
**UC-961-light chain variable region (VL) SEQ ID NO. 88**

DIVMTQTPLSLPVTPGEPASISCRASKSISKYLAWYQQKPGQAPRLLIYSGSTLQSGIPPRF
SGSGYGTDFTLTINNIESEDAAYYFCQQHDESPYTFGEGTKVEIK

The activity results of the comparative test are summarized as follows:

Referring to the assay methods in Examples 4, 8 and 9, the ELISA affinity of mAb001c, mAb005c and UC-961 against ROR1-ECD protein, the affinity of mAb001c, mAb005c and UC-961 against cell surface ROR1 and the activity of mAb001c, mAb005c and UC-961 to antigen binding region (epitope) were tested. The comparison results are summarized in Table 5;

**Table 5: Antibody test activity**

| Antibody Name | EC₅₀ for ROR1-ECD protein binding (nM) | EC₅₀ for MDA-MB-23 1 ROR1 binding (nM) | EC₅₀ for NCI-H226 ROR1 binding (nM) | EC₅₀ for CHO-ROR1 binding (nM) | ROR1-ECD region (Ig, Frizzled, Kringle) binding activity |
|---|---|---|---|---|---|
| mAb001c | 0.012 | 0.15 | 0.11 | 0.32 | Frizzled or/and Kringle region |
| mAb004c | 0.012 | 0.40 | 0.46 | 0.32 | Frizzled or/and Kringle region |
| mAb005c | 0.009 | 0.24 | 0.18 | 0.57 | Ig region |
| UC-961 | 0.013 | 0.21 | 0.13 | 0.74 | Ig region |

2. Referring to the detection method in Example 10, the in vivo anti-tumor effects of mAb001c, mAb005c and UC-961 on the NCI-H226 transplanted tumor model with high expression of ROR1 were detected. The comparison results are summarized in Table 6.

**Table 6: Antitumor activity of antibodies in nude mice**

| Antibody Name | Tumor volume ± SE on day 30 (mm³) | Tumor inhibition rate (%) | *P value (vs. hIgG)* |
|---|---|---|---|
| hIgG | 571.78±45.42 | Control | Control |
| mAb001c | 384.16±62.01 | 33 | *<0.05* |
| mAb004c | 393.40±32.28 | 31 | *<0.01* |
| mAb005c | 429.48±36.20 | 25 | *<0.05* |
| UC-961 | 609.52±30.33 | *ns* | *ns* |

In summary, the human-mouse chimeric antibody of the present application has following features:
(1) It has excellent biological activity and specificity, specifically, the preferred antibody has a high affinity for ROR1-ECD, with EC₅₀ value of 0.009 nM to 0.05 nM as determined by ELISA. In addition, the preferred antibody has a good binding affinity for ROR1 on the surface of tumor cells, with EC₅₀ value of 0.15 nM to 1.1 nM as determined by FACS, and can be used as a therapeutic antibody targeting ROR1.
(2) It has a unique binding epitope for ROR1, with better binding activity and binding ability.
(3) The antibody-drug conjugate (ADC) has specific ROR1-dependent anti-tumor activity. The preferred antibody-drug conjugate (ADC) has no obvious toxic side effects on ROR1-low-expressing cells, but has extremely high killing activity on ROR1-high-expressing tumor cells, with IC₅₀ value of 1 nM to 9 nM as determined by cell proliferation inhibition test.
(4) Both the antibody and ADC have significant in vivo anti-tumor activity, and have no obvious toxic and side effects on mammals themselves such as model mice.

### Example 16 Preparation of humanized antibodies

A humanized template that best matches the non-CDR regions (Framework regions) of mAb001c was searched and selected from the Germline database. The CDR regions of the antibody were then transplanted to the selected humanized template to replace the CDR region of the human template, and then recombined with the IgG1,κ constant region. Meanwhile, based on the three-dimensional structure of the mouse antibody, back mutations were performed on the buried residues, the residues that directly interacted with the CDR region, and the residues that had an important impact on the conformation of VL and VH.

Specifically, the humanization of mAb001c was implemented to obtain 5 humanized heavy chain variable regions (SEQ ID NO.28, SEQ ID NO.29, SEQ ID NO.30, SEQ ID NO.31, and SEQ ID NO.32), and 2 humanized light chain variable regions (SEQ ID NO.33, and SEQ ID NO.34).
**SEQ ID NO.28** mAb001_VHg0 
QVQLVQSGAEVKKPGASVKVSCKASGYTFTDYNLHWVRQAPGQGLEWMGYINPNHDATSYNQKF
QGRVTMTRDTSTSTVYMELSSLRSEDTAVYYCARRVRTGTGLDDWGQGTTVTVSS
**SEQ ID NO.29** mAb001_VHg1 
QVQLVQSGAEVKKPGASVKVSCKASGYTFTDYNLHWVRQAPGQGLEWMGYINPNHDATSYNQKF
QGKVTMTVDTSTSTVYMELSSLRSEDTAVYYCARRVRTGTGLDDWGQGTTVTVSS
**SEQ ID NO.30** mAb001_VHg2 
QVQLVQSGAEVKKPGASVKVSCKASGYTFTDYNLHWVKQAPGQGLEWIGYINPNHDATSYNQKFQ
GKVTMTVDTSTSTVYMELSSLRSEDTAVYYCARRVRTGTGLDDWGQGTTVTVSS
**SEQ ID NO.31** mAb001_VHg3 
QVQLVQSGAEVKKPGASVKVSCKASGYTFTDYNLHWVKQAPGQGLEWIGYINPNHDA
TSYNQKFQGKATLTVDTSTSTVYMELSSLRSEDTAVYYCARRVRTGTGLDDWGQGTTVTVSS
**SEQ ID NO.32** mAb001_VHg4 
QVQLVQSGAEVKKPGASVKVSCKASGYTFTDYNLHWVKQAPGQGLEWIGYINPNHDA
TSYNQKFQGKATLTVDTSTSTAYMELSSLTSEDTAVYYCARRVRTGTGLDDWGQGTTVTVSS
**SEQ ID NO.33** mAb001_VLg1 
DVVMTQSPLSLPVTLGQPASISCRSSHNIVHTNANTYLEWYQQRPGQSPRLLIYKVSNR
FSGVPDRFSGSGSGTDFTLKISRVEAEDVGVYYCFQGSRFPYTFGGGTKVEIK
**SEQ ID NO.34** mAb001_VLg2

DVVMTQSPLSLPVTLGQPASISCRSSHNIVHTNANTYLEWYQQRPGQSPKLLIYKVSNR
FSGVPDRFSGSGSGTDFTLKISRVEAEDVGVYYCFQGSRFPYTFGGGTKVEIK

Specifically, the humanization of mAb005c was implemented to obtain 8 humanized heavy chain variable regions (SEQ ID NO. 93, SEQ ID NO. 94, SEQ ID NO. 95, SEQ ID NO. 96, SEQ ID NO. 97, SEQ ID NO. 98, SEQ ID NO. 99, and SEQ ID NO. 100) and 1 humanized light chain variable region (SEQ ID NO. 101).
**SEQ ID NO.93** mAb005_VHg8 
EVQLVESGGGLVKPGGSLRLSCAASGFTFNSYAMSWVRQTPEKRLEWVATISDAGSYTY
YPDNEKGRFTISRDNAKNNLYLQMNSLRAEDTAIYYCVRGGYYYNLYAMDYWGQGTLVTVSS
**SEQ ID NO.94** mAb005_VHg13 
EVQLVESGGGLVKPGGSLRLSCAASGFTFNSYAMSWVRQTPEKGLEWVATISDAGSYTY
YPDNEKGRFTISRDNAKNNLYLQMNSLRAEDTAIYYCVRGGYYYNLYAMDYWGQGTLVTVSS
**SEQ ID NO.95** mAb005_VHg14 
EVQLVESGGGLVKPGGSLRLSCAASGFTFNSYAMSWVRQTPGKRLEWVATISDAGSYT
YYPDNEKGRFTISRDNAKNNLYLQMNSLRAEDTAIYYCVRGGYYYNLYAMDYWGQGTLVTVSS
**SEQ ID NO.96** mAb005_VHg15 
EVQLVESGGGLVKPGGSLRLSCAASGFTFNSYAMSWVRQTPEKRLEWVATISDAGSYTY
YPDNEKGRFTISRDNAKNSLYLQMNSLRAEDTAIYYCVRGGYYYNLYAMDYWGQGTLVTVSS
**SEQ ID NO.97** mAb005_VHg16 
EVQLVESGGGLVKPGGSLRLSCAASGFTFNSYAMSWVRQTPGKGLEWVATISDAGSYT
YYPDNEKGRFTISRDNAKNNLYLQMNSLRAEDTAIYYCVRGGYYYNLYAMDYWGQGTLVTVSS
**SEQ ID NO.98** mAb005_VHg17 
EVQLVESGGGLVKPGGSLRLSCAASGFTFNSYAMSWVRQTPGKGLEWVATISDAGSYT
YYPDNEKGRFTISRDNAKNSLYLQMNSLRAEDTAIYYCVRGGYYYNLYAMDYWGQGTLVTVSS
**SEQ ID NO.99** mAb005_VHg18 
EVQLVESGGGLVKPGGSLRLSCAASGFTFNSYAMSWVRQTPEKGLEWVATISDAGSYTY
YPDNEKGRFTISRDNAKNSLYLQMNSLRAEDTAIYYCVRGGYYYNLYAMDYWGQGTLVTVSS
**SEQ ID NO.100** mAb005 _VHg19 
EVQLVESGGGLVKPGGSLRLSCAASGFTFNSYAMSWVRQTPGKRLEWVATISDAGSYT
YYPDNEKGRFTISRDNAKNSLYLQMNSLRAEDTAIYYCVRGGYYYNLYAMDYWGQGTLVTVSS
**SEQ ID NO.101** mAb005_VLg22

DIVLTQSPDSLAVSLGERATINCKASQSVSFPGTSLMHWYQQKPGQQPKLLIYRASNLE
AGVPDRFSGSGSRTDFTLTISSLQAEDVAVYYCQQSREYPWTFGGGTKVEIK

The designed humanized variable region was cloned into a vector containing the human IgG1 heavy chain constant region and the Kappa chain constant region by gene recombination technology. After comfirmed by sequencing, the heavy chain and light chain combination was expressed in mammalian expression system (Expi293F cells) using the constructed humanized antibody expression vector by transfection technology and. Finally, 9 humanized antibodies were obtained for mAb001c group and 8 humanized antibodies for mAb005c group. The corresponding heavy chain and light chain combinations of each antibody are shown in Table 7.

**Table- 7: Preparation of humanized antibodies**

| Humanized antibody name | VH | VL |
|---|---|---|
| Hu001-02 | SEQ ID NO.28 | SEQ ID NO.33 |
| Hu001-03 | SEQ ID NO.28 | SEQ ID NO.34 |
| Hu001-05 | SEQ ID NO.29 | SEQ ID NO.33 |
| Hu001-06 | SEQ ID NO.29 | SEQ ID NO.34 |
| Hu001-08 | SEQ ID NO.30 | SEQ ID NO.33 |
| Hu001-11 | SEQ ID NO.31 | SEQ ID NO.33 |
| Hu001-12 | SEQ ID NO.31 | SEQ ID NO.34 |
| Hu001-14 | SEQ ID NO.32 | SEQ ID NO.33 |
| Hu001-15 | SEQ ID NO.32 | SEQ ID NO.34 |
| Hu005-35 | SEQ ID NO.93 | SEQ ID NO.101 |
| Hu005-40 | SEQ ID NO.94 | SEQ ID NO.101 |
| Hu005-41 | SEQ ID NO.95 | SEQ ID NO.101 |
| Hu005-42 | SEQ ID NO.96 | SEQ ID NO.101 |
| Hu005-43 | SEQ ID NO.97 | SEQ ID NO.101 |
| Hu005-44 | SEQ ID NO.98 | SEQ ID NO.101 |
| Hu005-45 | SEQ ID NO.99 | SEQ ID NO.101 |
| Hu005-46 | SEQ ID NO.100 | SEQ ID NO.101 |

### Example 17. Binding affinity of humanized antibodies to ROR1-ECD

17 humanized antibodies in Table 7 were serially diluted, and their affinity to the ROR1-ECD protein was determined by ELISA. The experimental method was referred to Example 4.

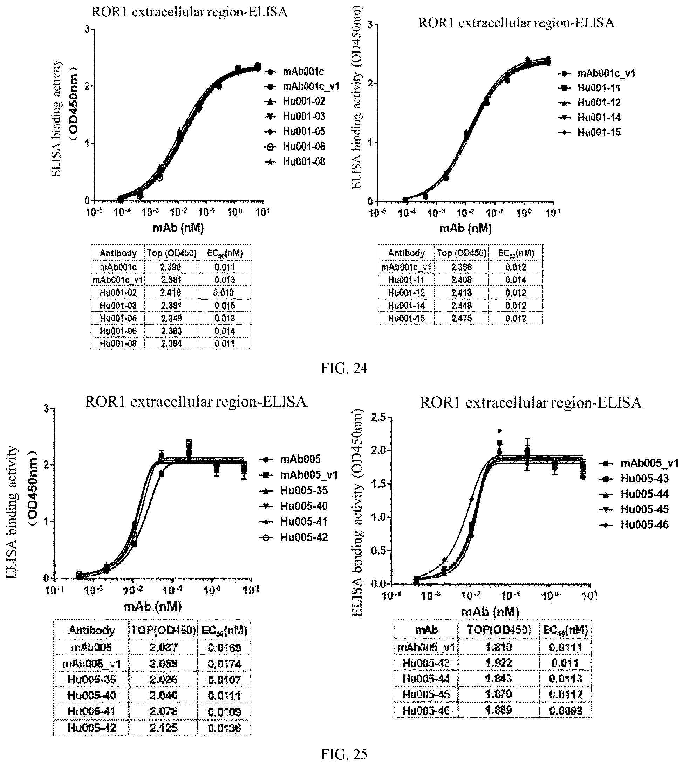

The experimental results are shown in FIG. 24. The humanized antibodies Hu001-02, 03, 05, 06, 08, 11, 12, 14, and 15 all had strong binding affinity to the ROR1-ECD protein, with EC₅₀ values of 0.010 nM to 0.015 nM.

The experimental results are shown in FIG. 2 5. The humanized antibodies Hu005-35, 40, 41, 42, 43, 44, 45, and 46 all had strong binding affinity to ROR1-ECD protein, with EC₅₀ values of 0.009 nM to 0.02 nM.

### Example 18 Binding Specificity of Humanized Antibodies to ROR1 Protein

The humanized antibodies Hu001-02 and Hu005-46 in Table 7 were serially diluted, and their affinity to the human ROR1-ECD and ROR2-ECD proteins was determined by ELISA. The experimental method was referred to Example 4.

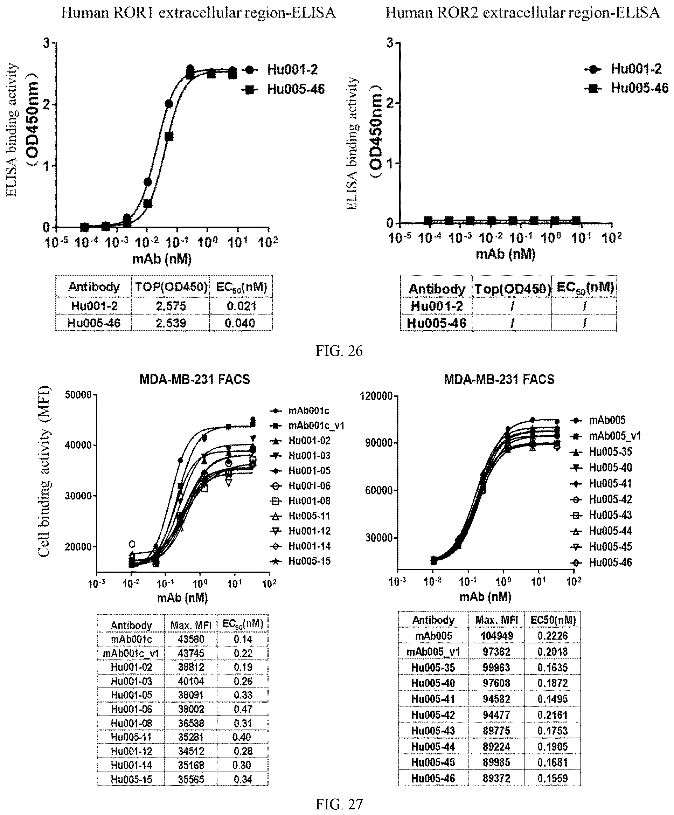

The experimental results are shown in FIG. 26. The humanized antibodies Hu001-02 and Hu005-46 bind specifically and with high affinity to the human ROR1-ECD protein, but have little binding activity to the human ROR2-ECD protein, indicating that the humanized antibodies have binding specificity to the ROR1 protein.

### Example 19. Binding affinity of humanized antibodies to ROR1 on the surface of tumor cells

The 17 humanized antibodies in Table 7 were serially diluted, and their affinity to ROR1 on the surface of MDA-MB-231 and NCI-H226 cells was determined by flow cytometry. The experimental method was referred to Example 8.

The experimental results are shown in FIG. 27. The humanized antibody has a high binding affinity activity to ROR1 on the surface of MDA-MB-231 cells. The EC50 values of Hu001-02, 03, 05, 06, 08, 11, 12, 14, and 15 for the binding were 0.19 nM to 0.47 nM; and the EC50 values of Hu005-35, 40, 41, 42, 43, 44, 45, and 46 for the binding were 0.16 nM to 0.22 nM.

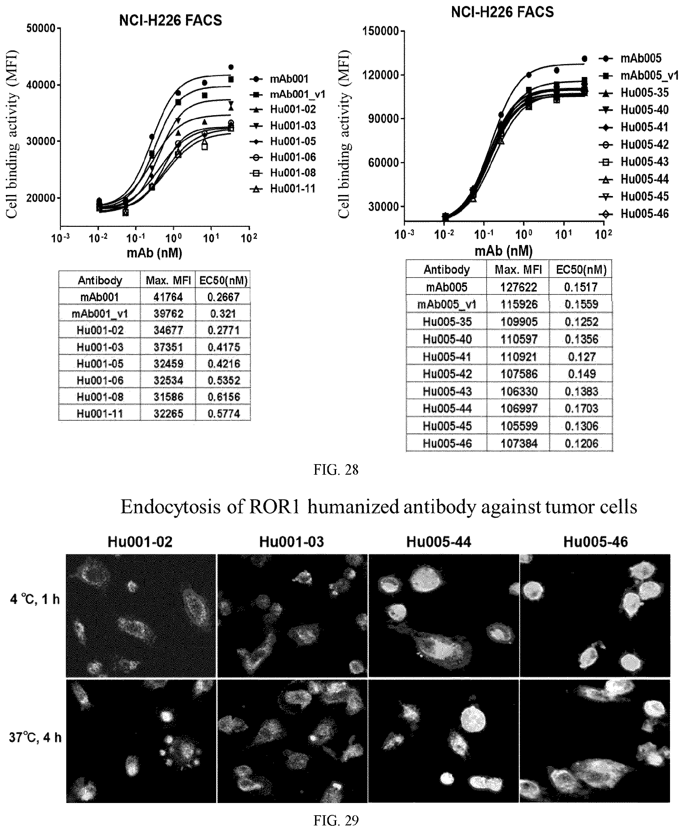

The experimental results are shown in FIG. 28. The humanized antibody has a high binding affinity activity to ROR1 on the surface of NCI-H226 cells. The EC50 values of Hu001-02, 03, 05, 06, 08, and 11 for the binding were 0.27 nM to 0.62 nM; and the EC50 values of Hu005-35, 40, 41, 42, 43, 44, 45, and 46 for the binding were 0.12 nM to 0.18 nM.

### Example 20. Binding of humanized antibodies to tumor cells resulting in internalization into intracellular lysosomes

MDA-MB-231 cells at a density of 60% were plated in a laser confocal culture dish. After the cells being cultured overnight at 37°C, 5 µg/mL of ROR1 humanized antibodies Hu001-02, Hu001-03, Hu005-44, and Hu005-46 were added, and incubated at 37°C for 4 h and 4°C for 1 h, respectively. The cells were washed three times with PBS to remove the antibodies that were not bound to the cells, and fixed with 4% paraformaldehyde at room temperature for 30 min. The cells were washed three times with PBS and permeabilized with 0.4% TritonX-100 for 10 min. After washing three times with PBS, the cells were incubated with Lamp-2 (rabbit anti-human) antibody at 37°C for 1 h to mark the location of cell lysosomes. After unbound antibodies were washed off with PBS, the cells were incubated with R-PE-labeled goat anti-human and Alexa Fluor 488-labeled donkey anti-rabbit secondary antibodies at 37°C for 30 min. Unbound secondary antibody was washed away, and the cells were stained with DAPI for 10 min to mark the location of the cell nucleus. The antibody internalization was observed using a laser confocal microscope (20×).

The results are shown in FIG. 29. Hu001-02, Hu001-03, Hu005-44, and Hu005-46 can be rapidly and significantly internalized into lysosomes by MDA-MB-231 cells. This result shows that the humanized antibody of the present application is suitable for preparing antibody-drug conjugates (ADCs) for broad-spectrum and highly specific targeted treatment of ROR1- overexpressing tumors.

### Example 21. Humanized Antibody Endocytosis Dynamics Detection

MDA-MB-231, NCI-N87 and NCI-H446 cells in logarithmic growth phase were collected and the cell density was adjusted to 1×10⁷ /mL. 600 µL of cell suspension and 600 µL of antibody working solution (60 µg/mL of Hu001-2, Hu001-3, Hu005-44, or Hu005-46) were taken for each group and incubated on ice for 30 min. The cells were washed twice with pre-cooled 1× PBS, collected by centrifugation and resuspended in 1.8 mL of 1640 culture medium (containing 2% FBS). 300 µL of cell suspension were taken and incubated at 37°C for 0.5 h, 1 h, 2 h, and 4 h (300 µL of cell suspension were taken as T0 and background reference). After incubation at each time point, the cells were immediately placed on ice to terminate the endocytosis, centrifuged and washed twice with pre-cooled 1× PBS, incubated on ice for 20 min after 100 µL of R-PE labeled goat anti-human secondary antibody addded, centrifuged and washed twice with pre-cooled 1 × PBS. 2% paraformaldehyde was added for 10 min and the fluorescence intensity (MFI) was determined by fluorescence-activated cell sorter (FACS).

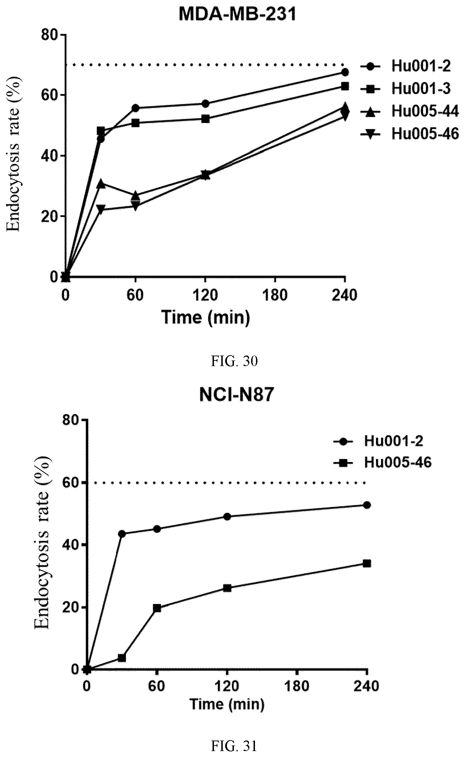

The results are shown in FIGS. 30, 31 and 32. Hu001-2, Hu001-3, Hu005-44 and Hu005-46 can mediate the internalization of the target into the cell to a large extent within 0.5 h to 1 h. Within the measured time of 4 h, the tested antibodies can basically achieve an internalization rate of 50%-70%. Among them, the internalization rate and degree of Hu001-2 (3) are better than those of Hu005-44 (46).

### Example 22 Preparation of humanized antibody-drug conjugates

The humanized antibodies Hu001-2, Hu001-3, Hu005-44 and Hu005-46 were preferred to be coupled with vcMMAE to prepare corresponding antibody-drug conjugates by using the specific coupling method referred to Example 12. The obtained antibody-drug conjugates were named Hu001-2-MMAE, Hu001-3-MMAE, Hu005-44-MMAE, Hu005-46-MMAE, respectively. At the same time, UC961-MMAE was prepared as a positive reference.

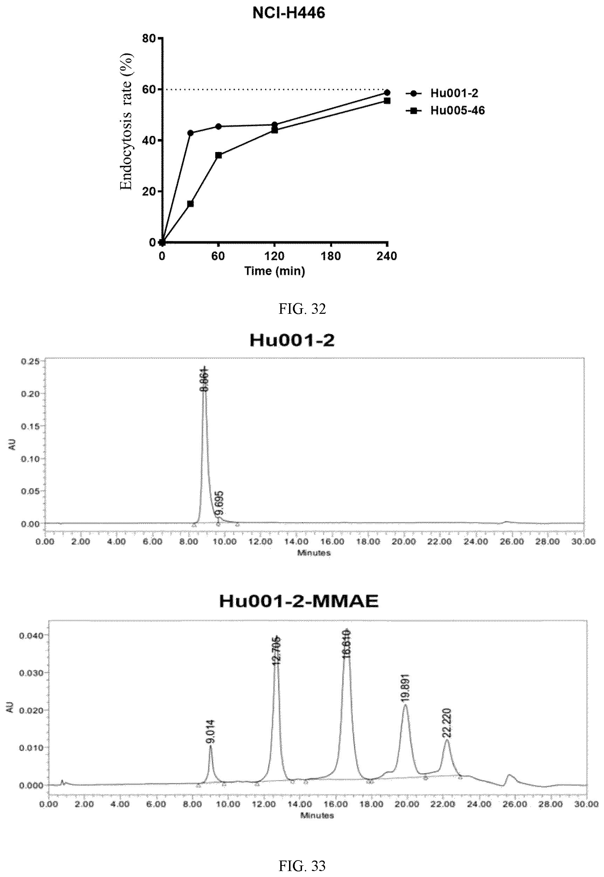

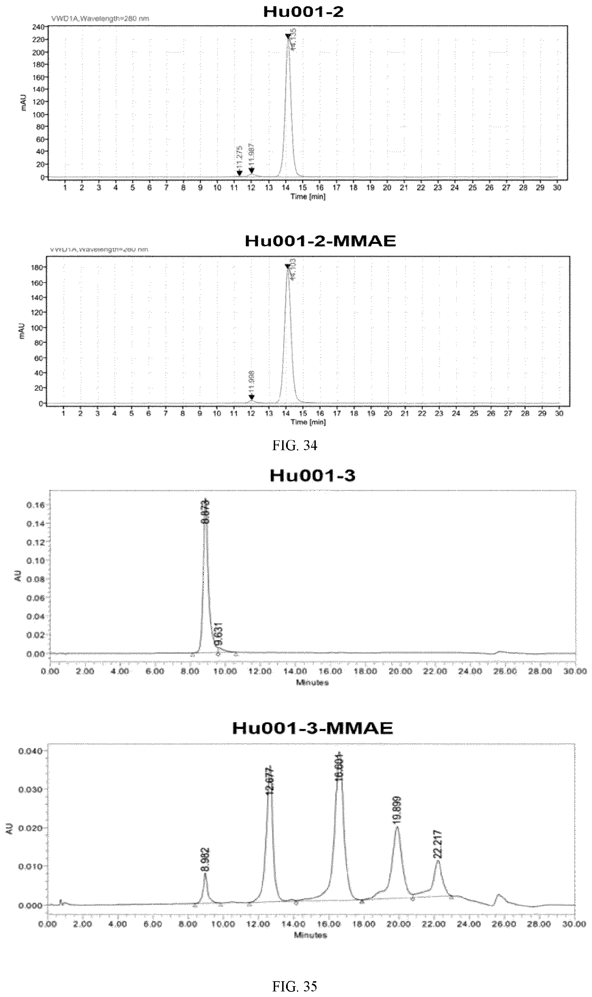

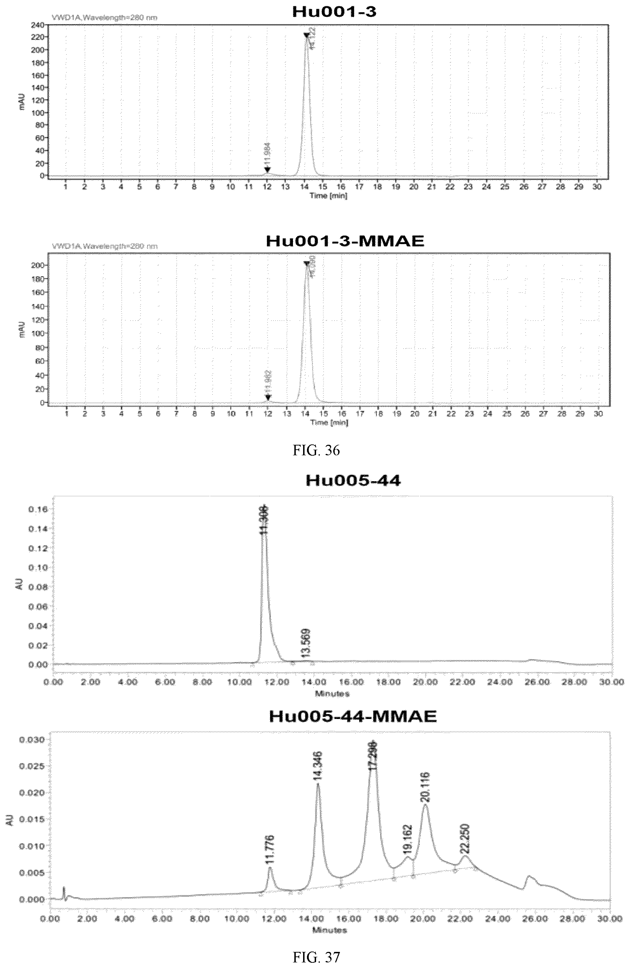

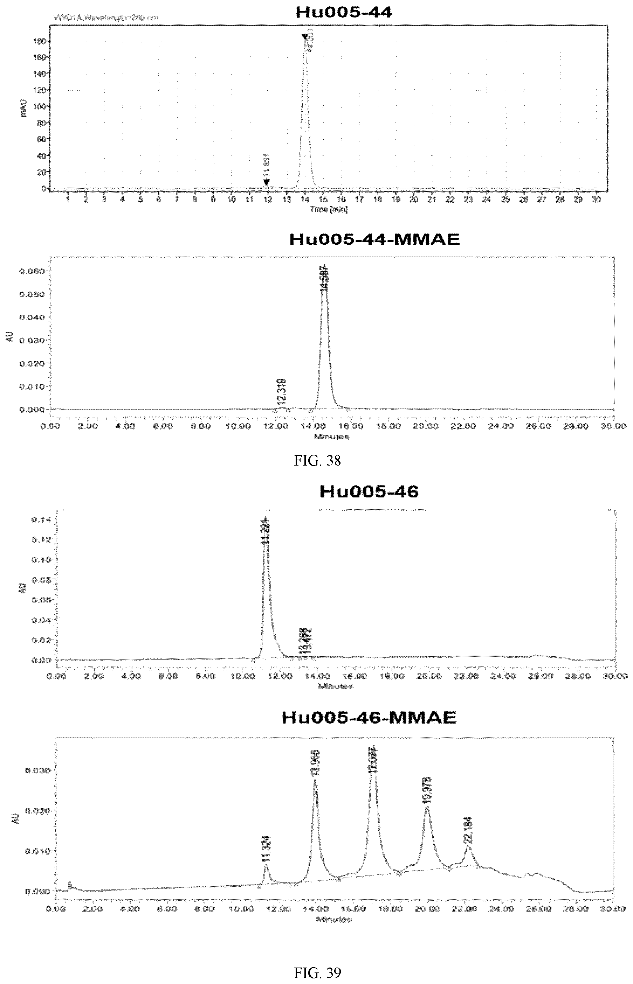

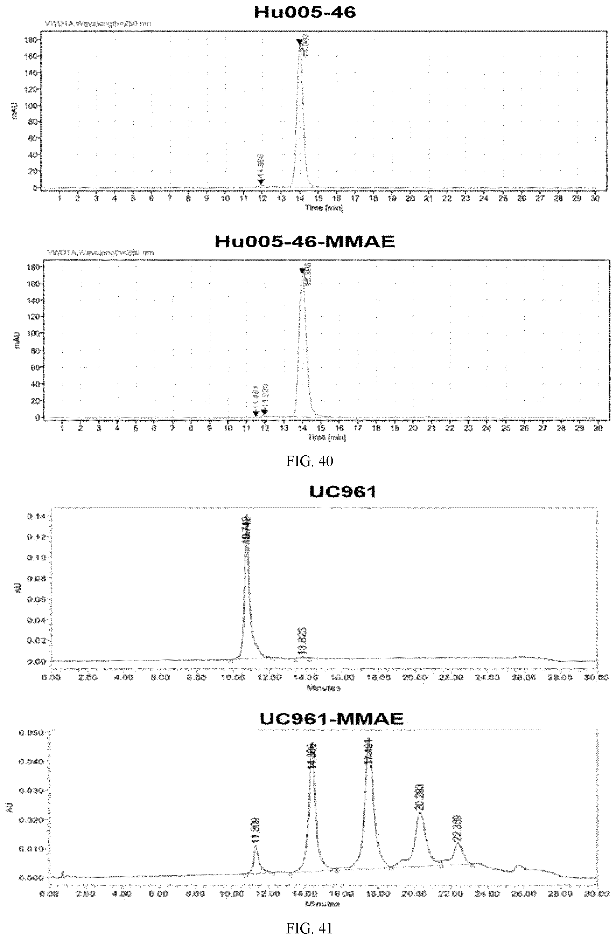

As shown in FIGS. 33 to 42, HIC-HPLC (FIGS. 33, 35, 37, 39, and 41) and SEC-HPLC (FIGS. 34, 36, 38, 40, and 42) for humanized antibodies Hu001-2, Hu001-3, Hu005-44, Hu005-46, UC961 and their antibody conjugates Hu001-2-MMAE, Hu001-3-MMAE, Hu005-44-MMAE, Hu005-46-MMAE, UC961-MMAE all showed that after the coupling reaction, the antibodies formed antibody conjugates with higher purity (> 97 %). The molecular weight of the conjugates was consistent with the expected value, with the average DAR values of about 4.15, 4.15, 4.12, 4.07, and 3.95, respectively.

### Example 23. Binding affinity of humanized antibody-drug conjugates to human ROR1 protein

Hu001-2-MMAE, Hu001-3-MMAE, Hu005-44-MMAE and Hu005-46-MMAE prepared in Example 22 were serially diluted, and their affinity for ROR1-ECD protein was determined by ELISA. Hu001-2, Hu001-3, Hu005-44 and Hu005-46 were used as parent antibody controls. The experimental method was referred to Example 4.

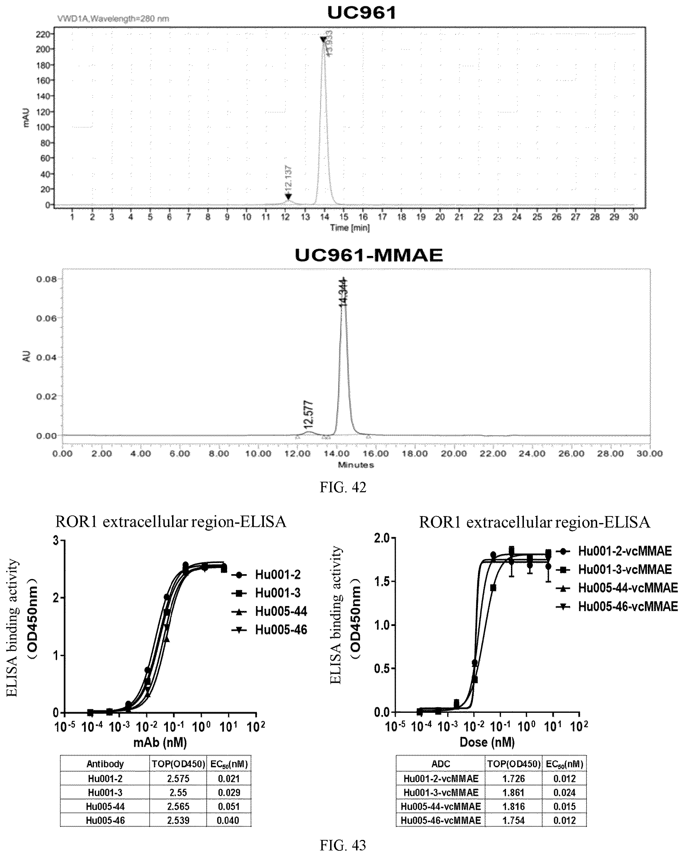

The results are shown in FIG. 43. The humanized antibody-conjugates can maintain the high binding affinity activity of the parent antibody against ROR1-ECD protein, with EC50 value for binding of 0.012 nM to 0.024 nM.

### Example 24. Binding affinity of humanized antibody-drug conjugates to ROR1 on the surface of tumor cells

Hu001-2-MMAE, Hu001-3-MMAE, Hu005-44-MMAE and Hu005-46-MMAE prepared in Example 22 were serially diluted, and their affinity to ROR1 on the surface of MDA-MB-231 and NCI-H226 cells was determined by fluorescence-activated cell sorter. Hu001-2, Hu001-3, Hu005-44 and Hu005-46 were used as parent antibody controls. The experimental method was referred to Example 8.

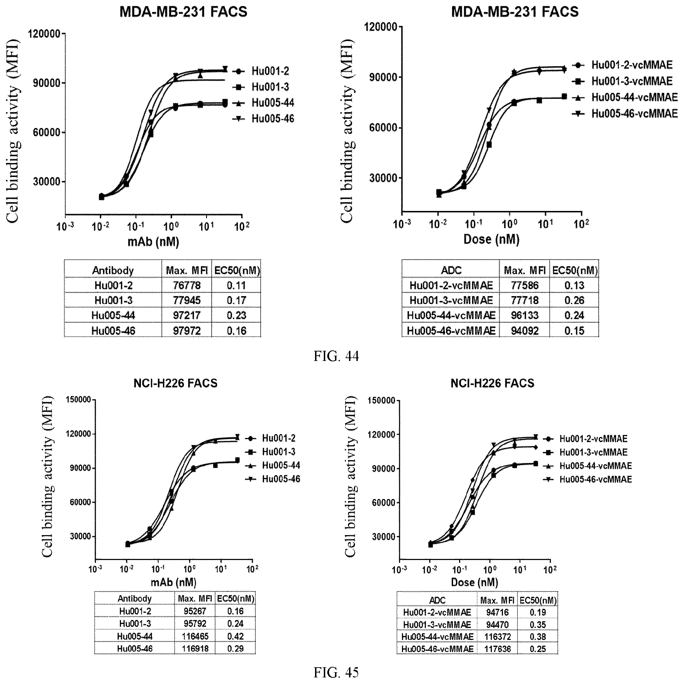

The results are shown in FIG. 44. The humanized antibody-conjugates can maintain the high binding affinity activity of the parent antibody against ROR1 on the surface of MDA-MB-231, with EC50 value for binding of 0.13 nM to 0.26 nM.

The results are shown in FIG. 45. The humanized antibody-conjugates can maintain the high binding affinity activity of the parent antibody against ROR1 on the surface of NCI-H226, with EC50 value for binding of 0.19 nM to 0.38 nM.

### Example 25 In vitro antiproliferative activity of humanized antibody-drug conjugates against tumor cells with high expression of ROR1

The cell lines used in this example, including breast cancer cell lines MCF-7, SK-BR-3, MDA-MB-231, and HCC1187, colorectal cancer cell lines HT-29, SK-CO-1, and COLO-678, lung cancer cell lines HCC827, and H446, ovarian cancer cell line PA-1, gastric cancer cell line NCI-N87, were purchased from Cobioer Biosciences, Nanjing, and Yuchi Biology, Shanghai, and cultured according to the corresponding instructions. The CCK8 method was used to determine the in vitro killing activity of Hu001-2-MMAE, Hu001-3-MMAE, Hu005-44-MMAE, and Hu005-46-MMAE against various cancer cells, as well as UC961-MMAE as a positive reference. The implementation method was referred to Example 13.

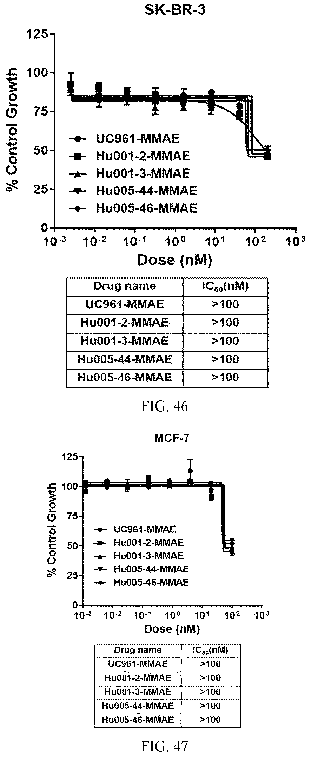

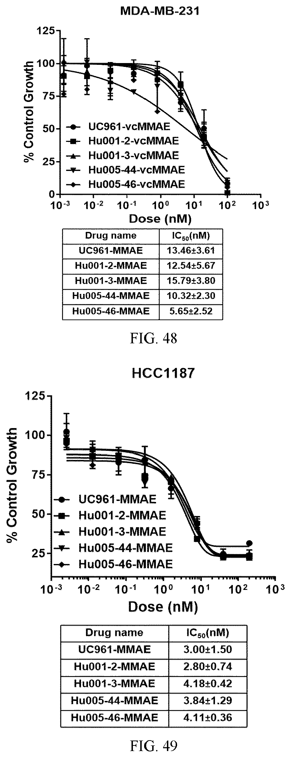

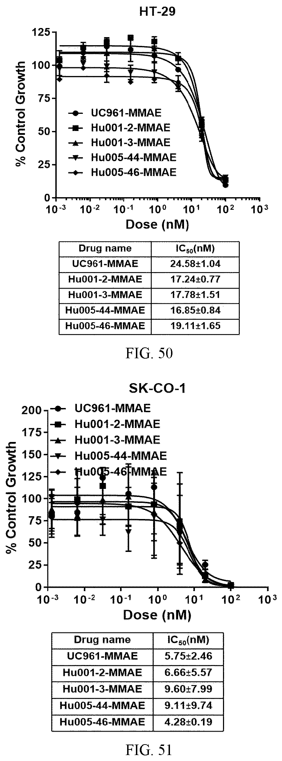

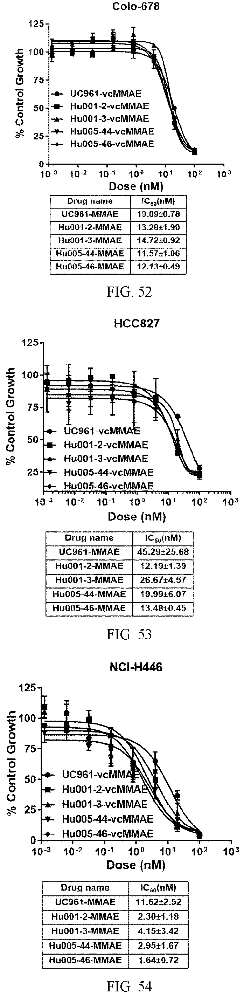

As shown in FIGS. 46 to 56, the humanized antibody-conjugates had no obvious inhibitory effect on the proliferation of SK-BR-3 and MCF-7 cells with low expression of ROR1 (IC50 > 100nM) (FIGS. 46, 47), but showed strong inhibitory effect on MDA-MB-231, HCC1187, HT-29, SK-CO-1, COLO-678, NCI-H446, HCC827, PA-1, and NCI-N87 cells with high expression of ROR1 (FIGS. 48, 49, 50, 51, 52, 53, 54, 55, and 56).

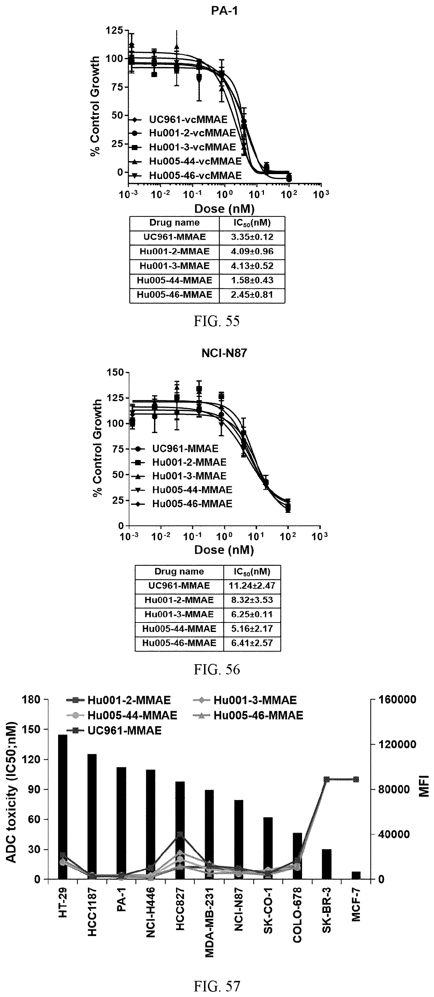

Overall, the cytotoxicity (IC50 value) of the ROR1 humanized antibody-drug conjugates indicated that the ROR1 humanized antibody-drug conjugates had cytotoxic activity directly correlated with the ROR1 expression level of the tested cells (FIG. 57), and thus was determined to be ROR1 target-specific cytotoxic. Table 8 summarizes the IC₅₀ values for the inhibition of test cell proliferation.

**Table- 8: In vitro antitumor activity of ROR1 humanized antibody -conjugates**

| Cancer type | Cell line | ROR1 level (MFI) | Proliferation inhibition rate IC50±SD (nM) | | | | |
|---|---|---|---|---|---|---|---|
| | | | Hu001-2-MM AE | Hu001-3-MM AE | Hu005-44-MMAE | Hu005-46-M MAE | UC961-MMAE |
| Breast cancer | SK-BR-3 | 26835.7 | >100 | >100 | >100 | >100 | >100 |
| | MCF-7 | 6610.4 | >100 | >100 | >100 | >100 | >100 |
| | MDA-MB-231 | 79464.6 | 12.54±5.67 | 15.79±3.80 | 10.32±2.30 | 5.65±2.52 | 13.46±3.61 |
| | HCC1187 | 111495.8 | 2.8±0.74 | 4.18±0.42 | 3.84±1.29 | 4.11±0.36 | 3.00±1.50 |
| Bowel cancer | HT-29 | 128559.5 | 17.24±0.77 | 17.78±1.51 | 16.85±0.84 | 19.11±1.65 | 24.58±1.04 |
| | SK-CO-1 | 54932.9 | 6.66±5.57 | 9.60±7.99 | 9.11±9.74 | 4.28±0.19 | 5.75±2.46 |
| | COLO-678 | 41436.9 | 13.28±1.90 | 14.72±0.92 | 11.57±1.06 | 12.13±0.49 | 19.09±0.78 |
| Lung cancer | NCI-H446 | 97567.9 | 2.30±1.18 | 4.15±3.42 | 2.95±1.67 | 1.64±0.72 | 11.62±2.52 |
| | HCC827 | 87015.2 | 12.19±1.39 | 26.67±4.57 | 19.99±6.07 | 13.48±0.45 | 45.29±25.68 |
| Ovarian cancer | PA-1 | 99645 | 4.09±0.96 | 4.13±0.52 | 1.58±0.43 | 2.45±0.81 | 3.35±0.12 |
| Gastric cancer | NCI-N87 | 70456.2 | 8.32±3.53 | 6.25±0.11 | 5.16±2.17 | 6.41±2.57 | 11.24±2.47 |

### Example 26. In vivo antitumor activity of humanized antibody-drug conjugate needles

200 µL of cell matrix gel suspension containing 5 × 10⁶ MDA-MB-231, 1 × 10⁷ HCC1187, 1 × 10⁷ PA-1, or 5 × 10⁶ NCI-N87 was inoculated subcutaneously on the back of immunodeficient mice (NCG or Balb/c-nude). When the tumor volume grew to 100 to 300 mm3, the mice were randomly divided into groups (n=8 to 10) according to the tumor volume and nude mouse weight. The mice were dosed at doses of 5 mg/kg and 2.5 mg/kg once a week through the tail vein for a total of 3 weeks, as well as a group with PBS set as a negative control. The tumor volume and nude mouse weight were measured and recorded 2 to 3 times a week to plot the tumor growth curve. The formula for calculating tumor volume (V) is: V=1/2×a×b2, where a and b represent the length and width of the tumor, respectively.

The in vivo anti-tumor results of four preferred ROR1 humanized antibody-conjugates: Hu001-2-MMAE, Hu001-3-MMAE, Hu005-44-MMAE, Hu005-46-MMAE and the positive reference UC961-MMAE are shown in FIGS. 57 to 60, respectively.

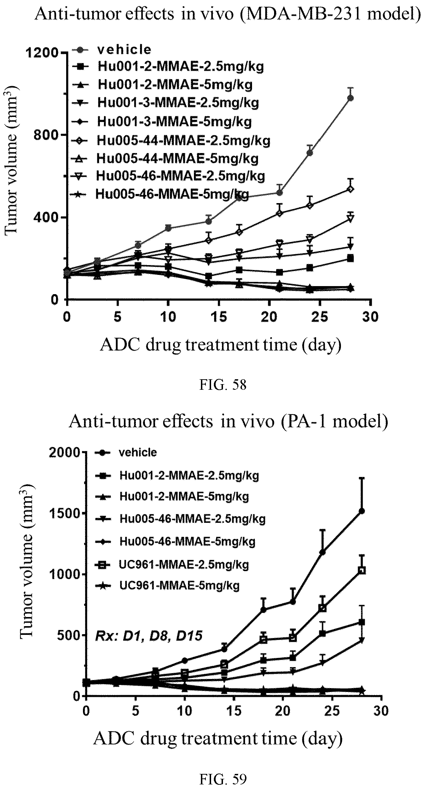

As shown in FIG. 58, in the MDA-MB-231 model, the four ROR1 humanized antibody-conjugates showed dose-related therapeutic effects when administered at 5 mg/kg and 2.5 mg/kg. At a dose of 5 mg/kg, the growth of MDA-MB-231 tumors with high ROR1 expression was completely inhibited (P< 0.0001), and no significant tumor growth recovery was observed after 2 weeks of drug withdrawal.

As shown in FIG. 59, in the PA-1 model, the Hu001-2-MMAE and Hu005-46-MMAE showed dose-related therapeutic effects when administered at 5 mg/kg and 2.5 mg/kg. At a dose of 5 mg/kg, the growth of PA-1 tumors with high expression of ROR1 was completely inhibited (P<0.0001; P <0.001) with the tumor regressed, and no significant tumor growth was observed after 2 weeks of drug withdrawal. Moreover, the anti-tumor activity at a dose of 2.5 mg/kg was significantly better than that of the positive reference UC961-MMAE.

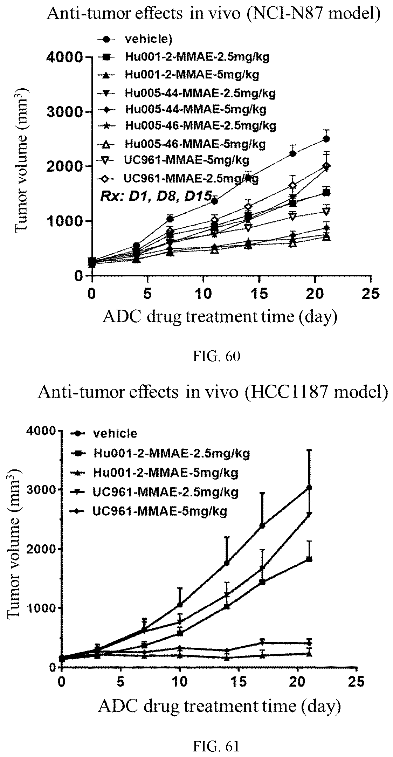

As shown in FIG. 60, in the NCI-N87 model, the Hu001-2-MMAE, Hu005-44-MMAE, and Hu005-46-MMAE showed dose-related therapeutic effects when administered at 5 mg/kg and 2.5 mg/kg. The growth of NCI-N87 tumors with medium-low expression of ROR1 was significantly inhibited at a dose of 5 mg/kg (P<0.0001), and the anti-tumor activity was significantly better than that of the positive reference UC961-MMAE.

As shown in FIG. 61, in the HCC1187 model, the Hu001-2-MMAE showed dose-related therapeutic effects when administered at 5 mg/kg and 2.5 mg/kg. At a dose of 5 mg/kg, the growth of HCC1187 tumors with high expression of ROR1 was completely inhibited (P < 0.001), and the anti- tumor activity was significantly better than that of the positive reference UC961-MMAE.

In general, the ROR1 humanized antibody-conjugate has significant anti-tumor activity against tumors with high, medium and low ROR1 expression. Tables 9 to 12 summarize the in vivo tumor inhibition rate (%) of the humanized antibody-conjugate.

### Table- 9: Tumor inhibition rate (%) of ROR1 humanized antibody-conjugate in MDA-MB-231 tumor model

| Administration group | Tumor volume ± SE (mm³) on day 28^{th} of administration | Tumor inhibition rate (%) | P value (vs. vehicle) |
|---|---|---|---|
| Vehicle | 979.34±49.70 | Control | Control |
| Hu001-2-MMAE-2.5mg/kg | 199.00±19.31 | 79.68 | <0.0001 |
| Hu001-2-MMAE-5mg/kg | 62.60±13.39 | 93.61 | <0.0001 |
| Hu001-3-MMAE-2.5mg/kg | 25 6.41±45.34 | 73.82 | <0.0001 |
| Hu001-3-MMAE-5mg/kg | 50.08±11.85 | 94.89 | <0.0001 |
| Hu005-44-MMAE-2.5mg/kg | 537.81±49.88 | 45.08 | <0.0001 |
| Hu005-44-MMAE-5mg/kg | 61.75±10.06 | 93.69 | <0.0001 |
| Hu005-46-MMAE-2.5mg/kg | 393.20±32.19 | 59.85 | <0.0001 |
| Hu005-46-MMAE-5mg/kg | 60.24±6.92 | 93.85 | <0.0001 |

### Table 10: Tumor inhibition rate (%) of ROR1 humanized antibody-conjugate in PA-1 tumor model

| Administration group | Tumor volume ± SE (mm³) on day 28^{th} of administration | Tumor inhibition rate (%) | P value (vs. vehicle) |
|---|---|---|---|
| Vehicle | 1519.79±269.13 | Control | Control |
| Hu001-2-MMAE-2.5mg/kg | 607.59±136.41 | 60.02 | <0.01 |
| Hu001-2-MMAE-5mg/kg | 64.01±7.56 | 95.79 | <0.0001 |
| Hu005-46-MMAE-2.5mg/kg | 456.21±136.19 | 69.98 | <0.01 |
| Hu005-46-MMAE-5mg/kg | 57.51±11.13 | 96.22 | <0.001 |
| UC961-MMAE-2.5mg/kg | 1032.13±123.29 | 32.09 | ns |
| UC961-MMAE-5mg/kg | 44.67±3.04 | 97.06 | <0.001 |

### Table 11: Tumor inhibition rate (%) of ROR1 humanized antibody-conjugate in NCI-N87 tumor model

| Administration group | Tumor volume ± SE (mm³) on day 21^{th} of administration | Tumor inhibition rate (%) | P value (vs. vehicle) |
|---|---|---|---|
| Vehicle | 2508.93±166.86 | Control | Control |
| Hu001-2-MMAE-2.5mg/kg | 1527.37±10⁷.41 | 39.12 | <0.001 |
| Hu001-2-MMAE-5mg/kg | 756.45±98.69 | 69.85 | <0.0001 |
| Hu005-44-MMAE-2.5mg/kg | 1953.43±265.03 | 22.14 | ns |
| Hu005-44-MMAE-5mg/kg | 878.98±110.73 | 64.97 | <0.0001 |
| Hu005-46-MMAE-2.5mg/kg | 1512.29±118.11 | 39.72 | <0.001 |
| Hu005-46-MMAE-5mg/kg | 715.42±73.82 | 71.49 | <0.0001 |
| UC961-MMAE-2.5mg/kg | 2010.44±266.07 | 19.87 | ns |
| UC961-MMAE-5mg/kg | 1171.18±130.32 | 53.32 | <0.0001 |

### Table 12: Tumor inhibition rate (%) of ROR1 humanized antibody-conjugate in HCC1187 tumor model

| Administration group | Tumor volume ± SE (mm³) on day 21^{th} of administration | Tumor inhibition rate (%) | P value (vs. vehicle) |
|---|---|---|---|
| Vehicle | 3037.57±63 6.46 | Control | Control |
| Hu001-2-MMAE-2.5mg/kg | 1831.48±304.82 | 39.71 | ns |
| Hu001-2-MMAE-5mg/kg | 233.70±93.90 | 92.31 | <0.001 |
| UC961-MMAE-2.5mg/kg | 2576.56±461.28 | 15.18 | ns |
| UC961-MMAE-5mg/kg | 407.09±69.85 | 86.60 | <0.01 |

In summary, the research results of the Examples related to ROR1 humanized antibodies and antibody-conjugates clearly show that:
(1) The humanized antibody has excellent antigen binding ability, with an EC₅₀ value of binding to ROR1-ECD protein of <0.03nM; and an EC₅₀ value of binding to ROR1 on the cell surface of <1nM;
(2) The humanized antibody has excellent endocytosis properties and can be used as a target for ADC drug development;
(3) The humanized antibody-conjugate (coupled with vcMMAE) maintains the high antigen binding affinity of the parent antibody for ROR1-ECD protein and ROR1 on the cell surface;
(4) The humanized antibody-conjugate has significant in vitro anti-proliferative activity against multiple cancer cells with the killing activity (IC 50 value) correlated with the ROR1 expression level;
(5) The humanized antibody-conjugate has excellent in vivo anti-tumor activity against CDX models with different ROR1 expression levels, and significantly better therapeutic effect than the positive reference UC961-MMAE.

The technical features of the above-mentioned implementation modes and examples can be combined in any appropriate manner. To make the description concise, not all possible combinations of the technical features in the above-mentioned implementation modes and examples are described. However, the combination of those technical features should be considered to be within the scope of this specification as long as there is no contradiction therein.

The above-described embodiments only express several implementation methods of the present application, which are convenient for understanding the technical solution of the present application in detail, but cannot be understood as a limitation on the scope of protection of the patent application. It should be pointed out that for ordinary technicians in this field, without departing from the concept of the present application, several variants and improvements can be made, which all fall within the protection scope of the present application. In addition, it should be understood that after reading the above-mentioned teaching content of the present application, the technicians in this field can make various changes or modifications to the present application, and the equivalent forms obtained also fall within the protection scope of the present application. It should also be understood that the technical solutions obtained by the technicians in this field through logical analysis, reasoning or limited experiments on the basis of the technical solutions provided in the present application are all within the protection scope of the claims attached to the present application. Therefore, the protection scope of the patent of the present application shall be based on the content of the attached claims, and the description and drawings can be used to explain the content of the claims.

## Claims

1. An antibody capable of specifically binding to ROR1, the antibody comprising a heavy chain variable region and a light chain variable region and meeting one or more of the following conditions (I) and (II):
(I) the heavy chain variable region comprising heavy chain complementarity determining regions selected from one or more sets of the following heavy chain complementarity determining regions or derivative fragments thereof:
i) VH-CDR1 as set forth in SEQ ID NO: 1, VH-CDR2 as set forth in SEQ ID NO: 2, and VH-CDR3 as set forth in SEQ ID NO: 3;
ii) VH-CDR1 as set forth in SEQ ID NO: 11, VH-CDR2 as set forth in SEQ ID NO: 12, and VH-CDR3 as set forth in SEQ ID NO: 13; and
iii) VH-CDR1 of SEQ ID NO.19, VH-CDR2 of SEQ ID NO.20, and VH-CDR3 of SEQ ID NO.21; and
(II) the light chain variable region comprising light chain complementarity determining regions selected from one or more sets of the following light chain complementarity determining regions or derivative fragments thereof:
i') VL-CDR1 as set forth in SEQ ID NO: 4, VL-CDR2 as set forth in SEQ ID NO: 5, and VL-CDR3 as set forth in SEQ ID NO: 6;
ii ') VL-CDR1 as set forth in SEQ ID NO: 14, VL-CDR2 as set forth in SEQ ID NO: 15, and VL-CDR3 as set forth in SEQ ID NO: 16; and
iii ') VL-CDR1 as set forth in SEQ ID NO: 22, VL-CDR2 as set forth in SEQ ID NO: 23, and VL-CDR3 as set forth in SEQ ID NO: 24;
wherein the derivative fragments have no more than 6 amino acid substitutions with respect to the corresponding complementary determining regions and are capable of retaining EC₅₀ of 0.009 nM to 0.05 nM for ROR1-ECD and EC₅₀ of 0.15 nM to 1.1 nM for tumor cell ROR1.

2. The antibody capable of specifically binding to ROR1 of claim 1, wherein the antibody meets one or more of the following conditions (A) and (B):
(A) the heavy chain complementarity determining regions of the heavy chain variable region are selected from one or more sets of the following heavy chain complementarity determining regions:
i) VH-CDR1 having a sequence represented by general formula D-Y-N-Xa1-H, VH-CDR2 having a sequence represented by general formula Y-I-N-P-N-Xa2-Xa3-Xa4-T-Xa5-Y-N-Q-K-F-Xa6-G, and VH-CDR3 having a sequence represented by general formula R-Xa7- Xa8- Xa9- Xa10- Xa11- Xa12-Xa13-D-Xa14; wherein Xa1 is I, M or L, Xa2 is N or H, Xa3 is D or G, Xa4 is A, N or G, Xa5 is S, N or T, Xa6 is Q, K or E, Xa7 is V or G, Xa8 is R or Y, Xa9 is T or G, Xa10 is S or G, Xa11 is S, T or G, Xa12 is G or Y, Xa13 is L, F or AM, and Xa14 is D, F or Y;
ii) VH-CDR1 as set forth in SEQ ID NO: 11, VH-CDR2 as set forth in SEQ ID NO: 12, and VH-CDR3 as set forth in SEQ ID NO: 13; and
iii) VH-CDR1 as set forth in SEQ ID NO: 19, VH-CDR2 having a sequence represented by general formula T-I-S-D-Xb1-G-S-Y-T-Y-Y-P-D-Xb2-Xb3-K-G, and VH-CDR3 as set forth in SEQ ID NO: 21; wherein Xb1 is A or G, Xb2 is S or N, and Xb3 is V or E;
(B) the light chain complementarity determining regions of the light chain variable region selected from one or more sets of the following light chain complementarity determining regions or the derivative fragments thereof:
i') VL-CDR1 having a sequence represented by general formula Xa1'-S-S-Xa2'-Xa3'-I-Xa4'-H-T-N-Xa5'-N-T-Y-L-E, VL-CDR2 having a sequence represented by general formula K-V-Xa6'-N-R-F-S, and VL-CDR3 having a sequence represented by general formula F-Q-G-S-Xa7'- Xa8'-P-Y-T; wherein Xa1' is R, K or T, Xa2' is H or Q, Xa3' is I, N or S, Xa4' is V or L, Xa5' is A or G, Xa6' is F or S, Xa7' is R or L, and Xa8' is F or V;
ii') VL-CDR1 as set forth in SEQ ID NO: 14, VL-CDR2 as set forth in SEQ ID NO: 15, and VL-CDR3 as set forth in SEQ ID NO: 16; and
iii') VL-CDR1 having a sequence represented by general formula K-A-S-Q-S-V-S-F-Xb1'-G-T-S-L-M-H, VL-CDR2 as set forth in SEQ ID NO: 23, and VL-CDR3 as set forth in SEQ ID NO: 24; wherein Xb1' is A or P.

3. The antibody capable of specifically binding to ROR1 of claim 2, wherein in the heavy chain complementary determining regions as shown in i):
Xa1 is L, Xa2 is H, Xa3 is D, Xa4 is A or G, Xa5 is S or T, Xa6 is Q, Xa7 is V, Xa8 is R, Xa9 is T, Xa10 is G, Xa11 is T, Xa12 is G, Xa13 is L or F, and Xa14 is D or Y; or,
Xa1 is I or M, Xa2 is N, Xa3 is G, Xa4 is N or G, Xa5 is S, N or T, Xa6 is K or E, Xa7 is G, Xa8 is Y, Xa9 is G, Xa10 is S, Xa11 is S or G, Xa12 is Y, Xa13 is AM, and Xa14 is F or Y

4. The antibody capable of specifically binding to ROR1 of claim 2, wherein in the light chain complementary determining regions as shown in i'):
Xa1' is R or K, Xa2' is H, Xa3' is N, Xa4' is V or L, Xa5' is A or G, Xa6' is S, Xa7' is R, and Xa8' is F; or,
Xa1' is R or T, Xa2' is H or Q, Xa3' is I or S, Xa4' is V, Xa5' is G, Xa6' is F or S, Xa7' is R or L, and Xa8' is F or V.

5. The antibody capable of specifically binding to ROR1 of claim 2, wherein the antibody has one or more of the following combinations of heavy chain complementary determining regions and light chain complementary determining regions:
Combination 1: the heavy chain complementary determining regions as shown in i) or derivative fragments thereof, and the light chain complementary determining region as shown in i') or derivative fragments thereof;
Combination 2: the heavy chain complementary determining regions as shown in ii) or derivative fragments thereof, and the light chain complementary determining region as shown in ii') or derivative fragments thereof; and
Combination 3: the heavy chain complementary determining regions as shown in iii) or derivative fragments thereof, and the light chain complementary determining region as shown in iii') or derivative fragments thereof.

6. The antibody capable of specifically binding to ROR1 of claim 5, wherein the combination 1 is selected from one or more of the following combinations:
Combination 1-1: VH-CDR1 as set forth in SEQ ID NO: 1, VH-CDR2 as set forth in SEQ ID NO: 2, VH-CDR3 as set forth in SEQ ID NO: 3, VL-CDR1 as set forth in SEQ ID NO: 4, VL-CDR2 as set forth in SEQ ID NO: 5, and VL-CDR3 as set forth in SEQ ID NO: 6;
Combination 1-2: VH-CDR1 as set forth in SEQ ID NO: 35, VH-CDR2 as set forth in SEQ ID NO: 36, VH-CDR3 as set forth in SEQ ID NO: 37, VL-CDR1 as set forth in SEQ ID NO: 38, VL-CDR2 as set forth in SEQ ID NO: 39, and VL-CDR3 as set forth in SEQ ID NO: 40;
Combination 1-3: VH-CDR1 as set forth in SEQ ID NO: 51, VH-CDR2 as set forth in SEQ ID NO: 52, VH-CDR3 as set forth in SEQ ID NO: 53, VL-CDR1 as set forth in SEQ ID NO: 54, VL-CDR2 as set forth in SEQ ID NO: 55, and VL-CDR3 as set forth in SEQ ID NO: 56;
Combination 1-4: VH-CDR1 as set forth in SEQ ID NO: 59, VH-CDR2 as set forth in SEQ ID NO: 60, VH-CDR3 as set forth in SEQ ID NO: 61, VL-CDR1 as set forth in SEQ ID NO: 62, VL-CDR2 as set forth in SEQ ID NO: 63, and VL-CDR3 as set forth in SEQ ID NO: 64;
Combination 1-5: VH-CDR1 as set forth in SEQ ID NO: 67, VH-CDR2 as set forth in SEQ ID NO: 68, VH-CDR3 as set forth in SEQ ID NO: 69, VL-CDR1 as set forth in SEQ ID NO: 70, VL-CDR2 as set forth in SEQ ID NO: 71, and VL-CDR3 as set forth in SEQ ID NO: 72;
Combination 1-6: VH-CDR1 as set forth in SEQ ID NO: 75, VH-CDR2 as set forth in SEQ ID NO: 76, VH-CDR3 as set forth in SEQ ID NO: 77, VL-CDR1 as set forth in SEQ ID NO: 78, VL-CDR2 as set forth in SEQ ID NO: 79, and VL-CDR3 as set forth in SEQ ID NO: 80; and
Combination 1-7: VH-CDR1 as set forth in SEQ ID NO: 1, VH-CDR2 as set forth in SEQ ID NO: 83, VH-CDR3 as set forth in SEQ ID NO: 3, VL-CDR1 as set forth in SEQ ID NO: 84, VL-CDR2 as set forth in SEQ ID NO: 5, and VL-CDR3 as set forth in SEQ ID NO: 6.

7. The antibody capable of specifically binding to ROR1 of claim 5, wherein the combination 3 is selected from one or more of the following combinations:
Combination 3-1: VH-CDR1 as set forth in SEQ ID NO: 43, VH -CDR2 as set forth in SEQ ID NO: 44, VH-CDR3 as set forth in SEQ ID NO: 45, VL-CDR1 as set forth in SEQ ID NO: 46, VL-CDR2 as set forth in SEQ ID NO: 47, and VL-CDR3 as set forth in SEQ ID NO: 48;
Combination 3-2: VH-CDR1 as set forth in SEQ ID NO: 19, VH-CDR2 as set forth in SEQ ID NO: 20, VH-CDR3 as set forth in SEQ ID NO: 21, VL-CDR1 as set forth in SEQ ID NO: 22, VL-CDR2 as set forth in SEQ ID NO: 23, and VL-CDR3 as set forth in SEQ ID NO: 24; and
Combination 3-3: VH-CDR1 as set forth in SEQ ID NO: 19, VH-CDR2 as set forth in SEQ ID NO: 85, VH-CDR3 as set forth in SEQ ID NO: 21, VL-CDR1 as set forth in SEQ ID NO: 22, VL-CDR2 as set forth in SEQ ID NO: 23, and VL-CDR3 as set forth in SEQ ID NO: 24.

8. The antibody capable of specifically binding to ROR1 of any one of claims 1 to 7, wherein the antibody has one or more of the following technical features:
(1) the antibody has a heavy chain constant region and a light chain constant region independently derived from a species selected from human, mouse, rabbit, sheep, cattle, horse, pig, dog, cat, camel, donkey, deer, mink, chicken, duck or goose;
(2) the constant region of the antibody has a sequence of the constant region selected from any one of IgG1, IgG2, IgG3, IgG4, IgA, IgM, IgE and IgD; and
(3) the constant region of the antibody has a sequence of the constant region selected from any one of κ light chain constant region and λ light chain constant region.

9. The antibody capable of specifically binding to ROR1 of claim 8, wherein the antibody has one or more combinations selected from the following combinations of a heavy chain variable region and a light chain variable region:
(1) a heavy chain variable region as set forth in SEQ ID NO: 7 and a light chain variable region as set forth in SEQ ID NO: 9;
(2) a heavy chain variable region as set forth in SEQ ID NO: 41 and a light chain variable region as set forth in SEQ ID NO: 42;
(3) a heavy chain variable region as set forth in SEQ ID NO: 49 and a light chain variable region as set forth in SEQ ID NO: 50;
(4) a heavy chain variable region as set forth in SEQ ID NO: 17 and a light chain variable region as set forth in SEQ ID NO: 18;
(5) a heavy chain variable region as set forth in SEQ ID NO: 25 and a light chain variable region as set forth in SEQ ID NO: 27;
(6) a heavy chain variable region as set forth in SEQ ID NO: 57 and a light chain variable region as set forth in SEQ ID NO: 58;
(7) a heavy chain variable region as set forth in SEQ ID NO: 65 and a light chain variable region as set forth in SEQ ID NO: 66;
(8) a heavy chain variable region as set forth in SEQ ID NO: 73 and a light chain variable region as set forth in SEQ ID NO: 74;
(9) a heavy chain variable region as set forth in SEQ ID NO: 81 and a light chain variable region as set forth in SEQ ID NO: 82;
(10) a heavy chain variable region as set forth in SEQ ID NO: 8 and a light chain variable region as set forth in SEQ ID NO: 10;
(11) a heavy chain variable region as set forth in SEQ ID NO: 26 and a light chain variable region as set forth in SEQ ID NO: 27;
(12) a heavy chain variable region as set forth in SEQ ID NO: 28 and a light chain variable region as set forth in SEQ ID NO: 33;
(13) a heavy chain variable region as set forth in SEQ ID NO: 28 and a light chain variable region as set forth in SEQ ID NO: 34;
(14) a heavy chain variable region as set forth in SEQ ID NO: 29 and a light chain variable region as set forth in SEQ ID NO: 33;
(15) a heavy chain variable region as set forth in SEQ ID NO: 29 and a light chain variable region as set forth in SEQ ID NO: 34;
(16) a heavy chain variable region as set forth in SEQ ID NO: 30 and a light chain variable region as set forth in SEQ ID NO: 33;
(17) a heavy chain variable region as set forth in SEQ ID NO: 31 and a light chain variable region as set forth in SEQ ID NO: 33;
(18) a heavy chain variable region as set forth in SEQ ID NO: 31 and a light chain variable region as set forth in SEQ ID NO: 34;
(19) a heavy chain variable region as set forth in SEQ ID NO: 32 and a light chain variable region as set forth in SEQ ID NO: 33;
(20) a heavy chain variable region as set forth in SEQ ID NO: 32 and a light chain variable region as set forth in SEQ ID NO: 34;
(21) a heavy chain variable region as set forth in SEQ ID NO: 93 and a light chain variable region as set forth in SEQ ID NO: 101;
(22) a heavy chain variable region as set forth in SEQ ID NO: 94 and a light chain variable region as set forth in SEQ ID NO: 101;
(23) a heavy chain variable region as set forth in SEQ ID NO: 95 and a light chain variable region as set forth in SEQ ID NO: 101;
(24) a heavy chain variable region as set forth in SEQ ID NO: 96 and a light chain variable region as set forth in SEQ ID NO: 101;
(25) a heavy chain variable region as set forth in SEQ ID NO: 97 and a light chain variable region as set forth in SEQ ID NO: 101;
(26) a heavy chain variable region as set forth in SEQ ID NO: 98 and a light chain variable region as set forth in SEQ ID NO: 101;
(27) a heavy chain variable region as set forth in SEQ ID NO: 99 and a light chain variable region as set forth in SEQ ID NO: 101; and
(28) A heavy chain variable region as set forth in SEQ ID NO: 100 and a light chain variable region as set forth in SEQ ID NO: 101.

10. A recombinant protein, comprising:
one or more of the heavy chain complementarity determining regions and light chain complementarity determining regions as defined in any one of claims 1 to 9; and
a tag fragment that facilitates expression and purification of the heavy chain complementary determining regions and the light chain complementary determining regions.

11. The recombinant protein of claim 10, wherein the recombinant protein further comprises the heavy chain constant region or the light chain constant region as defined in claim 8, or a combination thereof.

12. A CAR construct, wherein the CAR construct has a scFV domain having the heavy chain variable region and the light chain variable region as defined in any one of claims 1 to 9.

13. A nucleic acid encoding the antibody of any one of claims 1 to 9, the recombinant protein of claim 10 or 11, or the CAR construct of claim 12.

14. A vector, wherein the vector contains the nucleic acid of claim 13.

15. The vector of claim 14, wherein the vector is selected from a bacterial plasmid, a bacteriophage, a yeast plasmid, a plant cell virus, a mammalian cell virus such as adenovirus or retrovirus, or a combination thereof.

16. A host cell, wherein the host cell contains the nucleic acid of claim 13 or the vector of claim 14 or 15.

17. The host cell of claim 16, wherein the host cell is CHO cells, COS cells, NSO cells, HeLa cells, BHK cells or HEK293 cells.

18. A method for preparing the antibody of any one of claims 1 to 9, the recombinant protein of claim 10 or 11, or the CAR construct of claim 12, the method comprising:
culturing the host cell of claim 16 or 17, and isolating the antibody or antigen-binding fragment thereof, recombinant protein or CAR construct from the obtained culture.

19. A recombinant immune cell, wherein the recombinant immune cell expresses the exogenous CAR construct of claim 10.

20. The recombinant immune cell of claim 19, wherein the immune cell is selected from one of NK cells and T cells.

21. Use of the antibody of any one of claims 1 to 9, the recombinant protein of claim 10 or 11, the CAR construct of claim 12, or the recombinant immune cell of claim 19 or 20 in manufacturing a medicament for treating ROR1-related disease or a product for diagnosing a ROR1-related disease.

22. The use of claim 21, wherein the ROR1-related disease is a tumor, an autoimmune disease, a metabolism-related disease or an infectious disease.

23. The use of claim 22, wherein the tumor is breast cancer, lung cancer, pancreatic cancer, ovarian cancer, prostate cancer, colorectal cancer, gastric cancer, liver cancer, esophageal cancer, kidney cancer, brain glioma, bladder cancer, prostate cancer, endometrial cancer, cervical cancer, leukemia, lymphoma, bone marrow cancer or angiosarcoma;
the inflammation is rheumarthritis, osteoarthritis, gout, Reiter's syndrome, psoriatic arthropathy, tuberculous arthritis, glomerulonephritis, systemic lupus erythematosus, Crohn's disease, ulcerative colitis, acute lung injury, chronic obstructive pulmonary disease or idiopathic pulmonary fibrosis;
the autoimmune disease is rheumatoid arthritis, ankylosing spondylitis, systemic lupus erythematosus, Sjögren's syndrome or systemic vasculitis, ulcerative colitis, type I diabetes, psoriasis, or multiple sclerosis; the metabolic-related disease is diabetes, diet-induced obesity or fat inflammation; or
the infectious disease is a disease caused by bacterial infection or a disease caused by viral infection.

24. The use of any one of claims 21 to 23, wherein the product for diagnosing ROR1-related disease is a diagnostic reagent, a test strip, a test plate or a test kit.

25. An antibody-drug conjugate, wherein the antibody-drug conjugate comprises:
an antibody selected from the antibody of any one of claims 1 to 9; and,
a conjugant to be compled to the antibody.

26. The antibody-drug conjugate of claim 25, wherein the antibody-drug conjugate has one or more of the following technical features:
the conjugant is selected from a detectable label, a cytotoxic drug, a cytokine, a radionuclide, an enzyme or a combination thereof; and,
the antibody is coupled to the conjugant via a chemical linkage or a linker.

27. The antibody-drug conjugate of claim 25 or 26, wherein the antibody-drug conjugate has one or more of the following technical features:
the antibody-drug conjugate has a structure as shown in the following molecular formula: 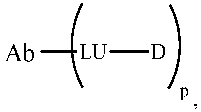 wherein
Ab is the antibody,
LU is a linker, and
D is a cytotoxic drug;
the linker comprises a sulfydryl-specific active reactive group; and
the cytotoxic drug is a microtubule-targeted drug, a DNA-targeted drug or a topoisomerase inhibitor.

28. A pharmaceutical composition, comprising:
the antibody of any one of claims 1 to 9, the recombinant protein of claim 10 or 11, the CAR construct of claim 12, the recombinant immune cell of claim 19 or 20, the antibody-drug conjugate of any one of claims 25 to 27, or a combination thereof; and
a pharmaceutically acceptable carrier.

29. A product for diagnosing ROR1-related disease, wherein
the product is a test plate coated with the antibody of any one of claims 1 to 9, the recombinant protein of claim 10 or 11, the CAR construct of claim 12, the recombinant immune cell of claim 19 or 20, or a combination thereof; or
the product is a test kit comprising a primary antibody and a secondary antibody for detecting ROR1, wherein the primary antibody is the antibody of any one of claims 1 to 9.

30. A method for detecting ROR1 for non-diagnostic purposes, comprising:
providing a sample to be tested, and
mixing the sample to be tested with the antibody of any one of claims 1 to 9 for reaction, detecting the reaction product, and determining the presence of ROR1 in the sample to be tested based on the test result.

31. The method of claim 30, wherein determining the presence of ROR1 in the sample to be tested based on the test result comprises:
when the antigen-antibody complex is detected in the reaction product, it is determined that the sample to be tested contains ROR1; or
when no antigen-antibody complex is detected in the reaction product, it is determined that the sample to be tested contains no ROR1.
